(19)

Europäisches
Patentamt
European
Patent Office
Office européen
des brevets

(11) **EP 4 308 729 B1**

(12) **EUROPEAN PATENT SPECIFICATION**

(45) Date of publication and mention
of the grant of the patent:
**29.10.2025 Bulletin 2025/44**

(21) Application number: **22705490.5**

(22) Date of filing: **14.01.2022**

(51) International Patent Classification (IPC):
***C12Q 1/6883*** *(2018.01)*

(52) Cooperative Patent Classification (CPC):
**C12Q 1/6883;** C12Q 2600/158

(86) International application number:
**PCT/US2022/012462**

(87) International publication number:
**WO 2022/197351 (22.09.2022 Gazette 2022/38)**

(54) **METHODS FOR DISCRIMINATING BACTERIAL AND VIRAL INFECTIONS IN A HUMAN SUBJECT**

VERFAHREN ZUR UNTERSCHEIDUNG BAKTERIELLER UND VIRALER INFEKTIONEN BEI EINER PERSON

PROCÉDÉS POUR DISTINGUER LES INFECTIONS BACTÉRIENNES ET VIRALES CHEZ UN SUJET HUMAIN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.03.2021 US 202163161576 P**

(43) Date of publication of application:
**24.01.2024 Bulletin 2024/04**

(73) Proprietor: **Cepheid**
**Sunnyvale, CA 94089 (US)**

(72) Inventors:
• **WALLACE, Stacey**
**171 54 Solna (SE)**
• **BAIG, Rebekah Gonzalez**
**Sunnyvale, California 94089 (US)**

• **LU, Xixi**
**Sunnyvale, California 94089 (US)**
• **KALDATE, Rajesh**
**Sunnyvale, California 94089 (US)**

(74) Representative: **Maiwald GmbH**
**Elisenhof**
**Elisenstraße 3**
**80335 München (DE)**

(56) References cited:
**WO-A1-2013/117746     WO-A1-2017/214061
WO-A2-2011/132086     US-A1- 2015 376 698
US-A1- 2020 216 904**

Remarks:
The complete document including Reference Table(s) and the Sequence Listing(s) can be downloaded from the EPO website

## Description

### GOVERNMENT SUPPORT

This invention was made with government support under Agreement No. W15QKN-16-9-1002 awarded by the ACC-NJ to the MCDC. The government has certain rights in the invention.

### SEQUENCE LISTING

The instant application contains a Sequence Listing which has been submitted in ASCII format via EFS-Web. Said ASCII copy, created on January 13, 2022, is named "CEPH-001_001WO_SeqList.txt" and is about 256,058 bytes in size.

### BACKGROUND

There exists a need in the art for methods and kits directed to the rapid, inexpensive and accurate identification of bacterial infections, viral infections and non-infectious causes of fever in a patient, including those that have recently undergone surgery. The present disclosure addresses this and other needs and provides methods and 2 non-claimed 2 kits for determining whether a patient has a bacterial infection, a viral infection, both a viral infection and a bacterial infection, or a non-infectious cause of fever. Without wishing to be bound by theory, the methods and kits presented in the present disclosure allow clinicians to rapidly and accurately identify viral and/or bacterial infections, allowing the clinicians to administer anti-viral and/or antibiotic therapies in a time-effective manner, increasing the probability of patient recovery.
US 2020/216904, US 2015/376698, WO 2017/214061, WO 201 1/132086 and WO 2013/117746 all relate to methods of using expression profiles to establish the local inflammation of a fever of unknown origin or distinguish viral and bacterial infections from each other or non-infectious disease. 2

### SUMMARY

The invention is defined by the scope of the appended claims. The present disclosure provides methods and non-claimed kits for the identification and/or treatment of bacterial infections, viral infections and/or non-infectious causes of fever in a subject in need thereof.
The present disclosure provides methods of determining if a subject has a bacterial infection, a viral infection, or a non-infectious cause of fever, the methods comprising: a) determining the expression levels of RUNX1, ITGAM, PSTPIP2, LY6E, IRF9 and ABL1 in a biological sample from the subject; b) determining that the expression level of ABL1 is greater than a first predetermined cutoff value; c) determining a viral infection score and a bacterial infection score based on the expression levels of RUNX1, ITGAM, PSTPIP2, LY6E and IRF9; d) comparing the viral infection score to a second predetermined cutoff value and the bacterial infection score to a third predetermined cutoff value; and e) determining: that the subject has a viral infection when the viral infection score is greater than the second predetermined cutoff value or that the subject does not have viral infection when the viral infection score is less than the second predetermined cutoff value, that the subject has a bacterial infection when the bacterial infection score is greater than the third predetermined cutoff value or the subject does not have a bacterial infection when the bacterial infection score is less than or equal to the third predetermined cutoff value, and that the subject has a non-infectious cause of fever in the subject when the viral infection score is less than the second predetermined cutoff value and the bacterial infection score is less than the third predetermined cutoff value. In some aspects of the methods disclosed herein, the subject can be identified as having both a bacterial infection and a viral infection when the viral infection score is greater than the second predetermined cutoff value; and the bacterial infection score is greater than the third predetermined cutoff value.
Disclosed but not claimed are methods of treating a bacterial infection, a viral infection, or a non-infectious cause of fever in a subject in need thereof, the methods comprising: a) determining the expression levels of RUNX1, ITGAM, PSTPIP2, LY6E, IRF9 and ABL1 in a biological sample from the subject; b) determining that the expression level of ABL1 is greater than a first predetermined cutoff value; c) determining a viral infection score and a bacterial infection score based on the expression levels of RUNX1, ITGAM, PSTPIP2, LY6E and IRF9; d) comparing the viral infection score to a second predetermined cutoff value and the bacterial infection score to a third predetermined cutoff value; e) determining: that the subject has a viral infection when the viral infection score is greater than the second predetermined cutoff value or that the subject does not have viral infection when the viral infection score is less than the second predetermined cutoff value, that the subject has a bacterial infection when the bacterial infection score is greater than the third predetermined cutoff value or the subject does not have a bacterial infection when the bacterial infection score is less than or equal to the third predetermined cutoff value, and that the subject has a non-infectious cause of fever in the subject when the viral infection score is less than the second predetermined cutoff value and the bacterial infection score is less than the third predetermined cutoff value, and f) administering to the subject: at least one antiviral therapy when the subject is identified to have a viral infection, at least one antibiotic therapy when the subject is identified to have a bacterial infection, and at least one antipyretic therapy when the subject is identified to have a non-infectious cause of fever. In some aspects of the methods disclosed herein, the method can comprise administering to a subject at least one antibiotic therapy and at least one antiviral therapy when the subject is identified as having both a bacterial infection and a viral infection.

The present disclosure provides a method of determining if a subject has a bacterial infection, a viral infection, or a non-infectious cause of fever, the method comprising: a) determining the expression levels of RUNX1, ITGAM, PSTPIP2, LY6E and IRF9 in a biological sample from the subject; b) determining a viral infection score and a bacterial infection score based on the expression levels of RUNX1, ITGAM, PSTPIP2, LY6E and IRF9; c) comparing the viral infection score to a first predetermined cutoff value and the bacterial infection score to a second predetermined cutoff value, and d) determining: that the subject has a viral infection when the viral infection score is greater than the first predetermined cutoff value or that the subject does not have viral infection when the viral infection score is less than the first predetermined cutoff value, that the subject has a bacterial infection when the bacterial infection score is greater than the second predetermined cutoff value or the subject does not have a bacterial infection when the bacterial infection score is less than or equal to the second predetermined cutoff value, and that the subject has a non-infectious cause of fever in the subject when the viral infection score is less than the first predetermined cutoff value and the bacterial infection score is less than the second predetermined cutoff value. In some aspects, the subject can be identified as having both a bacterial infection and a viral infection when: the viral infection score is greater than the second predetermined cutoff value; and the bacterial infection score is greater than the third predetermined cutoff value.

Disclosed but not claimed is a method of treating a bacterial infection, a viral infection, or a non-infectious cause of fever in a subject in need thereof, the method comprising: a) determining the expression levels of RUNX1, ITGAM, PSTPIP2, LY6E and IRF9 in a biological sample from the subject; b) determining a viral infection score and a bacterial infection score based on the expression levels of RUNX1, ITGAM, PSTPIP2, LY6E and IRF9; c) comparing the viral infection score to a first predetermined cutoff value and the bacterial infection score to a second predetermined cutoff value; d) determining: that the subject has a viral infection when the viral infection score is greater than the first predetermined cutoff value or that the subject does not have viral infection when the viral infection score is less than the first predetermined cutoff value, that the subject has a bacterial infection when the bacterial infection score is greater than the second predetermined cutoff value or the subject does not have a bacterial infection when the bacterial infection score is less than or equal to the second predetermined cutoff value, and that the subject has a non-infectious cause of fever in the subject when the viral infection score is less than the first predetermined cutoff value and the bacterial infection score is less than the second predetermined cutoff value; and f) administering to the subject: at least one antiviral therapy when the subject is identified to have a viral infection, at least one antibiotic therapy when the subject is identified to have a bacterial infection, and at least one antipyretic therapy when the subject is identified to have a non-infectious cause of fever.

Step (a) further comprises determining the expression level of ABL1, wherein the expression level of ABL1 is used as a positive control biomarker that is indicative of the quality of the biological sample. The of the present disclosure further comprise comparing the expression level of ABL1 to a third predetermined cutoff value wherein step (d) comprises: determining:

that the subject has a viral infection when the viral infection score is greater than the first predetermined cutoff value and the expression level of ABL1 is greater than the third predetermined cutoff value; that the subject does not have viral infection when the viral infection score is less than the first predetermined cutoff value and the expression level of ABL1 is greater than the third predetermined cutoff value; that the subject has a bacterial infection when the bacterial infection score is greater than the second predetermined cutoff value and the expression level of ABL1 is greater than the third predetermined cutoff value; that the subject does not have a bacterial infection when the bacterial infection score is less than or equal to the second predetermined cutoff value and the expression level of ABL1 is greater than the third predetermined cutoff value; and that the subject has a non-infectious cause of fever in the subject when the viral infection score is less than the first predetermined cutoff value, the bacterial infection score is less than the second predetermined cutoff value and the expression level of ABL1 is greater than the third predetermined cutoff value. In some aspects, a subject is identified as having both a bacterial infection and a viral infection when the viral infection score is greater than the first predetermined cutoff value, the bacterial infection score is greater than the second predetermined cutoff value and the expression level of ABL1 is greater than the third predetermined cutoff value.

In some aspects of the non-claimed methods of treating a bacterial infection, a viral infection, or a non-infectious cause of fever in a subject in need thereof, the methods comprise a) identifying the subject as having a bacterial infection, a viral infection, or a non-infectious cause of fever, based on the expression levels of biomarkers RUNX1, ITGAM, PSTPIP2, LY6E, IRF9 and ABL1 in a biological sample from the subject; and b) administering to the subject: at least one antiviral therapy when the subject is identified to have a viral infection, at least one antibiotic therapy when the subject is identified to have a bacterial infection, and at least one antipyretic therapy when the subject is identified to have a non-infectious cause of fever.

The present disclosure provides methods of triaging a patient suspected of having a bacterial infection, a viral infection, or a non-infectious cause of fever, the method comprising: a) determining if the patient has a bacterial infection, a viral infection, or a non-infectious cause of fever as described herein, and b) providing a diagnostic or treatment recommendation based on the determined infection. The methods of triaging the patient can further comprise determining one or more additional patient metrics selected from an infection status, clinical history, demography, nature and severity of symptoms, or a combination thereof. In some examples, the methods comprise determining an infection status of the patient including the severity of the infection, the characteristic (specific) infection, or a combination thereof.

In the methods disclosed herein, determining a viral infection score based on the expression levels of RUNX1, ITGAM, PSTPIP2, LY6E and IRF9 can comprise inputting the expression levels or the normalized expression levels of RUNX1, ITGAM, PSTPIP2, LY6E, IRF9 and optionally ABL1, into an algorithm that determines the viral infection score. The normalized expression levels of RUNX1, ITGAM, PSTPIP2, LY6E and IRF9 can be obtained by normalizing the expression levels of RUNX1, ITGAM, PSTPIP2, LY6E and IRF9 to the expression level of ABL1. The algorithm can be a product of a logistic regression model generated using the expression levels of RUNX1, ITGAM, PSTPIP2, LY6E, IRF9 and optionally ABL1, measured in a training set of biological samples, wherein the training set of biological samples comprises: i) a plurality of biological samples isolated from subjects identified as having a bacterial infection; ii) a plurality of biological samples isolated from subjects identified as having a viral infection; iii) a plurality of biological samples isolated from subjects identified has having both a bacterial infection and a viral infection; iv) a plurality of biological samples isolated from subjects identified as having a non-infectious cause of fever; and v) a plurality of biological samples isolated from healthy subjects not having a bacterial infection, viral infection or non-infectious cause of fever. The logistic regression model can be a product of univariate analysis and/or multivariate analysis of the expression levels of RUNX1, ITGAM, PSTPIP2, LY6E, IRF9 and optionally, ABL1, in the training set of biological samples.

In some aspects of the methods disclosed herein, the algorithm that determines the viral infection score is:

$$p = \frac{1}{1 + e^{-(b + m_1 * RUNX1 + m_2 * ITGAM + m_3 * PSTPIP2 + m_4 * LY6E + m_5 * IRF9)}},$$

wherein *RUNX1* is the expression level or the normalized expression level of RUNX1 in the biological sample from the subject,

*ITGAM* is the expression level or the normalized expression level of ITGAM in the biological sample from the subject,

*PSTPIP2* is the expression level or the normalized expression level of PSTPIP2 in the biological sample from the subject,

*LY6E* is the expression level or the normalized expression level of LY6E in the biological sample from the subject,

*IRF9* is the expression level or the normalized expression level of IRF9 in the biological sample from the subject, and

*b* is an intercept, $m_1$ is a scaling factor for the expression level of RUNX, $m_2$ is a scaling factor for the expression level of ITGAM, $m_3$ is a scaling factor for the expression level of PSTPIP2, $m_4$ is a scaling factor for the expression level of LY6E, $m_5$ is a scaling factor for the expression level of IRF9.

In some aspects of the methods disclosed herein, the algorithm that determines the viral infection score is:

$$p = \frac{1}{1 + e^{-(b + m_1 * RUNX1 + m_2 * ITGAM + m_3 * PSTPIP2 + m_4 * LY6E + m_5 * IRF9 + m_6 * ABL1)}},$$

wherein *RUNX1* is the expression level of RUNX1 in the biological sample from the subject,

*ITGAM* is the expression level of ITGAM in the biological sample from the subject,

*PSTPIP2* is the expression level of PSTPIP2 in the biological sample from the subject,

*LY6E* is the expression level of LY6E in the biological sample from the subject,

*IRF9* is the expression level of IRF9 in the biological sample from the subject,

*ABL1* is the expression level of ABL1 in the biological sample from the subject, and

*b* is an intercept, $m_1$ is a scaling factor for the expression level of RUNX, $m_2$ is a scaling factor for the expression level of ITGAM, $m_3$ is a scaling factor for the expression level of PSTPIP2, $m_4$ is a scaling factor for the expression level of LY6E, $m_5$ is a scaling factor for the expression level of IRF9, and $m_6$ is a scaling factor for the expression level of ABL1. In some examples, *b*, $m_1$, $m_2$, $m_3$, $m_4$, $m_5$ and/or $m_6$ are derived from the logistic regression model used to produce the algorithm.

In the methods disclosed herein, determining a bacterial infection score based on the expression levels of RUNX1, ITGAM, PSTPIP2, LY6E and IRF9 can comprise inputting the expression levels or the normalized expression levels of RUNX1, ITGAM, PSTPIP2, LY6E, IRF9 and optionally ABL1, into an algorithm that determines the bacterial infection score. As described herein, the normalized expression levels of RUNX1, ITGAM, PSTPIP2, LY6E and IRF9 can be obtained by normalizing the expression levels of RUNX1, ITGAM, PSTPIP2, LY6E and IRF9 to the expression level of ABL1. The algorithm can be a product of a logistic regression model generated using the expression levels of RUNX1, ITGAM, PSTPIP2, LY6E, IRF9 and optionally ABL1, measured in a training set of biological samples, wherein the training set of biological samples comprises: i) a plurality of biological samples isolated from subjects identified as having a bacterial infection; ii) a plurality of biological samples isolated from subjects identified as having a viral infection; iii) a plurality of biological samples isolated from subjects identified has having both a bacterial infection and a viral infection; iv) a plurality of biological samples isolated from subjects identified as having a non-infectious cause of fever; and v) a plurality of biological samples isolated from healthy subjects not having a bacterial infection, viral infection or non-infectious cause of fever.

In some aspects of the methods disclosed herein, the algorithm that determines the bacterial infection score is:

$$p = \frac{1}{1+e^{-(b+ m_1*RUNX1+ m_2*ITGAM + m_3*PSTPIP2+ m_4*LY6E+ m_5*IRF9)}},$$

wherein *RUNX1* is the expression level or the normalized expression level of RUNX1 in the biological sample from the subject,

*ITGAM* is the expression level or the normalized expression level of ITGAM in the biological sample from the subject,
*PSTPIP2* is the expression level or the normalized expression level of PSTPIP2 in the biological sample from the subject,
*LY6E* is the expression level or the normalized expression level of LY6E in the biological sample from the subject,
*IRF9* is the expression level or the normalized expression level of IRF9 in the biological sample from the subject, and
$b$ is an intercept, $m_1$ is a scaling factor for the expression level of RUNX, $m_2$ is a scaling factor for the expression level of ITGAM, $m_3$ is a scaling factor for the expression level of PSTPIP2, $m_4$ is a scaling factor for the expression level of LY6E, $m_5$ is a scaling factor for the expression level of IRF9.

In some aspects of the methods disclosed herein, the algorithm that determines the bacterial infection score is

$$p = \frac{1}{1+e^{-(b+ m_1*RUNX1+ m_2*ITGAM + m_3*PSTPIP2+ m_4*LY6E+ m_5*IRF9+ m_6*ABL1)}}.$$

wherein *RUNX1* is the expression level of RUNX1 in the biological sample from the subject,
*ITGAM* is the expression level of ITGAM in the biological sample from the subject,
*PSTPIP2* is the expression level of PSTPIP2 in the biological sample from the subject,
*LY6E* is the expression level of LY6E in the biological sample from the subject,
*IRF9* is the expression level of IRF9 in the biological sample from the subject,
*ABL1* is the expression level of ABL1 in the biological sample from the subject, and
$b$ is an intercept, $m_1$ is a scaling factor for the expression level of RUNX, $m_2$ is a scaling factor for the expression level of ITGAM, $m_3$ is a scaling factor for the expression level of PSTPIP2, $m_4$ is a scaling factor for the expression level of LY6E, $m_5$ is a scaling factor for the expression level of IRF9, and $m_6$ is a scaling factor for the expression level of ABL1. $b$, $m_1$, $m_2$, $m_3$, $m_4$, $m_5$ and/or $m_6$ can be derived from the logistic regression model used to produce the algorithm.

As described herein, the algorithm can be a product of a logistic regression model generated using the expression levels of RUNX1, ITGAM, PSTPIP2, LY6E, IRF9 and optionally ABL1, measured in a training set of biological samples. The training set typically comprises at least 200 unique biological samples. A bacterial infection in the subjects or training set herein can be characterized by infection with *Acinetobacter, Aerococcus, Bacillus, Bacteriodes, Borrelia, Clostridium, Enterobacter, Enterococcus, Escherichia, Klebsiella, Neisseria, Pseudomonas, Serratia, Staphylococcus, Streptococcus* or any combination thereof. A non-infectious cause of fever in the subjects or training set herein can be Hypersensitivity Pneuomonitis, immune response to IgG treatment, lupus, Metastatic Pancreatic Cancer, traumatic injury, NSTEMI (Non-ST-elevation myocardial infarction), polytrauma, poor GI motility, post-operative fever, pulmonary embolism, rheumatic fever, sarcoidosis, Sickle Cell Pain Crisis, Systemic inflammatory response syndrome (SIRS), Transaminitis, ureterovesical junction stone or any combination thereof. A viral infection in the subjects or training set herein can be characterized by infection with astrovirus, coronavirus, multisystem inflammatory syndrome in children (MIS-C), dengue, influenza, influenza A, influenza B, metapneumovirus, rhinovirus, RSV, Zika or any combination thereof.

In some aspects of the methods disclosed herein, the method can further comprise f) identifying a specific pathogenic viral infection, a specific pathogenic bacterial infection, or a specific pathogenic non-infectious cause of fever in the subject. Identifying the specific pathogenic viral infection or specific pathogenic bacterial infection in the subject can comprise: i) comparing the viral infection score and bacterial infection score to a library of predetermined cutoff values characterized by specific pathogenic infections; and ii) determining: the specific pathogenic viral infection when the viral infection score is greater than or equal to the predetermined cutoff value characterized by the specific pathogenic infection, or the specific pathogenic bacterial infection when the bacterial infection score is greater than or equal to the predetermined cutoff value characterized by the specific pathogenic infection.

In other instances, identifying the specific pathogenic viral infection or specific pathogenic bacterial infection in the subject can comprise assaying for a specific pathogenic viral infection signature or a specific pathogenic bacterial infection signature. Assaying for the specific pathogenic viral infection signature or the specific pathogenic bacterial infection signature can include performing a test selected from one or more of PCR, RT-PCR, qPCR, ELISA, immunoassay, flow cytometry, hematology parameter signature, or lateral flow assay. For example, assaying for a specific pathogenic viral infection signature or the specific pathogenic bacterial infection signature can comprise: i) determining a specific pathogenic viral infection score or a specific pathogenic bacterial infection score based on the expression levels of at least two biomarkers selected from RUNX1, ITGAM, PSTPIP2, LY6E, IRF9, and ABL1; ii) comparing the specific pathogenic viral infection score and the specific pathogenic bacterial infection score to a library of predetermined cutoff values characterized by specific pathogenic infections; and iii) determining the specific pathogenic viral infection when the specific pathogenic viral infection score is greater than or equal to the predetermined cutoff value characterized by the specific pathogenic infection, or the specific pathogenic bacterial infection when the specific pathogenic bacterial infection score is greater than or equal to the predetermined cutoff value characterized by the specific pathogenic infection.

Determining the specific pathogenic viral infection score and the specific pathogenic bacterial infection score can comprise inputting the expression levels of RUNX1, ITGAM, PSTPIP2, LY6E, IRF9 and optionally ABL1, into an algorithm that determines the specific pathogenic viral infection score or the specific pathogenic bacterial infection score. The algorithm can be a product of a logistic regression model generated using the expression levels of RUNX1, ITGAM, PSTPIP2, LY6E, IRF9 and optionally ABL1, measured in a training set of biological samples, wherein the training set of biological samples comprises: i) a plurality of biological samples isolated from subjects identified as having a specific pathogenic bacterial infection selected from *Acinetobacter, Aerococcus, Bacillus, Bacteriodes, Borrelia, Clostridium, Enterobacter, Enterococcus, Escherichia, Klebsiella, Neisseria, Pseudomonas, Serratia, Staphylococcus, Streptococcus* or any combination thereof; ii) a plurality of biological samples isolated from subjects identified as having a specific pathogenic viral infection selected from astrovirus, coronavirus, multisystem inflammatory syndrome in children (MIS-C), dengue, influenza, influenza A, influenza B, metapneumovirus, rhinovirus, RSV, Zika or any combination thereof; or iii) a plurality of biological samples isolated from subjects identified has having both a specific pathogenic bacterial infection and a specific pathogenic viral infection.

In the methods of identifying the specific pathogenic viral infection or the specific pathogenic bacterial infection in the subject, the subject can be identified as having a specific pathogenic bacterial infection caused by *Acinetobacter, Aerococcus, Bacillus, Bacteriodes, Borrelia, Clostridium, Enterobacter, Enterococcus, Escherichia, Klebsiella, Mycobacterium, Neisseria, Pseudomonas, Serratia, Staphylococcus, Streptococcus* or any combination thereof. For example, the subject can be identified as having a specific pathogenic bacterial infection caused by *Borrelia burgdorferi, Borrelia mayonii, Mycobacterium tuberculosis,* or any combination thereof. The subject can be identified as having a specific pathogenic viral infection caused by astrovirus, coronavirus, multisystem inflammatory syndrome in children (MIS-C), dengue, influenza, influenza A, influenza B, metapneumovirus, rhinovirus, RSV, Zika or any combination thereof. For example, the subject can be identified as having a specific pathogenic viral infection caused by RSV, influenza, SAR-CoV-2 infection, or any combination thereof. In other examples, the subject with a specific pathogenic viral infection can be identified as having multisystem inflammatory syndrome in children (MIS-C). The subject can be identified as having a specific pathogenic non-infectious cause of fever caused by Hypersensitivity Pneuomonitis, immune response to IgG treatment, lupus, Metastatic Pancreatic Cancer, traumatic injury, NSTEMI (Non-ST-elevation myocardial infarction), polytrauma, poor GI motility, post-operative fever, pulmonary embolism, rheumatic fever, sarcoidosis, Sickle Cell Pain Crisis, Systemic inflammatory response syndrome (SIRS), Transaminitis, ureterovesical junction stone or any combination thereof.

In some aspects of the methods disclosed herein, the methods comprise providing a diagnostic or treatment recommendation based on the determined infection. In some examples, the diagnostic or treatment recommendation is from a clinical decision support system. The diagnostic or treatment recommendation can be selected based on the infection status of the patient, the patient clinical history, the patient demography, algorithms, nature and severity of the infection, or a combination thereof. In some examples, the diagnostic or treatment recommendation is selected from one or more of administration of an antibiotics, administration of an anti-viral medication, lab test, a procedure, an exam, prescription, referral, scheduling of an appointment, discharge, lifestyle suggestions, health monitoring, invasive monitoring, sedation, mechanical ventilation, intensive care admission, surgical intervention, drug of last resort, hospital admittance, or a combination thereof. For example, the diagnostic or treatment recommendation comprises administering an antiviral to an individual diagnosed with a viral infection, administering an antibiotic to an individual diagnosed with a bacterial infection, or administering an antipyretic therapy to an individual diagnosed with a non-infectious cause of fever.

In some aspects of the methods disclosed herein, the methods can further comprise determining the expression level of IFI27 in the biological sample from the subject; and determining the viral infection score and the bacterial infection score based on the expression levels of RUNX1, ITGAM, PSTPIP2, LY6E, IRF9, and IFI27.

Kits for determining if a subject has a bacterial infection, a viral infection, or a non-infectious cause of fever in a sample from a human subject are disclosed herein but are not claimed. The kits can comprise (a) a first primer pair for detecting a RUNX1 gene, wherein the first primer pair comprises a RUNX1_forward primer and a RUNX1_reverse primer; (b) a second primer pair for detecting an ITGAM gene, wherein the second primer pair comprises an ITGAM_forward primer and an ITGAM_reverse primer, (c) a third primer pair for detecting a PSTPIP2 gene, wherein the third primer pair comprises a PSTPIP2_forward primer and a PSTPIP2_reverse primer; (d) a fourth primer pair for detecting a LY6E gene, wherein the fourth primer pair comprises a LY6E _forward primer and a LY6E_reverse primer, (e) a fifth primer pair for detecting an IRF9 gene, wherein the fifth primer pair comprises an IRF9_forward primer and an IRF9_reverse primer, and (f) a sixth primer pair for detecting an ABL1 gene, wherein the sixth primer pair comprises an ABL1_forward primer and an ABL1_reverse primer. A kit may further comprise (a) a probe for detecting the RUNX1 gene; (b) a probe for detecting the ITGAM gene; (c) a probe for detecting the PSTPIP2 gene; (d) a probe for detecting the LY6E gene; (e) a probe for detecting the IRF9 gene; and (f) a probe for detecting the ABL1 gene.

The kits can further comprise a primer pair for detecting an exogenous control and/or an endogenous control, wherein the exogenous control is a sample processing control, and wherein the endogenous control is a sample adequacy control. The kits can further comprise an exogenous control probe and/or endogenous control probe. In some embodiments, at least one primer or probe is detectably labeled.

Any of the above aspects or embodiments, or any other aspect or embodiment described herein, can be combined with any other aspect or embodiment described herein.

Unless otherwise defined, all technical and scientific terms used herein have the same meaning as commonly understood by one of ordinary skill in the art to which this disclosure belongs. In the specification, the singular forms also include the plural unless the context clearly dictates otherwise; as examples, the terms "a," "an," and "the" are understood to be singular or plural and the term "or" is understood to be inclusive. By way of example, "an element" means one or more element. Throughout the specification the word "comprising," or variations such as "comprises" or "comprising," will be understood to imply the inclusion of a stated element, integer or step, or group of elements, integers or steps, but not the exclusion of any other element, integer or step, or group of elements, integers or steps. About can be understood as within 10%, 9%, 8%, 7%, 6%, 5%, 4%, 3%, 2%, 1%, 0.5%, 0.1%, 0.05%, or 0.01% of the stated value. Unless otherwise clear from the context, all numerical values provided herein are modified by the term "about." Unless specifically stated or obvious from context, as used herein, the term "or" is understood to be inclusive and covers both "or" and "and".

Although methods and materials similar or equivalent to those described herein can be used in the practice or testing of the present disclosure, suitable methods and materials are described below. The references cited herein are not admitted to be prior art to the claimed invention. In the case of conflict, the present specification, including definitions, will control. In addition, the materials, methods, and examples are illustrative only and are not intended to be limiting. Other features and advantages of the disclosure will be apparent from the following detailed description and claim.

## BRIEF DESCRIPTION OF THE DRAWINGS

The above and further features will be more clearly appreciated from the following detailed description when taken in conjunction with the accompanying drawings.

FIG. 1 shows a boxplot of the measured expression levels, expressed as a cycle threshold (Ct) value, of ABL1 (a reference gene) vs. sample preservative type and infection status.

FIG. 2 shows a boxplot of the measured expression levels, expressed as a delta cycle threshold (dCt) value, of LY6E vs. sample preservative type and infection status.

FIG. 3 shows a boxplot of the measured expression levels, expressed as a dCt value, of IRF9 vs. sample preservative type and infection status.

FIG. 4 shows a boxplot of the measured expression levels, expressed as a dCt value, of RUNX1 vs. sample preservative type and infection status.

FIG. 5 shows a boxplot of the measured expression levels expressed as a dCt value, of PSTPIP2 vs. sample preservative type and infection status.

FIG. 6 shows a boxplot of the measured expression levels, expressed as a dCt value, of ITGAM vs. sample preservative type and infection status.

FIG. 7 shows a boxplot of the measured expression levels, expressed as a cycle threshold (Ct) value, and change in cycle threshold (Delta Ct) value, of LY6E vs. bacterial infection status.

FIG. 8 shows a boxplot of the measured expression levels, expressed as a cycle threshold (Ct) value, and change in cycle threshold (Delta Ct) value, of RUNX1 vs. bacterial infection status.

FIG. 9 shows a boxplot of the measured expression levels, expressed as a cycle threshold (Ct) value, and change in cycle threshold (Delta Ct) value, of IRF9 vs. bacterial infection status.

FIG. 10 shows a boxplot of the measured expression levels, expressed as a cycle threshold (Ct) value, and change in cycle threshold (Delta Ct) value, of ITGAM vs. bacterial infection status.

FIG. 11 shows a boxplot of the measured expression levels, expressed as a cycle threshold (Ct) value, and change in cycle threshold (Delta Ct) value, of PSTPIP2 vs. bacterial infection status.

FIG. 12 shows a boxplot of the SARS-CoV-2 yes/no model vs. viral infection status.

FIG. 13 shows a boxplot of the measured expression levels, expressed as a cycle threshold (Ct) value, of ABL1 vs. infection status.

FIG. 14 shows a boxplot of the measured expression levels, expressed as a cycle threshold (Ct) value (left), and change in cycle threshold (Delta Ct) value (right), of RUNX1 vs. infection status.

FIG. 15 shows a boxplot of the measured expression levels, expressed as a cycle threshold (Ct) value (left), and change in cycle threshold (Delta Ct) value (right), of LY6E vs. infection status.

FIG. 16 shows a boxplot of the measured expression levels, expressed as a cycle threshold (Ct) value (left), and change in cycle threshold (Delta Ct) value (right), of IRF9 vs. infection status.

FIG. 17 shows a boxplot of the measured expression levels, expressed as a cycle threshold (Ct) value (left), and change in cycle threshold (Delta Ct) value (right), of ITGAM vs. infection status.

FIG. 18 shows a boxplot of the measured expression levels, expressed as a cycle threshold (Ct) value (left), and change in cycle threshold (Delta Ct) value (right), of PSTPIP2 vs. infection status.

FIG. 19 shows a boxplot of the bacterial y/n model vs. infection status. The bacterial prediction model had an AUCROC of 0.96 using re-substitution.

FIG. 20 shows a boxplot of the measured expression levels, expressed as a cycle threshold (Ct) value, of ABL1 vs. healthy/viral/bacterial/MIS-C/other infection status.

FIG. 21 shows a boxplot of the measured expression levels, expressed as a cycle threshold (Ct) value (top), and change in cycle threshold (Delta Ct) value (bottom), of RUNX1 vs. healthy/viral/bacterial/MIS-C/other infection status.

FIG. 22 shows a boxplot of the measured expression levels, expressed as a cycle threshold (Ct) value (top), and change in cycle threshold (Delta Ct) value (bottom), of LY6E vs. healthy/viral/bacterial/MIS-C/other infection status.

FIG. 23 shows a boxplot of the measured expression levels, expressed as a cycle threshold (Ct) value (top), and change in cycle threshold (Delta Ct) value (bottom), of IRF9 vs. healthy/viral/bacterial/MIS-C/other infection status.

FIG. 24 shows a boxplot of the measured expression levels, expressed as a cycle threshold (Ct) value (top), and change in cycle threshold (Delta Ct) value (bottom), of ITGAM vs. healthy/viral/bacterial/MIS-C/other infection status.

FIG. 25 shows a boxplot of the measured expression levels, expressed as a cycle threshold (Ct) value (top), and change in cycle threshold (Delta Ct) value (bottom), of PSTPIP2 vs. healthy/viral/bacterial/NHS-C/other infection status.

FIG. 26 shows nominal logistic fits for a bacterial y/n model, viral y/n model, and MIS-C y/n model. The bacterial prediction model had an AUCROC of 0.784 using re-substitution. The viral prediction model had an AUCROC of 0.869 using re-substitution. The MIS-C prediction model had an AUCROC of 0.868 using re-substitution.

FIG. 27 shows a boxplot of the measured expression levels, expressed as a cycle threshold (Ct) value, of ABL1 vs. sample preservative and infection status. Grey is Borrelia.

FIG. 28 shows a boxplot of the measured expression levels, expressed as a cycle threshold (Ct) value, of IRF9 vs. sample preservative and infection status. Grey is Borrelia.

FIG. 29 shows a boxplot of the Borrelia yes/no model determined using nominal logistic fit The Borrelia prediction model had an AUC of 0.99127.

## DETAILED DESCRIPTION

The present disclosure provides methods and kits for the identification and/or treatment of bacterial infections, viral infections and/or non-infectious causes of fever in a subject in need thereof.

Methods

The present disclosure provides methods of determining if a subject has a bacterial infection, a viral infection, or a non-infectious cause of fever, the method comprising: a) determining the expression levels of RUNX1, ITGAM, PSTPIP2, LY6E, IRF9 and ABL1 in a biological sample from the subject; b) determining that the expression level of ABL1 is greater than a first predetermined cutoff value; c) determining a viral infection score and a bacterial infection score based on the expression levels of RUNX1, ITGAM, PSTPIP2, LY6E and IRF9; d) comparing the viral infection score to a second predetermined cutoff value and the bacterial infection score to a third predetermined cutoff value; and e) determining: that the subject has a viral infection when the viral infection score is greater than the second predetermined cutoff value or that the subject does not have viral infection when the viral infection score is less than or equal to the second predetermined cutoff value, that the subject has a bacterial infection when the bacterial infection score is greater than the third predetermined cutoff value or the subject does not have a bacterial infection when the bacterial infection score is less than or equal to the third predetermined cutoff value, and that the subject has a non-infectious cause of fever in the subject when the viral infection score is less than or equal to the second predetermined cutoff value and the bacterial infection score is less than or equal to the third predetermined cutoff value.

The present disclosure provides methods of determining if a subject has a bacterial infection, a viral infection, or a non-infectious cause of fever, the method comprising: a) determining the expression levels of RUNX1, ITGAM, PSTPIP2, LY6E and IRF9 in a biological sample from the subject; b) determining a viral infection score and a bacterial infection score based on the expression levels of RUNX1, ITGAM, PSTPIP2, LY6E and IRF9; c) comparing the viral infection score to a second predetermined cutoff value and the bacterial infection score to a third predetermined cutoff value; and d) determining: that the subject has a viral infection when the viral infection score is greater than the second predetermined cutoff value or that the subject does not have viral infection when the viral infection score is less than or equal to the second predetermined cutoff value, that the subject has a bacterial infection when the bacterial infection score is greater than the third predetermined cutoff value or the subject does not have a bacterial infection when the bacterial infection score is less than or equal to the third predetermined cutoff value, and that the subject has a non-infectious cause of fever in the subject when the viral infection score is less than or equal to the second predetermined cutoff value and the bacterial infection score is less than or equal to the third predetermined cutoff value.

Disclosed but not claimed are methods of treating a bacterial infection, a viral infection or a non-infectious cause of fever in a subject in need thereof, the method comprising: a) determining the expression levels of RUNX1, ITGAM, PSTPIP2, LY6E, IRF9 and ABL1 in a biological sample from the subject; b) determining that the expression level of ABL1 is greater than a first predetermined cutoff value; c) determining a viral infection score and a bacterial infection score based on the expression levels of RUNX1, ITGAM, PSTPIP2, LY6E and IRF9; d) comparing the viral infection score to a second predetermined cutoff value and the bacterial infection score to a third predetermined cutoff value; e) determining: that the subject has a viral infection when the viral infection score is greater than the second predetermined cutoff value or that the subject does not have viral infection when the viral infection score is less than or equal to the second predetermined cutoff value, that the subject has a bacterial infection when the bacterial infection score is greater than the third predetermined cutoff value or the subject does not have a bacterial infection when the bacterial infection score is less than or equal to the third predetermined cutoff value, and that the subject has a non-infectious cause of fever in the subject when the viral infection score is less than or equal to the second predetermined cutoff value and the bacterial infection score is less than or equal to the third predetermined cutoff value; and f) administering to the subject: at least one antiviral therapy when the subject is identified to have a viral infection, at least one antibiotic therapy when the subject is identified to have a bacterial infection, and at least one antipyretic therapy when the subject is identified to have a non-infectious cause of fever.

Disclosed but not claimed are methods of treating a bacterial infection, a viral infection or a non-infectious cause of fever in a subject in need thereof, the method comprising: a) determining the expression levels of RUNX1, ITGAM, PSTPIP2, LY6E and IRF9 in a biological sample from the subject; b) determining a viral infection score and a bacterial infection score based on the expression levels of RUNX1, ITGAM, PSTPIP2, LY6E and IRF9; c) comparing the viral infection score to a second predetermined cutoff value and the bacterial infection score to a third predetermined cutoff value; d) determining: that the subject has a viral infection when the viral infection score is greater than the second predetermined cutoff value or that the subject does not have viral infection when the viral infection score is less than or equal to the second predetermined cutoff value, that the subject has a bacterial infection when the bacterial infection score is greater than the third predetermined cutoff value or the subject does not have a bacterial infection when the bacterial infection score is less than or equal to the third predetermined cutoff value, and that the subject has a non-infectious cause of fever in the subject when the viral infection score is less than or equal to the second predetermined cutoff value and the bacterial infection score is less than or equal to the third predetermined cutoff value; and e) administering to the subject: at least one antiviral therapy when the subject is identified to have a viral infection, at least one antibiotic therapy when the subject is identified to have a bacterial infection, and at least one antipyretic therapy when the subject is identified to have a non-infectious cause of fever.

The present disclosure provides methods of determining if a subject has a bacterial infection, a viral infection, or a non-infectious cause of fever, the method comprising: a) determining the expression levels of at least one of, or at least two of, or at least three of, or at least four of, or each of RUNX1, ITGAM, PSTPIP2, LY6E, IRF9 in a biological sample from the subject; b) determining the expression level of ABL1 in the biological sample from the subject; c) determining that the expression level of ABL1 is greater than a first predetermined cutoff value; d) determining a viral infection score and a bacterial infection score based on one or more of the expression levels determined in step (a); e) comparing the viral infection score to a second predetermined cutoff value and the bacterial infection score to a third predetermined cutoff value; and f) determining: that the subject has a viral infection when the viral infection score is greater than the second predetermined cutoff value or that the subject does not have viral infection when the viral infection score is less than or equal to the second predetermined cutoff value, that the subject has a bacterial infection when the bacterial infection score is greater than the third predetermined cutoff value or the subject does not have a bacterial infection when the bacterial infection score is less than or equal to the third predetermined cutoff value, and that the subject has a non-infectious cause of fever in the subject when the viral infection score is less than or equal to the second predetermined cutoff value and the bacterial infection score is less than or equal to the third predetermined cutoff value.

Disclosed but not claimed are methods of treating a bacterial infection, a viral infection or a non-infectious cause of fever in a subject in need thereof, the method comprising: a) determining the expression levels of at least one of, or at least two of, or at least three of, or at least four of, or each of RUNX1, ITGAM, PSTPIP2, LY6E, IRF9 in a biological sample from the subject; b) determining the expression level of ABL 1 in the biological sample from the subject; c) determining that the expression level of ABL1 is greater than a first predetermined cutoff value; d) determining a viral infection score and a bacterial infection score based on one or more of the expression levels determined in step (a); e) comparing the viral infection score to a second predetermined cutoff value and the bacterial infection score to a third predetermined cutoff value; f) determining: that the subject has a viral infection when the viral infection score is greater than the second predetermined cutoff value or that the subject does not have viral infection when the viral infection score is less than or equal to the second predetermined cutoff value, that the subject has a bacterial infection when the bacterial infection score is greater than the third predetermined cutoff value or the subject does not have a bacterial infection when the bacterial infection score is less than or equal to the third predetermined cutoff value, and that the subject has a non-infectious cause of fever in the subject when the viral infection score is less than or equal to the second predetermined cutoff value and the bacterial infection score is less than or equal to the third predetermined cutoff value and g) administering to the subject: at least one antiviral therapy when the subject is identified to have a viral infection, at least one antibiotic therapy when the subject is identified to have a bacterial infection, and at least one antipyretic therapy when the subject is identified to have a non-infectious cause of fever.

The present disclosure provides methods of determining if a subject has a bacterial infection, a viral infection, or a non-infectious cause of fever, the method comprising: a) determining the expression levels of RUNX1, ITGAM, PSTPIP2, LY6E, IRF9 and ABL1 in a biological sample from the subject; b) determining that the expression level of ABL1 is greater than a first predetermined cutoff value; c) determining a viral infection score and a bacterial infection score based on the expression levels of RUNX1, ITGAM, PSTPIP2, LY6E, IRF9 and ABL1; d) comparing the viral infection score to a second predetermined cutoff value and the bacterial infection score to a third predetermined cutoff value; and e) determining: that the subject has a viral infection when the viral infection score is greater than the second predetermined cutoff value or that the subject does not have viral infection when the viral infection score is less than or equal to the second predetermined cutoff value, that the subject has a bacterial infection when the bacterial infection score is greater than the third predetermined cutoff value or the subject does not have a bacterial infection when the bacterial infection score is less than or equal to the third predetermined cutoff value, and that the subject has a non-infectious cause of fever in the subject when the viral infection score is less than or equal to the second predetermined cutoff value and the bacterial infection score is less than or equal to the third predetermined cutoff value.

The present disclosure provides methods of determining if a subject has a bacterial infection, a viral infection, or a non-infectious cause of fever, the method comprising: a) determining the expression levels of RUNX1, ITGAM, PSTPIP2, LY6E, IRF9 and ABL1 in a biological sample from the subject; b) determining a viral infection score and a bacterial infection score based on the expression levels of RUNX1, ITGAM, PSTPIP2, LY6E, IRF9 and ABL1; c) comparing the viral infection score to a second predetermined cutoff value and the bacterial infection score to a third predetermined cutoff value; and d) determining: that the subject has a viral infection when the viral infection score is greater than the second predetermined cutoff value or that the subject does not have viral infection when the viral infection score is less than or equal to the second predetermined cutoff value, that the subject has a bacterial infection when the bacterial infection score is greater than the third predetermined cutoff value or the subject does not have a bacterial infection when the bacterial infection score is less than or equal to the third predetermined cutoff value, and that the subject has a non-infectious cause of fever in the subject when the viral infection score is less than or equal to the second predetermined cutoff value and the bacterial infection score is less than or equal to the third predetermined cutoff value.

Disclosed but not claimed are methods of treating a bacterial infection, a viral infection or a non-infectious cause of fever in a subject in need thereof, the method comprising: a) determining the expression levels of RUNX1, ITGAM, PSTPIP2, LY6E, IRF9 and ABL1 in a biological sample from the subject; b) determining that the expression level of ABL1 is greater than a first predetermined cutoff value; c) determining a viral infection score and a bacterial infection score based on the expression levels of RUNX1, ITGAM, PSTPIP2, LY6E, IRF9 and ABL1; d) comparing the viral infection score to a second predetermined cutoff value and the bacterial infection score to a third predetermined cutoff value; e) determining: that the subject has a viral infection when the viral infection score is greater than the second predetermined cutoff value or that the subject does not have viral infection when the viral infection score is less than or equal to the second predetermined cutoff value, that the subject has a bacterial infection when the bacterial infection score is greater than the third predetermined cutoff value or the subject does not have a bacterial infection when the bacterial infection score is less than or equal to the third predetermined cutoff value, and that the subject has a non-infectious cause of fever in the subject when the viral infection score is less than or equal to the second predetermined cutoff value and the bacterial infection score is less than or equal to the third predetermined cutoff value; and f) administering to the subject: at least one antiviral therapy when the subject is identified to have a viral infection, at least one antibiotic therapy when the subject is identified to have a bacterial infection, and at least one antipyretic therapy when the subject is identified to have a non-infectious cause of fever.

Disclosed but not claimed are methods of treating a bacterial infection, a viral infection or a non-infectious cause of fever in a subject in need thereof, the method comprising: a) determining the expression levels of RUNX1, ITGAM, PSTPIP2, LY6E, IRF9 and ABL1 in a biological sample from the subject; b) determining a viral infection score and a bacterial infection score based on the expression levels of RUNX1, ITGAM, PSTPIP2, LY6E, IRF9 and ABL1; c) comparing the viral infection score to a second predetermined cutoff value and the bacterial infection score to a third predetermined cutoff value; d) determining: that the subject has a viral infection when the viral infection score is greater than the second predetermined cutoff value or that the subject does not have viral infection when the viral infection score is less than or equal to the second predetermined cutoff value, that the subject has a bacterial infection when the bacterial infection score is greater than the third predetermined cutoff value or the subject does not have a bacterial infection when the bacterial infection score is less than or equal to the third predetermined cutoff value, and that the subject has a non-infectious cause of fever in the subject when the viral infection score is less than or equal to the second predetermined cutoff value and the bacterial infection score is less than or equal to the third predetermined cutoff value; and e) administering to the subject: at least one antiviral therapy when the subject is identified to have a viral infection, at least one antibiotic therapy when the subject is identified to have a bacterial infection, and at least one antipyretic therapy when the subject is identified to have a non-infectious cause of fever.

The present disclosure provides methods of determining if a subject has a bacterial infection, a viral infection, or a non-infectious cause of fever, the method comprising: a) determining the expression levels of at least one of, at least two of, at least three of, at least four of, at least five of, or each of RUNX1, ITGAM, PSTPIP2, LY6E, IRF9 and ABL1 in a biological sample from the subject; b) determining that the expression level of ABL1 is greater than a first predetermined cutoff value; c) determining a viral infection score and a bacterial infection score based on one or more of the expression levels determined in step (a); d) comparing the viral infection score to a second predetermined cutoff value and the bacterial infection score to a third predetermined cutoff value; and e) determining: that the subject has a viral infection when the viral infection score is greater than the second predetermined cutoff value or that the subject does not have viral infection when the viral infection score is less than or equal to the second predetermined cutoff value, that the subject has a bacterial infection when the bacterial infection score is greater than the third predetermined cutoff value or the subject does not have a bacterial infection when the bacterial infection score is less than or equal to the third predetermined cutoff value, and that the subject has a non-infectious cause of fever in the subject when the viral infection score is less than or equal to the second predetermined cutoff value and the bacterial infection score is less than or equal to the third predetermined cutoff value.

The present disclosure provides methods of determining if a subject has a bacterial infection, a viral infection, or a non-infectious cause of fever, the method comprising: a) determining the expression levels of at least one of, at least two of, at least three of, at least four of, at least five of, or each of RUNX 1, ITGAM, PSTPIP2, LY6E, IRF9 and ABL1 in a biological sample from the subject; b) determining a viral infection score and a bacterial infection score based on one or more of the expression levels determined in step (a); c) comparing the viral infection score to a second predetermined cutoff value and the bacterial infection score to a third predetermined cutoff value; and d) determining: that the subject has a viral infection when the viral infection score is greater than the second predetermined cutoff value or that the subject does not have viral infection when the viral infection score is less than or equal to the second predetermined cutoff value, that the subject has a bacterial infection when the bacterial infection score is greater than the third predetermined cutoff value or the subject does not have a bacterial infection when the bacterial infection score is less than or equal to the third predetermined cutoff value, and that the subject has a non-infectious cause of fever in the subject when the viral infection score is less than or equal to the second predetermined cutoff value and the bacterial infection score is less than or equal to the third predetermined cutoff value.

Disclosed but not claimed are methods of treating a bacterial infection, a viral infection or a non-infectious cause of fever in a subject in need thereof, the method comprising: a) determining the expression levels of at least one of, at least two of, at least three of, at least four of, at least five of, or each of RUNX1, ITGAM, PSTPIP2, LY6E, IRF9 and ABL1 in a biological sample from the subject; b) determining that the expression level of ABL1 is greater than a first predetermined cutoff value; c) determining a viral infection score and a bacterial infection score based on one or more of the expression levels determined in step (a); d) comparing the viral infection score to a second predetermined cutoff value and the bacterial infection score to a third predetermined cutoff value; and e) determining: that the subject has a viral infection when the viral infection score is greater than the second predetermined cutoff value or that the subject does not have viral infection when the viral infection score is less than or equal to the second predetermined cutoff value, that the subject has a bacterial infection when the bacterial infection score is greater than the third predetermined cutoff value or the subject does not have a bacterial infection when the bacterial infection score is less than or equal to the third predetermined cutoff value, and that the subject has a non-infectious cause of fever in the subject when the viral infection score is less than or equal to the second predetermined cutoff value and the bacterial infection score is less than or equal to the third predetermined cutoff value; and f) administering to the subject: at least one antiviral therapy when the subject is identified to have a viral infection, at least one antibiotic therapy when the subject is identified to have a bacterial infection, and at least one antipyretic therapy when the subject is identified to have a non-infectious cause of fever.

Disclosed but not claimed are methods of treating a bacterial infection, a viral infection or a non-infectious cause of fever in a subject in need thereof, the method comprising: a) determining the expression levels of at least one of, at least two of, at least three of, at least four of, at least five of, or each of RUNX1, ITGAM, PSTPIP2, LY6E, IRF9 and ABL1 in a biological sample from the subject; b) determining a viral infection score and a bacterial infection score based on one or more of the expression levels determined in step (a); c) comparing the viral infection score to a second predetermined cutoff value and the bacterial infection score to a third predetermined cutoff value; and d) determining: that the subject has a viral infection when the viral infection score is greater than the second predetermined cutoff value or that the subject does not have viral infection when the viral infection score is less than or equal to the second predetermined cutoff value, that the subject has a bacterial infection when the bacterial infection score is greater than the third predetermined cutoff value or the subject does not have a bacterial infection when the bacterial infection score is less than or equal to the third predetermined cutoff value, and that the subject has a non-infectious cause of fever in the subject when the viral infection score is less than or equal to the second predetermined cutoff value and the bacterial infection score is less than or equal to the third predetermined cutoff value; and e) administering to the subject: at least one antiviral therapy when the subject is identified to have a viral infection, at least one antibiotic therapy when the subject is identified to have a bacterial infection, and at least one antipyretic therapy when the subject is identified to have a non-infectious cause of fever.

The present disclosure provides methods of determining if a subject has a bacterial infection, a viral infection, or a non-infectious cause of fever, the method comprising: a) determining the expression levels of RUNX1, ITGAM, PSTPIP2, LY6E, IRF9 and ABL1 in a biological sample from the subject; b) determining that ABL1 is present in the biological sample based on the expression level of ABL1 measured in step (a); c) determining a viral infection score and a bacterial infection score based on the expression levels of RUNX1, ITGAM, PSTPIP2, LY6E and IRF9; d) comparing the viral infection score to a second predetermined cutoff value and the bacterial infection score to a third predetermined cutoff value; and e) determining: that the subject has a viral infection when the viral infection score is greater than the second predetermined cutoff value or that the subject does not have viral infection when the viral infection score is less than or equal to the second predetermined cutoff value, that the subject has a bacterial infection when the bacterial infection score is greater than the third predetermined cutoff value or the subject does not have a bacterial infection when the bacterial infection score is less than or equal to the third predetermined cutoff value, and that the subject has a non-infectious cause of fever in the subject when the viral infection score is less than or equal to the second predetermined cutoff value and the bacterial infection score is less than or equal to the third predetermined cutoff value.

Disclosed but not claimed are methods of treating a bacterial infection, a viral infection or a non-infectious cause of fever in a subject in need thereof, the method comprising: a) determining the expression levels of RUNX1, ITGAM, PSTPIP2, LY6E, IRF9 and ABL1 in a biological sample from the subject; b) determining that ABL1 is present in the biological sample based on the expression level of ABL1 measured in step (a); c) determining a viral infection score and a bacterial infection score based on the expression levels of RUNX1, ITGAM, PSTPIP2, LY6E and IRF9; d) comparing the viral infection score to a second predetermined cutoff value and the bacterial infection score to a third predetermined cutoff value; and e) determining: that the subject has a viral infection when the viral infection score is greater than the second predetermined cutoff value or that the subject does not have viral infection when the viral infection score is less than or equal to the second predetermined cutoff value, that the subject has a bacterial infection when the bacterial infection score is greater than the third predetermined cutoff value or the subject does not have a bacterial infection when the bacterial infection score is less than or equal to the third predetermined cutoff value, and that the subject has a non-infectious cause of fever in the subject when the viral infection score is less than or equal to the second predetermined cutoff value and the bacterial infection score is less than or equal to the third predetermined cutoff value; and f) administering to the subject: at least one antiviral therapy when the subject is identified to have a viral infection, at least one antibiotic therapy when the subject is identified to have a bacterial infection, and at least one antipyretic therapy when the subject is identified to have a non-infectious cause of fever.

The present disclosure provides methods of determining if a subject has a bacterial infection, a viral infection, or a non-infectious cause of fever, the method comprising: a) determining the expression levels of at least one of, or at least two of, or at least three of, or at least four of, or each of RUNX1, ITGAM, PSTPIP2, LY6E, IRF9 in a biological sample from the subject; b) determining the expression level of ABL1 in the biological sample from the subject; c) determining that ABL1 is present in the biological sample based on the expression level of ABL1 measured in step (b); d) determining a viral infection score and a bacterial infection score based on one or more of the expression levels determined in step (a); e) comparing the viral infection score to a second predetermined cutoff value and the bacterial infection score to a third predetermined cutoff value; and f) determining: that the subject has a viral infection when the viral infection score is greater than the second predetermined cutoff value or that the subject does not have viral infection when the viral infection score is less than or equal to the second predetermined cutoff value, that the subject has a bacterial infection when the bacterial infection score is greater than the third predetermined cutoff value or the subject does not have a bacterial infection when the bacterial infection score is less than or equal to the third predetermined cutoff value, and that the subject has a non-infectious cause of fever in the subject when the viral infection score is less than or equal to the second predetermined cutoff value and the bacterial infection score is less than or equal to the third predetermined cutoff value.

Disclosed but not claimed are methods of treating a bacterial infection, a viral infection or a non-infectious cause of fever in a subject in need thereof, the method comprising: a) determining the expression levels of at least one of, or at least two of, or at least three of, or at least four of, or each of RUNX1, ITGAM, PSTPIP2, LY6E, IRF9 in a biological sample from the subject; b) determining the expression level of ABL1 in the biological sample from the subject; c) determining that ABL1 is present in the biological sample based on the expression level of ABL1 measured in step (b); d) determining a viral infection score and a bacterial infection score based on one or more of the expression levels determined in step (a); e) comparing the viral infection score to a second predetermined cutoff value and the bacterial infection score to a third predetermined cutoff value; and f) determining: that the subject has a viral infection when the viral infection score is greater than the second predetermined cutoff value or that the subject does not have viral infection when the viral infection score is less than or equal to the second predetermined cutoff value, that the subject has a bacterial infection when the bacterial infection score is greater than the third predetermined cutoff value or the subject does not have a bacterial infection when the bacterial infection score is less than or equal to the third predetermined cutoff value, and that the subject has a non-infectious cause of fever in the subject when the viral infection score is less than or equal to the second predetermined cutoff value and the bacterial infection score is less than or equal to the third predetermined cutoff value; and g) administering to the subject: at least one antiviral therapy when the subject is identified to have a viral infection, at least one antibiotic therapy when the subject is identified to have a bacterial infection, and at least one antipyretic therapy when the subject is identified to have a non-infectious cause of fever.

The present disclosure provides methods of determining if a subject has a bacterial infection, a viral infection, or a non-infectious cause of fever, the method comprising: a) determining the expression levels of RUNX1, ITGAM, PSTPIP2, LY6E, IRF9 and ABL1 in a biological sample from the subject; b) determining that ABL1 is present in the biological sample based on the expression level of ABL1 measured in step (a); c) determining a viral infection score and a bacterial infection score based on the expression levels of RUNX1, ITGAM, PSTPIP2, LY6E, IRF9 and ABL1; d) comparing the viral infection score to a second predetermined cutoff value and the bacterial infection score to a third predetermined cutoff value; and e) determining: that the subject has a viral infection when the viral infection score is greater than the second predetermined cutoff value or that the subject does not have viral infection when the viral infection score is less than or equal to the second predetermined cutoff value, that the subject has a bacterial infection when the bacterial infection score is greater than the third predetermined cutoff value or the subject does not have a bacterial infection when the bacterial infection score is less than or equal to the third predetermined cutoff value, and that the subject has a non-infectious cause of fever in the subject when the viral infection score is less than or equal to the second predetermined cutoff value and the bacterial infection score is less than or equal to the third predetermined cutoff value.

Disclosed but not claimed are methods of treating a bacterial infection, a viral infection or a non-infectious cause of fever in a subject in need thereof, the method comprising: a) determining the expression levels of RUNX1, ITGAM, PSTPIP2, LY6E, IRF9 and ABL1 in a biological sample from the subject; b) determining that ABL1 is present in the biological sample based on the expression level of ABL1 measured in step (a); c) determining a viral infection score and a bacterial infection score based on the expression levels of RUNX1, ITGAM, PSTPIP2, LY6E, IRF9 and ABL1; d) comparing the viral infection score to a second predetermined cutoff value and the bacterial infection score to a third predetermined cutoff value; and e) determining: that the subject has a viral infection when the viral infection score is greater than the second predetermined cutoff value or that the subject does not have viral infection when the viral infection score is less than or equal to the second predetermined cutoff value, that the subject has a bacterial infection when the bacterial infection score is greater than the third predetermined cutoff value or the subject does not have a bacterial infection when the bacterial infection score is less than or equal to the third predetermined cutoff value, and that the subject has a non-infectious cause of fever in the subject when the viral infection score is less than or equal to the second predetermined cutoff value and the bacterial infection score is less than or equal to the third predetermined cutoff value; and f) administering to the subject: at least one antiviral therapy when the subject is identified to have a viral infection, at least one antibiotic therapy when the subject is identified to have a bacterial infection, and at least one antipyretic therapy when the subject is identified to have a non-infectious cause of fever.

The present disclosure provides methods of determining if a subject has a bacterial infection, a viral infection, or a non-infectious cause of fever, the method comprising: a) determining the expression levels of at least one of, at least two of, at least three of, at least four of, at least five of, or each of RUNX1, ITGAM, PSTPIP2, LY6E, IRF9 and ABL1 in a biological sample from the subject; b) determining that ABL1 is present in the biological sample based on the expression level of ABL1 measured in step (a); c) determining a viral infection score and a bacterial infection score based on one or more of the expression levels determined in step (a); d) comparing the viral infection score to a second predetermined cutoff value and the bacterial infection score to a third predetermined cutoff value; and e) determining: that the subject has a viral infection when the viral infection score is greater than the second predetermined cutoff value or that the subject does not have viral infection when the viral infection score is less than or equal to the second predetermined cutoff value, that the subject has a bacterial infection when the bacterial infection score is greater than the third predetermined cutoff value or the subject does not have a bacterial infection when the bacterial infection score is less than or equal to the third predetermined cutoff value, and that the subject has a non-infectious cause of fever in the subject when the viral infection score is less than or equal to the second predetermined cutoff value and the bacterial infection score is less than or equal to the third predetermined cutoff value.

Disclosed but not claimed are methods of treating a bacterial infection, a viral infection or a non-infectious cause of fever in a subject in need thereof, the method comprising: a) determining the expression levels of at least one of, at least two of, at least three of, at least four of, at least five of, or each of RUNX 1, ITGAM, PSTPIP2, LY6E, IRF9 and ABL1 in a biological sample from the subject; b) determining that ABL1 is present in the biological sample based on the expression level of ABL1 measured in step (a); c) determining a viral infection score and a bacterial infection score based on one or more of the expression levels determined in step (a); d) comparing the viral infection score to a second predetermined cutoff value and the bacterial infection score to a third predetermined cutoff value; and e) determining: that the subject has a viral infection when the viral infection score is greater than the second predetermined cutoff value or that the subject does not have viral infection when the viral infection score is less than or equal to the second predetermined cutoff value, that the subject has a bacterial infection when the bacterial infection score is greater than the third predetermined cutoff value or the subject does not have a bacterial infection when the bacterial infection score is less than or equal to the third predetermined cutoff value, and that the subject has a non-infectious cause of fever in the subject when the viral infection score is less than or equal to the second predetermined cutoff value and the bacterial infection score is less than or equal to the third predetermined cutoff value, and f) administering to the subject: at least one antiviral therapy when the subject is identified to have a viral infection, at least one antibiotic therapy when the subject is identified to have a bacterial infection, and at least one antipyretic therapy when the subject is identified to have a non-infectious cause of fever.

The present disclosure provides a method of determining if a subject has a bacterial infection, a viral infection or a non-infectious cause of fever the method comprising: a) determining the expression levels of RUNX1, ITGAM, PSTPIP2, LY6E and IRF9 in a biological sample from the subject; and b) determining that the subject has a viral infection, a bacterial infection, both a viral infection and a bacterial infection, or a non-infectious cause of fever based on the expression levels measured in step (a).

The present disclosure provides a method of determining if a subject has a bacterial infection, a viral infection or a non-infectious cause of fever the method comprising: a) determining the expression levels of at least one of, at least two of, at least three of, at least four of, or each of RUNX1, ITGAM, PSTPIP2, LY6E and IRF9 in a biological sample from the subject; and b) determining that the subject has a viral infection, a bacterial infection, both a viral infection and a bacterial infection, or a non-infectious cause of fever based on the expression levels measured in step (a).

The present disclosure provides a method of determining if a subject has a bacterial infection, a viral infection or a non-infectious cause of fever the method comprising: a) determining the expression levels of RUNX1, ITGAM, PSTPIP2, LY6E, IRF9 and ABL1 in a biological sample from the subject; and b) determining that the subject has a viral infection, a bacterial infection, both a viral infection and a bacterial infection, or a non-infectious cause of fever based on the expression levels measured in step (a).

The present disclosure provides a method of determining if a subject has a bacterial infection, a viral infection or a non-infectious cause of fever the method comprising: a) determining the expression levels of at least one of, at least two of, at least three of, at least four of, at least five of, or each of RUNX1, ITGAM, PSTPIP2, LY6E, IRF9 and ABL1 in a biological sample from the subject; and b) determining that the subject has a viral infection, a bacterial infection, both a viral infection and a bacterial infection, or a non-infectious cause of fever based on the expression levels measured in step (a).

For clarity, in methods of the present disclosure that recite a steps of determining a viral infection score and a bacterial infection score based on the expression levels of measured biomarkers; and comparing the viral infection score to a first predetermined cutoff value and the bacterial infection score to a second predetermined cutoff value, the method can also be described with the following steps: i) determining a viral infection score based on the expression levels of the measured biomarker and comparing the viral infection score to a first predetermined cutoff value; and ii) determining a bacterial infection score based on the expression levels of the measured biomarkers and comparing the bacterial infection score to a second predetermined cutoff value. That is, a in a non-limiting example, the present disclosure provides a method of determining if a subject has a bacterial infection, a viral infection, or a non-infectious cause of fever, the method comprising: a) determining the expression levels of RUNX1, ITGAM, PSTPIP2, LY6E and IRF9 in a biological sample from the subject; b) determining a viral infection score based on the expression levels of RUNX1, ITGAM, PSTPIP2, LY6E and IRF9 and comparing the viral infection score a first predetermined cutoff value; c) determining a bacterial infection score based on the expression levels of RUNX1, ITGAM, PSTPIP2, LY6E and IRF9 and comparing the bacterial infection score a second predetermined cutoff value; and d) determining: that the subject has a viral infection when the viral infection score is greater than the first predetermined cutoff value or that the subject does not have viral infection when the viral infection score is less than the first predetermined cutoff value, that the subject has a bacterial infection when the bacterial infection score is greater than the second predetermined cutoff value or the subject does not have a bacterial infection when the bacterial infection score is less than or equal to the second predetermined cutoff value, and that the subject has a non-infectious cause of fever in the subject when the viral infection score is less than the first predetermined cutoff value and the bacterial infection score is less than the second predetermined cutoff value.

That is, the determination of viral infection scores, the determination of bacterial infection scores, and the comparison of these scores to corresponding predetermined cutoff values can be performed in any order and in any combination.

Disclosed but not claimed are methods of treating a bacterial infection, a viral infection or a non-infectious cause of fever in a subject in need thereof, the method comprising administering to the subject: at least one antiviral therapy when the subject is identified to have a viral infection, at least one antibiotic therapy when the subject is identified to have a bacterial infection, and at least one antipyretic therapy when the subject is identified to have a non-infectious cause of fever, wherein the subject is identified has having a viral infection, a bacterial infection or a non-infectious cause of fever using any of the methods described herein.

In some aspects of the preceding methods, the first predetermined cutoff value can be a Ct value that is between about 30 to about 40, or about 32 to about 38, or about 34 to about 36. In some aspects of the preceding methods, the first predetermined cutoff value can be a Ct value that is between about 30, or about 31, or about 32, or about 33, or about 34, or about 35, or about 36, or about 37, or about 38, or about 39, or about 40. In some aspects of the preceding methods, the first predetermined cutoff value can be a Ct value that is about 35. In some aspects of the preceding methods, the first predetermined cutoff value can be a Ct value that is at least about 30, or at least about 31, or at least about 32, or at least about 33, or at least about 34, or at least about 35, or at least about 36, or at least about 37, or at least about 38, or at least about 39, or at least about 40. In some aspects of the preceding methods, the first predetermined cutoff value can be a Ct value that is at least about 35.

In some aspects of the preceding methods, a viral infection score can be a probability that the subject has a viral infection. In some aspects, the probability can be expressed on a scale of 0 to 1. In some aspects, the probability can be expressed on a scale of 0% to 100%.

In some aspects of the preceding methods wherein the viral infection score is expressed as a probability on a scale of 0 to 1, a second predetermined cutoff value can be a value of about 0.25 to about 0.75, or about 0.3 to about 0.7, or about 0.35 to about 0.65, or about 0.4 to about 0.6, or about 0.45 to about 0.55. In some aspects of the preceding methods wherein a viral infection score is expressed as a probability on a scale of 0 to 1, a second predetermined cutoff value can be a value of about at least about 0.3, or at least about 0.35, or at least about 0.4, or at least about 0.45, or at least about 0.5, or at least about 0.55, or at least about 0.6, or at least about 0.65, or at least about 0.7 or at least about 0.75. In some aspects wherein a viral infection score is expressed as a probability on a scale of 0 to 1, a second predetermined cutoff value can be a value of about 0.3, or about 0.35, or about 0.4, or about 0.45, or about 0.5, or about 0.55, or about 0.6, or about 0.65, or about 0.7, or about 0.75.

In some aspects of the preceding methods wherein the viral infection score is expressed as a probability on a scale of 0% to 100%, a second predetermined cutoff value can be a value of about 25% to about 75%, or about 30% to about 70%, or about 35% to about 65%, or about 40% to about 60%, or about 45% to about 55%. In some aspects of the preceding methods wherein a viral infection score is expressed as a probability on a scale of 0% to 100%, a second predetermined cutoff value can be a value of about at least about 30%, or at least about 35%, or at least about 40%, or at least about 45%, or at least about 50%, or at least about 55%, or at least about 60%, or at least about 65%, or at least about 70% or at least about 75%. In some aspects wherein a viral infection score is expressed as a probability on a scale of 0% to 100%, a second predetermined cutoff value can be a value of about 30%, or about 35%, or about 40%, or about 45%, or about 50%, or about 55%, or about 60%, or about 65%, or about 70%, or about 75%.

In some aspects of the preceding methods, a bacterial infection score can be a probability that the subject has a bacterial infection. In some aspects, the probability can be expressed on a scale of 0 to 1. In some aspects, the probability can be expressed on a scale of 0% to 100%.

In some aspects of the preceding methods wherein the bacterial infection score is expressed as a probability on a scale of 0 to 1, a third predetermined cutoff value can be a value of about 0.25 to about 0.75, or about 0.3 to about 0.7, or about 0.35 to about 0.65, or about 0.4 to about 0.6, or about 0.45 to about 0.55. In some aspects of the preceding methods wherein a bacterial infection score is expressed as a probability on a scale of 0 to 1, a third predetermined cutoff value can be a value of about at least about 0.3, or at least about 0.35, or at least about 0.4, or at least about 0.45, or at least about 0.5, or at least about 0.55, or at least about 0.6, or at least about 0.65, or at least about 0.7 or at least about 0.75. In some aspects wherein a bacterial infection score is expressed as a probability on a scale of 0 to 1, a third predetermined cutoff value can be a value of about 0.3, or about 0.35, or about 0.4, or about 0.45, or about 0.5, or about 0.55, or about 0.6, or about 0.65, or about 0.7, or about 0.75.

In some aspects of the preceding methods wherein the bacterial infection score is expressed as a probability on a scale of 0% to 100%, a third predetermined cutoff value can be a value of about 25% to about 75%, or about 30% to about 70%, or about 35% to about 65%, or about 40% to about 60%, or about 45% to about 55%. In some aspects of the preceding methods wherein a bacterial infection score is expressed as a probability on a scale of 0% to 100%, a third predetermined cutoff value can be a value of about at least about 30%, or at least about 35%, or at least about 40%, or at least about 45%, or at least about 50%, or at least about 55%, or at least about 60%, or at least about 65%, or at least about 70% or at least about 75%. In some aspects wherein a bacterial infection score is expressed as a probability on a scale of 0% to 100%, a third predetermined cutoff value can be a value of about 30%, or about 35%, or about 40%, or about 45%, or about 50%, or about 55%, or about 60%, or about 65%, or about 70%, or about 75%.

As would be appreciated by the skilled artisan, the second predetermined cutoff value and/or the third predetermined cutoff value can be generated using area under the receiver operating characteristics curve analysis.

In some aspects of the preceding methods, ABL1 can be used as a positive control biomarker that is indicative of the quality of the sample. Without wishing to be bound by theory, the use of ABL1 as a positive control means that when ABL1 expression is detected at a sufficient level, the sample is deemed to be of high enough quality to continue with analysis, and/or, when ABL1 expression is not detected at a sufficient level, the sample is deemed to be of low quality and further analysis is not performed using that sample. Accordingly, in some aspects, the first predetermined cutoff value in the preceding methods can be a threshold value of measured ABL1 expression that indicates that ABL1 is present in the biological sample. As would be appreciated by the skilled artisan, said threshold value can be derived by the user performing the preceding methods based on the experimental conditions being used to measure the expression levels of the recited biomarkers.

Thus, in some aspects of the methods of the present disclosure, the methods can further comprise determining the expression level of ABL1 in the biological sample and comparing the expression level of ABL1 to a predetermined cutoff value. In these aspects, the conclusions drawn regarding whether the subject has a bacterial infection, a viral infection or a non-infectious cause of fever are determined to be valid only when the expression level of ABL1 is greater that the predetermined cutoff value. Thus, in a non-limiting example, the present disclosure provides a method of determining if a subject has a bacterial infection, a viral infection, or a non-infectious cause of fever, the method comprising: a) determining the expression levels of RUNX1, ITGAM, PSTPIP2, LY6E, IRF9 and ABL1 in a biological sample from the subject; b) determining a viral infection score and a bacterial infection score based on the expression levels of RUNX1, ITGAM, PSTPIP2, LY6E and IRF9; c) comparing the viral infection score to a first predetermined cutoff value and the bacterial infection score to a second predetermined cutoff value; d) comparing the expression level of ABL1 to a third predetermined cutoff value and e) determining: that the subject has a viral infection when the viral infection score is greater than the first predetermined cutoff value and the expression level of ABL1 is greater than the third predetermined cutoff value; that the subject does not have viral infection when the viral infection score is less than the first predetermined cutoff value and the expression level of ABL1 is greater than the third predetermined cutoff value; that the subject has a bacterial infection when the bacterial infection score is greater than the second predetermined cutoff value and the expression level of ABL1 is greater than the third predetermined cutoff value that the subject does not have a bacterial infection when the bacterial infection score is less than or equal to the second predetermined cutoff value and the expression level of ABL1 is greater than the third predetermined cutoff value; and that the subject has a non-infectious cause of fever in the subject when the viral infection score is less than the first predetermined cutoff value, the bacterial infection score is less than the second predetermined cutoff value and the expression level of ABL1 is greater than the third predetermined cutoff value. The use of expression level of ABL1 as a positive control in this way can be applied to any of the methods of the present disclosure.

In some aspects, the preceding methods can identify a subject as having either: a) a viral infection only; b) a bacterial infection only; c) both a viral infection and a bacterial infection; or d) a non-infectious cause of fever.

In some aspects of the preceding methods, a subject can be identified as having only a viral infection. In some aspects, a subject is identified as having only a viral infection when the viral infection score is greater than the second predetermined cutoff value, the bacterial infection score is less than or equal to the third predetermined cutoff value.

In some aspects of the preceding methods, a subject can be identified as having only a bacterial infection. In some aspects, a subject is identified as having only a bacterial infection when the bacterial infection score is greater than the third predetermined cutoff value and the viral infection score is less than or equal to the second predetermined cutoff value.

In some aspects of the preceding methods, a subject can be identified as having both a viral infection and a bacterial infection. In some aspects, a subject is identified as having both a viral infection and a bacterial infection when the viral infection score is greater than the second predetermined cutoff value and the bacterial infection score is greater than the third predetermined cutoff value.

In some aspects of the non-claimed methods disclosed herein, the method can comprise administering to a subject at least one antibiotic therapy and at least one antiviral therapy when the subject is identified as having both a bacterial infection and a viral infection.

In some aspects of the preceding methods, determining a viral infection score based on the expression levels of RUNX1, ITGAM, PSTPIP2, LY6E and IRF9 can comprise inputting the expression levels of RUNX1, ITGAM, PSTPIP2, LY6E and IRF9 into an algorithm that determines the viral infection score.

In some aspects of the preceding methods, determining a viral infection score based on the expression levels of RUNX1, ITGAM, PSTPIP2, LY6E and IRF9 can comprise inputting the normalized expression levels of RUNX1, ITGAM, PSTPIP2, LY6E and IRF9 into an algorithm that determines the viral infection score.

In some aspects, normalized expression levels of RUNX1, ITGAM, PSTPIP2, LY6E and IRF9 can be obtained by normalizing the expression of RUNX1, ITGAM, PSTPIP2, LY6E and IRF9 to the expression level of a control biomarker measured in the biological sample. Accordingly, determining a viral infection score based on the expression levels of RUNX1, ITGAM, PSTPIP2, LY6E and IRF9 can comprise: i) normalizing the expression levels of RUNX1, ITGAM, PSTPIP2, LY6E and IRF9 to the expression level of a control biomarker to obtain normalized expression levels of RUNX1, ITGAM, PSTPIP2, LY6E and IRF9; and ii) inputting the normalized expression levels of RUNX1, ITGAM, PSTPIP2, LY6E and IRF9 into an algorithm that determines the viral infection score.

In some aspects, the control biomarker can be ABL1. Accordingly, determining a viral infection score based on the expression levels of RUNX1, ITGAM, PSTPIP2, LY6E and IRF9 can comprise: i) normalizing the expression levels of RUNX1, ITGAM, PSTPIP2, LY6E and IRF9 to the expression level of ABL1 to obtain normalized expression levels of RUNX1, ITGAM, PSTPIP2, LY6E and IRF9; and ii) inputting the normalized expression levels of RUNX1, ITGAM, PSTPIP2, LY6E and IRF9 into an algorithm that determines the viral infection score.

In some aspects of the preceding methods, determining a viral infection score based on the expression levels of RUNX1, ITGAM, PSTPIP2, LY6E, IRF9 and ABL1 can comprise inputting the expression levels of RUNX1, ITGAM, PSTPIP2, LY6E, IRF9 and ABL1 into an algorithm that determines the viral infection score.

In some aspects of the preceding methods, determining a bacterial infection score based on the expression levels of RUNX1, ITGAM, PSTPIP2, LY6E and IRF9 can comprise inputting the expression levels of RUNX1, ITGAM, PSTPIP2, LY6E and IRF9 into an algorithm that determines the bacterial infection score.

In some aspects of the preceding methods, determining a bacterial infection score based on the expression levels of RUNX1, ITGAM, PSTPIP2, LY6E and IRF9 can comprise inputting the normalized expression levels of RUNX1, ITGAM, PSTPIP2, LY6E and IRF9 into an algorithm that determines the bacterial infection score.

In some aspects, normalized expression levels of RUNX1, ITGAM, PSTPIP2, LY6E and IRF9 can be obtained by normalizing the expression of RUNX1, ITGAM, PSTPIP2, LY6E and IRF9 to the expression level of a control biomarker measured in the biological sample. Accordingly, determining a bacterial infection score based on the expression levels of RUNX1, ITGAM, PSTPIP2, LY6E and IRF9 can comprise: i) normalizing the expression levels of RUNX1, ITGAM, PSTPIP2, LY6E and IRF9 to the expression level of a control biomarker to obtain normalized expression levels of RUNX1, ITGAM, PSTPIP2, LY6E and IRF9; and ii) inputting the normalized expression levels of RUNX1, ITGAM, PSTPIP2, LY6E and IRF9 into an algorithm that determines the bacterial infection score.

In some aspects, the control biomarker can be ABL1. Accordingly, determining a bacterial infection score based on the expression levels of RUNX1, ITGAM, PSTPIP2, LY6E and IRF9 can comprise: i) normalizing the expression levels of RUNX1, ITGAM, PSTPIP2, LY6E and IRF9 to the expression level of ABL1 to obtain normalized expression levels of RUNX1, ITGAM, PSTPIP2, LY6E and IRF9; and ii) inputting the normalized expression levels of RUNX1, ITGAM, PSTPIP2, LY6E and IRF9 into an algorithm that determines the bacterial infection score,

In some aspects of the preceding methods, determining a bacterial infection score based on the expression levels of RUNX1, ITGAM, PSTPIP2, LY6E, IRF9 and ABL1 can comprise inputting the expression levels of RUNX1, ITGAM, PSTPIP2, LY6E, IRF9 and ABL1 into an algorithm that determines the bacterial infection score.

In some aspects, the expression level of a biomarker (e.g. RUNX1, ITGAM, PSTPIP2, LY6E and IRF9) can be normalized to the expression level of a control biomarker (*e.g.* ABL1) by any normalization method known in the art. In a non-limiting example, the expression level of a biomarker can be normalized to the expression level of a control biomarker by dividing the expression level of the biomarker by the expression level of the control biomarker. In another non-limiting example, the expression level of a biomarker can be normalized to the expression level of a control biomarker by subtracting the expression level of the control biomarker from the expression level of the biomarker. In another non-limiting example, the expression level of a biomarker can be normalized to the expression level of a control biomarker by subtracting the expression level of the biomarker from the expression of the control biomarker.

In some aspects, an algorithm that determines the viral infection score can be a product of a logistic regression model generated using the expression levels of RUNX1, ITGAM, PSTPIP2, LY6E and IRF9 measured in a training set of biological samples, wherein the training set of biological samples comprises at least one of, at least two of, at least three of, at least four of, or each of: i) a plurality of biological samples isolated from subjects identified as having a bacterial infection; ii) a plurality of biological samples isolated from subjects identified as having a viral infection; iii) a plurality of biological samples isolated from subjects identified has having both a bacterial infection and a viral infection; iv) a plurality of biological samples isolated from subjects identified as having a non-infectious cause of fever, and v) a plurality of biological samples isolated from healthy subjects not having a bacterial infection, viral infection or non-infectious cause of fever.

In some aspects, an algorithm that determines the viral infection score can be a product of a logistic regression model generated using the expression levels of RUNX1, ITGAM, PSTPIP2, LY6E, IRF9 and ABL1 measured in a training set of biological samples, wherein the training set of biological samples comprises at least one of, at least two of, at least three of, at least four of, or each of: i) a plurality of biological samples isolated from subjects identified as having a bacterial infection; ii) a plurality of biological samples isolated from subjects identified as having a viral infection; iii) a plurality of biological samples isolated from subjects identified has having both a bacterial infection and a viral infection; iv) a plurality of biological samples isolated from subjects identified as having a non-infectious cause of fever, and v) a plurality of biological samples isolated from healthy subjects not having a bacterial infection, viral infection or non-infectious cause of fever.

In some aspects, an algorithm that determines the viral infection score can be a product of a logistic regression model generated using the normalized expression levels of RUNX1, ITGAM, PSTPIP2, LY6E and IRF9 measured in a training set of biological samples, wherein the expression levels of RUNX1, ITGAM, PSTPIP2, LY6E and IRF9 are normalized to the expression level of a control biomarker measured in the training set of biological samples, wherein the training set of biological samples comprises at least one of, at least two of, at least three of, at least four of, or each of: i) a plurality of biological samples isolated from subjects identified as having a bacterial infection; ii) a plurality of biological samples isolated from subjects identified as having a viral infection; iii) a plurality of biological samples isolated from subjects identified has having both a bacterial infection and a viral infection, iv) a plurality of biological samples isolated from subjects identified as having a non-infectious cause of fever; and v) a plurality of biological samples isolated from healthy subjects not having a bacterial infection, viral infection or non-infectious cause of fever. In some aspects the control biomarker can be ABL1.

In some aspects, a logistic regression model used to produce the algorithm that determines a viral infection score can be a product of univariate analysis and/or multivariate analysis of the expression levels of RUNX1, ITGAM, PSTPIP2, LY6E and IRF9 in the training set of biological samples. In some aspects, a logistic regression model used to produce the algorithm that determines a viral infection score can be a product of multivariate analysis of the expression levels of RUNX1, ITGAM, PSTPIP2, LY6E and IRF9 in the training set of biological samples.

In some aspects, a logistic regression model used to produce the algorithm that determines a viral infection score can be a product of univariate analysis and/or multivariate analysis of the normalized expression levels of RUNX1, ITGAM, PSTPIP2, LY6E and IRF9 in the training set of biological samples. In some aspects, a logistic regression model used to produce the algorithm that determines a viral infection score can be a product of multivariate analysis of the normalized expression levels of RUNX1, ITGAM, PSTPIP2, LY6E and IRF9 in the training set of biological samples.

In some aspects, a logistic regression model used to produce the algorithm that determines a viral infection score can be a product of univariate analysis and/or multivariate analysis of the expression levels of RUNX1, ITGAM, PSTPIP2, LY6E, IRF9 and ABL1 in the training set of biological samples. In some aspects, a logistic regression model used to produce the algorithm that determines a viral infection score can be a product of multivariate analysis of the expression levels of RUNX1, ITGAM, PSTPIP2, LY6E, IRF9 and ABL1 in the training set of biological samples.

In some aspects, an algorithm that determines the bacterial infection score can be a product of a logistic regression model generated using the expression levels of RUNX1, ITGAM, PSTPIP2, LY6E and IRF9 measured in a training set of biological samples, wherein the training set of biological samples comprises at least one of, at least two of, at least three of, at least four of, or each of: i) a plurality of biological samples isolated from subjects identified as having a bacterial infection; ii) a plurality of biological samples isolated from subjects identified as having a viral infection; iii) a plurality of biological samples isolated from subjects identified has having both a bacterial infection and a viral infection; iv) a plurality of biological samples isolated from subjects identified as having a non-infectious cause of fever, and v) a plurality of biological samples isolated from healthy subjects not having a bacterial infection, viral infection or non-infectious cause of fever.

In some aspects, an algorithm that determines the bacterial infection score can be a product of a logistic regression model generated using the expression levels of RUNX1. ITGAM, PSTPIP2, LY6E, IRF9 and ABL1 measured in a training set of biological samples, wherein the training set of biological samples comprises at least one of, at least two of, at least three of, at least four of, or each of: i) a plurality of biological samples isolated from subjects identified as having a bacterial infection; ii) a plurality of biological samples isolated from subjects identified as having a viral infection; iii) a plurality of biological samples isolated from subjects identified has having both a bacterial infection and a viral infection; iv) a plurality of biological samples isolated from subjects identified as having a non-infectious cause of fever, and v) a plurality of biological samples isolated from healthy subjects not having a bacterial infection, viral infection or non-infectious cause of fever.

In some aspects, an algorithm that determines the bacterial infection score can be a product of a logistic regression model generated using the normalized expression levels of RUNX1, ITGAM, PSTPIP2, LY6E and IRF9 measured in a training set of biological samples, wherein the expression levels of RUNX1, ITGAM, PSTPIP2, LY6E and IRF9 are normalized to the expression level of a control biomarker measured in the training set of biological samples, wherein the training set of biological samples comprises at least one of, at least two of, at least three of, at least four of, or each of: i) a plurality of biological samples isolated from subjects identified as having a bacterial infection; ii) a plurality of biological samples isolated from subjects identified as having a viral infection; iii) a plurality of biological samples isolated from subjects identified has having both a bacterial infection and a viral infection; iv) a plurality of biological samples isolated from subjects identified as having a non-infectious cause of fever; and v) a plurality of biological samples isolated from healthy subjects not having a bacterial infection, viral infection or non-infectious cause of fever. In some aspects the control biomarker can be ABL1.

In some aspects, a logistic regression model used to produce the algorithm that determines a viral infection score can be a product of univariate analysis and/or multivariate analysis of the expression levels of RUNX1, ITGAM, PSTPIP2, LY6E and IRF9 in the training set of biological samples. In some aspects, a logistic regression model used to produce the algorithm that determines a viral infection score can be a product of multivariate analysis of the expression levels of RUNX1, ITGAM, PSTPIP2, LY6E and IRF9 in the training set of biological samples.

In some aspects, a logistic regression model used to produce the algorithm that determines a bacterial infection score can be a product of univariate analysis and/or multivariate analysis of the normalized expression levels of RUNX1, ITGAM, PSTPIP2, LY6E and IRF9 in the training set of biological samples. In some aspects, a logistic regression model used to produce the algorithm that determines a bacterial infection score can be a product of multivariate analysis of the normalized expression levels of RUNX1, ITGAM, PSTPIP2, LY6E and IRF9 in the training set of biological samples.

In some aspects, a logistic regression model used to produce the algorithm that determines a bacterial infection score can be a product of univariate analysis and/or multivariate analysis of the expression levels of RUNX1, ITGAM, PSTPIP2, LY6E, IRF9 and ABL1 in the training set of biological samples. In some aspects, a logistic regression model used to produce the algorithm that determines a bacterial infection score can be a product of multivariate analysis of the expression levels of RUNX1, ITGAM, PSTPIP2, LY6E, IRF9 and ABL1 in the training set of biological samples.

In some aspects of the preceding methods, determining a viral infection score based on one or more expression levels can comprise inputting the expression levels of the one or more expression levels into an algorithm that determines the viral infection score. In some aspects of the preceding methods, determining a viral infection score based on one or more expression levels can comprise inputting the one or more normalized expression levels into an algorithm that determines the viral infection score. Normalized expression levels can be obtained by normalizing the one or more expression levels to the expression level of a control biomarker measured in the biological sample. In some aspects, the control biomarker can be ABL1.

In some aspects, an algorithm that determines the viral infection score can be a product of a logistic regression model generated using the one or more expression levels measured in a training set of biological samples, wherein the training set of biological samples comprises at least one of, at least two of, at least three of, at least four of, or each of: i) a plurality of biological samples isolated from subjects identified as having a bacterial infection; ii) a plurality of biological samples isolated from subjects identified as having a viral infection; iii) a plurality of biological samples isolated from subjects identified has having both a bacterial infection and a viral infection; iv) a plurality of biological samples isolated from subjects identified as having a non-infectious cause of fever, and v) a plurality of biological samples isolated from healthy subjects not having a bacterial infection, viral infection or non-infectious cause of fever. In some aspects, an algorithm that determines the viral infection score can be a product of a logistic regression model generated using the one or more normalized expression levels measured in a training set of biological samples, wherein the training set of biological samples comprises at least one of, at least two of, at least three of, at least four of, or each of: i) a plurality of biological samples isolated from subjects identified as having a bacterial infection; ii) a plurality of biological samples isolated from subjects identified as having a viral infection; iii) a plurality of biological samples isolated from subjects identified has having both a bacterial infection and a viral infection; iv) a plurality of biological samples isolated from subjects identified as having a non-infectious cause of fever; and v) a plurality of biological samples isolated from healthy subjects not having a bacterial infection, viral infection or non-infectious cause of fever.

In some aspects, a logistic regression model used to produce the algorithm that determines a viral infection score can be a product of univariate analysis and/or multivariate analysis of the one or more expression levels in the training set of biological samples. In some aspects, a logistic regression model used to produce the algorithm that determines a viral infection score can be a product of multivariate analysis of the one or more expression levels in the training set of biological samples. In some aspects, a logistic regression model used to produce the algorithm that determines a viral infection score can be a product of univariate analysis and/or multivariate analysis of the one or more normalized expression levels in the training set of biological samples. In some aspects, a logistic regression model used to produce the algorithm that determines a viral infection score can be a product of multivariate analysis of the one or more normalized expression levels in the training set of biological samples.

In some aspects of the preceding methods, determining a bacterial infection score based on one or more expression levels can comprise inputting the expression levels of the one or more expression levels into an algorithm that determines the bacterial infection score.

In some aspects of the preceding methods, determining a bacterial infection score based on one or more expression levels can comprise inputting the one or more normalized expression levels into an algorithm that determines the bacterial infection score. Normalized expression levels can be obtained by normalizing the one or more expression levels to the expression level of a control biomarker measured in the biological sample. In some aspects, the control biomarker can be ABL1.

In some aspects, an algorithm that determines the bacterial infection score can be a product of a logistic regression model generated using the one or more expression levels measured in a training set of biological samples, wherein the training set of biological samples comprises at least one of, at least two of, at least three of, at least four of, or each of: i) a plurality of biological samples isolated from subjects identified as having a bacterial infection, ii) a plurality of biological samples isolated from subjects identified as having a viral infection; iii) a plurality of biological samples isolated from subjects identified has having both a bacterial infection and a viral infection; iv) a plurality of biological samples isolated from subjects identified as having a non-infectious cause of fever; and v) a plurality of biological samples isolated from healthy subjects not having a bacterial infection, viral infection or non-infectious cause of fever. In some aspects, an algorithm that determines the bacterial infection score can be a product of a logistic regression model generated using the one or more normalized expression levels measured in a training set of biological samples, wherein the training set of biological samples comprises at least one of, at least two of, at least three of, at least four of, or each of: i) a plurality of biological samples isolated from subjects identified as having a bacterial infection; ii) a plurality of biological samples isolated from subjects identified as having a viral infection; iii) a plurality of biological samples isolated from subjects identified has having both a bacterial infection and a viral infection; iv) a plurality of biological samples isolated from subjects identified as having a non-infectious cause of fever; and v) a plurality of biological samples isolated from healthy subjects not having a bacterial infection, viral infection or non-infectious cause of fever.

In some aspects, a logistic regression model used to produce the algorithm that determines a bacterial infection score can be a product of univariate analysis and/or multivariate analysis of the one or more expression levels in the training set of biological samples. In some aspects, a logistic regression model used to produce the algorithm that determines a bacterial infection score can be a product of multivariate analysis of the one or more expression levels in the training set of biological samples. In some aspects, a logistic regression model used to produce the algorithm that determines a bacterial infection score can be a product of univariate analysis and/or multivariate analysis of the one or more normalized expression levels in the training set of biological samples. In some aspects, a logistic regression model used to produce the algorithm that determines a bacterial infection score can be a product of multivariate analysis of the one or more normalized expression levels in the training set of biological samples.

In some aspects of the preceding methods, the logistic regression model used to generate the algorithm that determines a viral infection score is independent from the logistic regression model used to generate the algorithm used to determine a bacterial infection score.

In some aspects of the preceding methods, the one or more expression levels used to determine the viral infection score can be distinct from the one or more expression levels used to determine the bacterial infection score.

In some aspects of the preceding methods, the algorithm that determines the viral infection score can be anyone of:

i)

$$p = \frac{1}{1+e^{-(-17.49 + 0.69*RUNX1 + 0.55*ITGAM + 0.33*PSTPIP2 - 1.09*LY6E - 0.25*IRF9)}},$$

ii)

$$p = \frac{1}{1+e^{-(1.62 + 0.69*RUNX1 + 0.55*ITGAM + 0.33*PSTPIP2 - 1.09*LY6E - 0.25*IRF9)}}, \text{ or}$$

iii)

$$p = \frac{1}{1+e^{-(15.13 + 0.69*RUNX1 + 0.55*ITGAM + 0.33*PSTPIP2 - 1.09*LY6E - 0.25*IRF9)}},$$

wherein *RUNX1* is the expression level of RUNX1 in the biological sample from the subject,
*ITGAM* is the expression level of ITGAM in the biological sample from the subject,
*PSTPIP2* is the expression level of PSTPIP2 in the biological sample from the subject,
*LY6E* is the expression level of LY6E in the biological sample from the subject, and
*IRF9* is the expression level of IRF9 in the biological sample from the subject.

In some aspects of the preceding methods, the algorithm that determines the viral infection score can be anyone of:

i)

$$p = \frac{1}{1+e^{-(-17.49+ 0.69*RUNX1+ 0.55*ITGAM + 0.33*PSTPIP2-1.09*LY6E - 0.25*IRF9)}},$$

ii)

$$p = \frac{1}{1+e^{-(1.62+ 0.69*RUNX1+ 0.55*ITGAM + 0.33*PSTPIP2-1.09*LY6E - 0.25*IRF9)}}; \text{ or}$$

iii)

$$p = \frac{1}{1+e^{-(15.13+ 0.69*RUNX1+ 0.55*ITGAM + 0.33*PSTPIP2-1.09*LY6E - 0.25*IRF9)}},$$

wherein *RUNX1* is the normalized expression level of RUNX1 in the biological sample from the subject,
*ITGAM* is the normalized expression level of ITGAM in the biological sample from the subject,
*PSTPIP2* is the normalized expression level of PSTPIP2 in the biological sample from the subject,
*LY6E* is the normalized expression level of LY6E in the biological sample from the subject, and
*IRF9* is the normalized expression level of IRF9 in the biological sample from the subject.

In some aspects, the normalized expression level of RUNX, ITGAM, PSTPIP2, LY6E and IRF9 can be obtained by normalizing the expression level of RUNX, ITGAM, PSTPIP2, LY6E and IRF9 to the expression level of a control biomarker measured in the sample. In some aspects, the control biomarker can be ABL1. In a non-limiting example, the normalized expression level can be calculated by subtracting the expression level of RUNX, ITGAM, PSTPIP2, LY6E or IRF9 from the expression level of ABL1.

In some aspects of the preceding methods, the algorithm that determines the viral infection score can be:

$$p = \frac{1}{1+e^{-(b+ m_1*RUNX1+ m_2*ITGAM + m_3*PSTPIP2+ m_4*LY6E+ m_5*IRF9)}},$$

wherein *RUNX1* is the expression level of RUNX1 in the biological sample from the subject,
*ITGAM* is the expression level of ITGAM in the biological sample from the subject, *PSTPIP2* is the expression level of PSTPIP2 in the biological sample from the subject,
*LY6E* is the expression level of LY6E in the biological sample from the subject,
*IRF9* is the expression level of IRF9 in the biological sample from the subject, and
*b* is an intercept,
$m_1$ is a scaling factor for the expression level of RUNX,
$m_2$ is a scaling factor for the expression level of ITGAM,
$m_3$ is a scaling factor for the expression level of PSTPIP2,
$m_4$ is a scaling factor for the expression level of LY6E,
$m_5$ is a scaling factor for the expression level of IRF9.

In some aspects, *b*, $m_1$, $m_2$, $m_3$, $m_4$, and $m_5$ are derived from the logistic regression model used to produce the algorithm.

In some aspects of the preceding methods, the algorithm that determines the viral infection score can be:

$$p = \frac{1}{1+e^{-(b+ m_1*RUNX1+ m_2*ITGAM + m_3*PSTPIP2+ m_4*LY6E+ m_5*IRF9)}},$$

wherein *RUNX1* is the normalized expression level of RUNX1 in the biological sample from the subject,
*ITGAM* is the normalized expression level of ITGAM in the biological sample from the subject,
*PSTPIP2* is the normalized expression level of PSTPIP2 in the biological sample from the subject,
*LY6E* is the normalized expression level of LY6E in the biological sample from the subject,
*IRF9* is the normalized expression level of IRF9 in the biological sample from the subject, and
*b* is an intercept,
$m_1$ is a scaling factor for the normalized expression level of RUNX,
$m_2$ is a scaling factor for the normalized expression level of ITGAM,
$m_3$ is a scaling factor for the normalized expression level of PSTPIP2,

$m_4$ is a scaling factor for the normalized expression level of LY6E,
$m_5$ is a scaling factor for the normalized expression level of IRF9.

In some aspects, $b$, $m_1$, $m_2$, $m_3$, $m_4$, and $m_5$ are derived from the logistic regression model used to produce the algorithm. In some aspects, the normalized expression level of RUNX, ITGAM, PSTPIP2, LY6E and IRF9 can be obtained by normalizing the expression level of RUNX, ITGAM, PSTPIP2, LY6E and IRF9 to the expression level of a control biomarker measured in the sample. In some aspects, the control biomarker can be ABL1. In a non-limiting example, the normalized expression level can be calculated by subtracting the expression level of RUNX, ITGAM, PSTPIP2, LY6E or IRF9 from the expression level of ABL1.

In some aspects of the preceding methods, the algorithm that determines the viral infection score can be:

$$p = \frac{1}{1+e^{-(b+ m_1*RUNX1+ m_2*ITGAM + m_3*PSTPIP2+ m_4*LY6E+ m_5*IRF9+ m_6*ABL1)}},$$

wherein *RUNX1* is the expression level of RUNX1 in the biological sample from the subject,
*ITGAM* is the expression level of ITGAM in the biological sample from the subject,
*PSTPIP2* is the expression level of PSTPIP2 in the biological sample from the subject,
*LY6E* is the expression level of LY6E in the biological sample from the subject,
*JRF9* is the expression level of IRF9 in the biological sample from the subject,
*ABL1* is the expression level of ABL1 in the biological sample from the subject, and
*b* is an intercept,
$m_1$ is a scaling factor for the expression level of RUNX,
$m_2$ is a scaling factor for the expression level of ITGAM,
$m_3$ is a scaling factor for the expression level of PSTPIP2,
$m_4$ is a scaling factor for the expression level of LY6E,
$m_5$ is a scaling factor for the expression level of IRF9, and
$m_6$ is a scaling factor for the expression level of ABL1.

In some aspects, $b$, $m_1$, $m_2$, $m_3$, $m_4$, $m_5$ and $m_6$ are derived from the logistic regression model used to produce the algorithm.
In some aspects of the preceding methods, the algorithm that determines the viral infection score can be:

$$p = \frac{1}{1+e^{-(b+ m_1*RUNX1+ m_2*ITGAM + m_3*PSTPIP2+ m_4*LY6E+ m_5*IRF9+ m_6*ABL1)}},$$

wherein *RUNX1* is the normalized expression level of RUNX1 in the biological sample from the subject,
*ITGAM* is the normalized expression level of ITGAM in the biological sample from the subject,
*PSTPIP2* is the normalized expression level of PSTPIP2 in the biological sample from the subject,
*LY6E* is the normalized expression level of LY6E in the biological sample from the subject,
*IRF9* is the normalized expression level of IRF9 in the biological sample from the subject,
*ABL1* is the normalized expression level of ABL1 in the biological sample from the subject, and
*b* is an intercept,
$m_1$ is a scaling factor for the normalized expression level of RUNX,
$m_2$ is a scaling factor for the expression level of ITGAM,
$m_3$ is a scaling factor for the normalized expression level of PSTPIP2,
$m_4$ is a scaling factor for the normalized expression level of LY6E,
$m_5$ is a scaling factor for the normalized expression level of IRF9, and
$m_6$ is a scaling factor for the normalized expression level of ABL1.

In some aspects, $b$, $m_1$, $m_2$, $m_3$, $m_4$, $m_5$ and $m_6$ are derived from the logistic regression model used to produce the algorithm. In some aspects, the normalized expression level of RUNX, ITGAM, PSTPIP2, LY6E, IRF9 and ABL1 can be obtained by normalizing the expression level of RUNX, ITGAM, PSTPIP2, LY6E, IRF9 and ABL1 to the expression level of a control biomarker measured in the sample.
In some aspects of the preceding methods, the algorithm that determines the bacterial infection score can be anyone of:

i)

$$p = \frac{1}{1+e^{-(15.03 - 0.24*RUNX1 - 0.96*ITGAM + 0.46*PSTPIP2 + 1.14*LY6E - 0.80*IRF9)}};$$

ii)

$$p = \frac{1}{1+e^{-(0.97 - 0.24*RUNX1 - 0.96*ITGAM + 0.46*PSTPIP2 + 1.14*LY6E - 0.80*IRF9)}}, \text{ or}$$

iii)

$$p = \frac{1}{1+e^{-(4.82 - 0.24*RUNX1 - 0.96*ITGAM + 0.46*PSTPIP2 + 1.14*LY6E - 0.80*IRF9)}};$$

wherein *RUNX1* is the expression level of RUNX1 in the biological sample from the subject,
*ITGAM* is the expression level of ITGAM in the biological sample from the subject,
*PSTPIP2* is the expression level of PSTPIP2 in the biological sample from the subject,
*LY6E* is the expression level of LY6E in the biological sample from the subject, and
*IRF9* is the expression level of IRF9 in the biological sample from the subject.

In some aspects of the preceding methods, the algorithm that determines the bacterial infection score can be anyone of:

i)

$$p = \frac{1}{1+e^{-(15.03 - 0.24*RUNX1 - 0.96*ITGAM + 0.46*PSTPIP2 + 1.14*LY6E - 0.80*IRF9)}};$$

ii)

$$p = \frac{1}{1+e^{-(0.97 - 0.24*RUNX1 - 0.96*ITGAM + 0.46*PSTPIP2 + 1.14*LY6E - 0.80*IRF9)}}, \text{ or}$$

iii)

$$p = \frac{1}{1+e^{-(4.82 - 0.24*RUNX1 - 0.96*ITGAM + 0.46*PSTPIP2 + 1.14*LY6E - 0.80*IRF9)}};$$

wherein *RUNX1* is the normalized expression level of RUNX1 in the biological sample from the subject,
*ITGAM* is the normalized expression level of ITGAM in the biological sample from the subject,
*PSTPIP2* is the normalized expression level of PSTPIP2 in the biological sample from the subject,
*LY6E* is the normalized expression level of LY6E in the biological sample from the subject, and
*IRF9* is the normalized expression level of IRF9 in the biological sample from the subject.

In some aspects, the normalized expression level of RUNX, ITGAM, PSTPIP2, LY6E and IRF9 can be obtained by normalizing the expression level of RUNX, ITGAM, PSTPIP2, LY6E and IRF9 to the expression level of a control biomarker measured in the sample. In some aspects, the control biomarker can be ABL1. In a non-limiting example, the normalized expression level can be calculated by subtracting the expression level of RUNX, ITGAM, PSTPIP2, LY6E or IRF9 from the expression level of ABL1.
In some aspects of the preceding methods, the algorithm that determines the bacterial infection score can be:

$$p = \frac{1}{1+e^{-(b+ m_1*RUNX1+ m_2*ITGAM + m_3*PSTPIP2+ m_4*LY6E+ m_5*IRF9)}};$$

wherein *RUNX1* is the expression level of RUNX1 in the biological sample from the subject,
*ITGAM* is the expression level of ITGAM in the biological sample from the subject,
*PSTPIP2* is the expression level of PSTPIP2 in the biological sample from the subject,
*LY6E* is the expression level of LY6E in the biological sample from the subject,
IRF9 is the expression level of IRF9 in the biological sample from the subject, and

*b* is an intercept,
$m_1$ is a scaling factor for the expression level of RUNX,
$m_2$ is a scaling factor for the expression level of ITGAM,
$m_3$ is a scaling factor for the expression level of PSTPIP2,
$m_4$ is a scaling factor for the expression level of LY6E,
$m_5$ is a scaling factor for the expression level of IRF9.

In some aspects, *b, $m_1$, $m_2$, $m_3$, $m_4$,* and *$m_5$* are derived from the logistic regression model used to produce the algorithm.

In some aspects of the preceding methods, the algorithm that determines the bacterial infection score can be:

$$p = \frac{1}{1 + e^{-(b + m_1 * RUNX1 + m_2 * ITGAM + m_3 * PSTPIP2 + m_4 * LY6E + m_5 * IRF9)}}.$$

wherein *RUNX1* is the normalized expression level of RUNX1 in the biological sample from the subject,
*ITGAM* is the normalized expression level of ITGAM in the biological sample from the subject,
*PSTPIP2* is the normalized expression level of PSTPIP2 in the biological sample from the subject,
*LY6E* is the normalized expression level of LY6E in the biological sample from the subject,
*IRF9* is the normalized expression level of IRF9 in the biological sample from the subject, and
*b* is an intercept,
$m_1$ is a scaling factor for the normalized expression level of RUNX,
$m_2$ is a scaling factor for the normalized expression level of ITGAM,
$m_J$ is a scaling factor for the normalized expression level of PSTPIP2,
$m_4$ is a scaling factor for the normalized expression level of LY6E,
$m_5$ is a scaling factor for the normalized expression level of IRF9.

In some aspects, *b, $m_1$, $m_2$, $m_3$, $m_4$,* and *$m_5$* are derived from the logistic regression model used to produce the algorithm. In some aspects, the normalized expression level of RUNX, ITGAM, PSTPIP2, LY6E and IRF9 can be obtained by normalizing the expression level of RUNX, ITGAM, PSTPIP2, LY6E and IRF9 to the expression level of a control biomarker measured in the sample. In some aspects, the control biomarker can be ABL1. In a non-limiting example, the normalized expression level can be calculated by subtracting the expression level of RUNX, ITGAM, PSTPIP2, LY6E or IRF9 from the expression level of ABL1.

In some aspects of the preceding methods, the algorithm that determines the bacterial infection score can be:

$$p = \frac{1}{1 + e^{-(b + m_1 * RUNX1 + m_2 * ITGAM + m_3 * PSTPIP2 + m_4 * LY6E + m_5 * IRF9 + m_6 * ABL1)}}.$$

wherein *RUNX1* is the expression level of RUNX1 in the biological sample from the subject,
*ITGAM* is the expression level of ITGAM in the biological sample from the subject,
*PSTPIP2* is the expression level of PSTPIP2 in the biological sample from the subject,
*LY6E* is the expression level of LY6E in the biological sample from the subject,
*IRF9* is the expression level of IRF9 in the biological sample from the subject,
*ABL1* is the expression level of ABL1 in the biological sample from the subject, and
*b* is an intercept,
$m_1$ is a scaling factor for the expression level of RUNX,
$m_2$ is a scaling factor for the expression level of ITGAM,
$m_3$ is a scaling factor for the expression level of PSTPIP2,
$m_4$ is a scaling factor for the expression level of LY6E,
$m_5$ is a scaling factor for the expression level of IRF9, and
$m_6$ is a scaling factor for the expression level of ABL1.

In some aspects, *b, $m_1$, $m_2$, $m_3$, $m_4$, $m_5$* and *$m_6$* are derived from the logistic regression model used to produce the algorithm.

In some aspects of the preceding methods, the algorithm that determines the bacterial infection score can be:

$$p = \frac{1}{1 + e^{-(b + m_1 * RUNX1 + m_2 * ITGAM + m_3 * PSTPIP2 + m_4 * LY6E + m_5 * IRF9 + m_6 * ABL1)}}.$$

wherein *RUNX1* is the normalized expression level of RUNX1 in the biological sample from the subject,
*ITGAM* is the normalized expression level of ITGAM in the biological sample from the subject,
*PSTPIP2* is the normalized expression level of PSTPIP2 in the biological sample from the subject,
*LY6E* is the normalized expression level of LY6E in the biological sample from the subject,
*IRF9* is the normalized expression level of IRF9 in the biological sample from the subject,
*ABL1* is the normalized expression level of ABL1 in the biological sample from the subject, and
*b* is an intercept,
$m_1$ is a scaling factor for the normalized expression level of RUNX,
$m_1$ is a scaling factor for the expression level of ITGAM,
$m_3$ is a scaling factor for the normalized expression level of PSTPIP2,
$m_4$ is a scaling factor for the normalized expression level of LY6E,
$m_5$ is a scaling factor for the normalized expression level of IRF9, and
$m_6$ is a scaling factor for the normalized expression level of ABL1.

In some aspects, $b$, $m_1$, $m_2$, $m_3$, $m_4$, $m_5$ and $m_6$ are derived from the logistic regression model used to produce the algorithm. In some aspects, the normalized expression level of RUNX, ITGAM, PSTPIP2, LY6E, IRF9 and ABL1 can be obtained by normalizing the expression level of RUNX, ITGAM, PSTPIP2, LY6E, IRF9 and ABL1 to the expression level of a control biomarker measured in the sample.

In some aspects, a training set can comprise at least about 25, or at least about 50, or at least about 75, or at least about 100, or at least about 125, or at least about 150, or at least about 175, or at least about 200, or at least about 225, or at least about 250, or at least about 275, or at least about 300 unique biological samples. In some aspects, a training set can comprise at least about 200 unique biological samples.

In some aspects, a training set can comprise about 25, or about 50, or about 75, or about 100, or about 125, or about 150, or about 175, or about 200, or about 225, or about 250, or about 275, or about 300 unique biological samples. In some aspects, the training set can comprise about 200 unique biological samples.

In some aspects of the preceding methods, a bacterial infection (*i.e.* a bacterial infection in a subject identified as having a bacterial infection by the methods described herein and/or a bacterial infections in the samples used in a training set described herein) can be characterized by infection with *Acinetobacter, Aerococcus, Bacillus, Bacteriodes, Barrelia, Clostridium, Enterobacter, Enterococcus, Escherichia, Klebsiella, Neisseria, Pseudomonas, Serratia, Staphylococcus, Streptococcus* or any combination thereof. In some aspects of the preceding methods, a bacterial infection can be characterized by infection with any infectious bacterium known in the art.

In some aspects of the preceding methods, a viral infection (*i.e.* a viral infection in a subject identified as having a viral infection by the methods described herein and/or a viral infections in the samples used in a training set described herein) can be characterized by infection with astrovirus, coronavirus, dengue, influenza, influenza A, influenza B, metapneumovirus, rhinovirus, RSV, Zika or any combination thereof. In other examples, the viral infection can be characterized as multisystem inflammatory syndrome in children (MIS-C). Without wishing to be bound by theory, Yonker L. et al. (J Clin Invest. 2021;131(14):e149633) MIS-C can occur by a mechanism in which viral particles remaining in the gut long after an initial COVID-19 infection travel into the bloodstream. MIS-C, the presence of SARS-CoV-2 in the GI tract may lead to local mucosal inflammation, increased zonulin release, and a subsequent increase in gut permeability allowing SARS-CoV-2 antigens, including the superantigen-like motif of the spike protein, to traffic across mucosal barriers and into the bloodstream. In some aspects of the preceding methods, a viral infection can be characterized by infection with any infectious virus known in the art.

In some aspects of the preceding methods, a non-infectious of cause of fever can be Hypersensitivity Pneuomonitis, immune response to IgG treatment, lupus, Metastatic Pancreatic Cancer, traumatic injury, NSTEMI (Non-ST-elevation myocardial infarction), polytrauma, poor GI motility, post-operative fever, pulmonary embolism, rheumatic fever, sarcoidosis, Sickle Cell Pain Crisis, Systemic inflammatory response syndrome (SIRS), Transaminitis, ureterovesical junction stone or any combination thereof.

In some aspects of the preceding methods, the methods can include identifying a specific pathogenic viral infection, a specific pathogenic bacterial infection, or a specific pathogenic non-infectious cause of fever in the subject. The term "specific" can be used interchangeably with the term "characteristic" and refers to a disorder produced by one or more pathological stimuli which have their origin in a known virus, bacteria, or other known causes. For example, a specific pathogenic viral infection refers to a disorder produced by one or more viruses. A specific pathogenic non-infectious cause of fever refers to fever produced by one or more pathological stimuli which originates from a malignant/neoplastic disorders, a rheumatic/inflammatory disorders, or other miscellaneous disorders.

The method of identifying the specific pathogenic viral infection or specific pathogenic bacterial infection in the subject can include i) comparing the viral infection score and bacterial infection score to a library of predetermined cutoff values characterized by specific pathogenic infections; and ii) determining: a) the specific pathogenic viral infection when the viral infection score is greater than or equal to the predetermined cutoff value characterized by the specific pathogenic infection, or b) the specific pathogenic bacterial infection when the bacterial infection score is greater than or equal to the predetermined cutoff value characterized by the specific pathogenic infection.

In other embodiments, the method of identifying the specific pathogenic viral infection or specific pathogenic bacterial infection in the subject can include assaying for a specific pathogenic viral infection signature or a specific pathogenic bacterial infection signature. Assaying for the specific pathogenic viral infection signature or the specific pathogenic bacterial infection signature can include performing a test selected from one or more of PCR, RT-PCR, qPCR, ELISA, immunoassay, flow cytometry, hematology parameter signature, or lateral flow assay. For example, assaying for a specific pathogenic viral infection signature or the specific pathogenic bacterial infection signature can comprise: i) determining a specific pathogenic viral infection score or a specific pathogenic bacterial infection score based on the expression levels of at least two biomarkers selected from RUNX1, ITGAM, PSTPIP2, LY6E, IRF9, and ABL1; ii) comparing the specific pathogenic viral infection score and the specific pathogenic bacterial infection score to a library of predetermined cutoff values characterized by specific pathogenic infections; and iii) determining a)the specific pathogenic viral infection when the specific pathogenic viral infection score is greater than or equal to the predetermined cutoff value characterized by the specific pathogenic infection, or b) the specific pathogenic bacterial infection when the specific pathogenic bacterial infection score is greater than or equal to the predetermined cutoff value characterized by the specific pathogenic infection.

In some aspects of the methods disclosed herein, determining the specific pathogenic viral infection score and the specific pathogenic bacterial infection score can comprise inputting the expression levels of RUNX1, ITGAM, PSTPIP2, LY6E, IRF9 and optionally ABL1, into an algorithm that determines the specific pathogenic viral infection score or the specific pathogenic bacterial infection score. The algorithm can be a product of a logistic regression model generated using the expression levels of RUNX1, ITGAM, PSTPIP2, LY6E, IRF9 and optionally ABL1, measured in a training set of biological samples. In some examples, the training set of biological samples comprises: i) a plurality of biological samples isolated from subjects identified as having a specific pathogenic bacterial infection selected from *Acinetobacter, Aerococcus, Bacillus, Bacteriodes, Borrelia, Clostridium, Enterobacter, Enterococcus, Escherichia, Klebsiella, Neisseria, Pseudonneurs, Serratia, Staphylococcus, Streptococcus* or any combination thereof; ii) a plurality of biological samples isolated from subjects identified as having a specific pathogenic viral infection selected from astrovirus, coronavirus, multisystem inflammatory syndrome in children (MIS-C), dengue, influenza, influenza A, influenza B, metapneumovirus, rhinovirus, RSV, Zika or any combination thereof, or iii) a plurality of biological samples isolated from subjects identified has having both a specific pathogenic bacterial infection and a specific pathogenic viral infection.

As described herein, the methods can include identifying the specific pathogenic viral infection or the specific pathogenic bacterial infection in the subject. In some embodiments, the subject can be identified as having a specific pathogenic bacterial infection caused by *Acinetobacter, Aerococcus, Bacillus, Bacteriodes, Borrelia, Clostridium, Enterobacter, Enterococcus, Escherichia, Klebstella, Mycobacterium, Neisseria, Pseudomonas, Serratia, Staphylococcus, Streptococcus* or any combination thereof. For example, the subject can be identified as having a specific pathogenic bacterial infection caused by *Borrelia burgdorferi, Borrelia mayonti, Mycobacterium tuberculosis,* or any combination thereof. The subject can be identified as having a specific pathogenic viral infection caused by astrovirus, coronavirus, multisystem inflammatory syndrome in children (MIS-C), dengue, influenza, influenza A, influenza B, metapneumovirus, rhinovirus, RSV, Zika or any combination thereof. For example, the subject can be identified as having a specific pathogenic viral infection caused by RSV, influenza, SAR-CoV-2 infection, or any combination thereof. The subject can be identified as having a specific pathogenic non-infectious cause of fever caused by Hypersensitivity Pneuomonitis, immune response to IgG treatment, lupus, Metastatic Pancreatic Cancer, traumatic injury, NSTEMI (Non-ST-elevation myocardial infarction), polytrauma, poor GI motility, post-operative fever, pulmonary embolism, rheumatic fever, sarcoidosis, Sickle Cell Pain Crisis, Systemic inflammatory response syndrome (SIRS), Transaminitis, ureterovesical junction stone or any combination thereof.

In some aspects of the preceding methods, the methods can include triaging a patient suspected of having a bacterial infection, a viral infection, or a non-infectious cause of fever. The method of triaging a patient can include a) determining if the patient has a bacterial infection, a viral infection, or a non-infectious cause of fever as described herein, and b) providing a diagnostic or treatment recommendation based on the determined infection.

The methods of triaging a patient suspected of having a bacterial infection, a viral infection, or a non-infectious cause of fever. The method of triagingertreating a patient can further include determining one or more additional patient metrics selected from an infection status, clinical history, demography, nature and severity of symptoms, or a combination thereof. For example, the method can include determining an infection status of the patient including the severity of the infection, the characteristic infection, or a combination thereof.

The diagnostic or treatment recommendation in the methods of triaging a patient can be from a clinical decision support system. Clinical decision support systems are described herein. The diagnostic or treatment recommendation can be selected based on the infection status of the patient, the patient clinical history, the patient demography, algorithms, nature and severity of the infection, or a combination thereof. In some examples, the diagnostic or treatment recommendation is selected from one or more of administration of an antibiotics, administration of an anti-viral medication, lab test, a procedure, an exam, prescription, referral, scheduling of an appointment, discharge, lifestyle suggestions, health monitoring, invasive monitoring, sedation, mechanical ventilation, intensive care admission, surgical intervention, drug of last resort, hospital admittance, or a combination thereof. In some examples, the diagnostic or treatment recommendation is administering an antiviral to an individual diagnosed with a viral infection. In some examples, the diagnostic or treatment recommendation is administering an antibiotic to an individual diagnosed with a bacterial infection.

Non-claimed methods can include treating a subject suspected of having a bacterial infection, a viral infection, or a non-infectious cause of fever. The methods of treating a subject can include a) identifying the subject as having a bacterial infection, a viral infection, or a non-infectious cause of fever, based on the expression levels of biomarkers RUNX1, ITGAM, PSTPIP2, LY6E, IRF9 and ABL1 in a biological sample from the subject; b) administering to the subject: i) at least one antiviral therapy when the subject is identified to have a viral infection, ii) at least one antibiotic therapy when the subject is identified to have a bacterial infection, or iii) at least one antipyretic therapy when the subject is identified to have a non-infectious cause of fever. As described herein, the expression level of each biomarker can be measured by performing microarray analysis, polymerase chain reaction (PCR), reverse transcriptase polymerase chain reaction (RT-PCR), or RNA sequencing analysis. In some examples, the biomarkers are RNA biomarkers quantified by PCR.

In some aspects of the preceding methods disclosed herein, the methods can further include identifying the subject as having a bacterial infection, a viral infection, or a non-infectious cause of fever, based on the expression levels of one or more biomarkers in addition to RUNX1, ITGAM, PSTPIP2, LY6E and IRF9 or RUNX1, ITGAM, PSTPIP2, LY6E, IRF9 and ABL1 in a biological sample from the subject. For example, the viral infection score, the bacterial infection score, and/or the non-infectious cause of fever score can be determined based on the expression levels of RUNX1, ITGAM, PSTPIP2, LY6E, IRF9, and IFI27 and optionally ABL1. In a non-limiting example, the methods of treating or determining if a subject has a bacterial infection, a viral infection, or a non-infectious cause of fever can comprise determining the expression levels of RUNX1, ITGAM, PSTPIP2, LY6E, IRF9, IFI27, and ABL1 in a biological sample from the subject and determining a viral infection score and a bacterial infection score based on the expression levels of RUNX1, ITGAM, PSTPIP2, LY6E, IRF9, IFIF27 and ABL1. In another non-limiting example, the methods of determining if a subject has a bacterial infection, a viral infection, or a non-infectious cause of fever can comprise determining the expression levels of RUNX1, ITGAM, PSTPIP2, LY6E, IRF9 and IFI27 in a biological sample from the subject and determining a viral infection score and a bacterial infection score based on the expression levels of RUNX1, ITGAM, PSTPIP2, LY6E, IRF9 and IFIF27. In another non-limiting example, the methods of triaging a patient suspected of having a bacterial infection, a viral infection, or a non-infectious cause of fever in a subject in need thereof can comprise identifying the subject as having a bacterial infection, a viral infection, or a non-infectious cause of fever, based on the expression levels of biomarkers RUNX1, ITGAM, PSTPIP2, LY6E, IRF9, 1FI27, and ABL1 in a biological sample from the subject. In another non-limiting example, the methods of triaging a patient suspected of having a bacterial infection, a viral infection, or a non-infectious cause of fever in a subject in need thereof can comprise identifying the subject as having a bacterial infection, a viral infection, or a non-infectious cause of fever, based on the expression levels of biomarkers RUNX1, ITGAM, PSTPIP2, LY6E, IRF9 and IFI27 in a biological sample from the subject. In another non-limiting example, the methods for identifying a specific pathogenic viral infection or specific pathogenic bacterial infection can comprise determining a specific pathogenic viral infection score or a specific pathogenic bacterial infection score based on the expression levels of at least two biomarkers selected from RUNX1, ITGAM, PSTPIP2, LY6E, IRF9, IFI27, and ABL1. In another non-limiting example, the methods for identifying a specific pathogenic viral infection or specific pathogenic bacterial infection can comprise determining a specific pathogenic viral infection score or a specific pathogenic bacterial infection score based on the expression levels of at least two biomarkers selected from RUNX1, ITGAM, PSTPIP2, LY6E, IRF9 and IFI27. That is, as described herein, the use of the combination of RUNX1, ITGAM, PSTPIP2, LY6E and IRF9, and RUNX1, ITGAM, PSTPIP2, LY6E, IRF9, and ABL1, for example for input into an algorithm or in the derivation of an algorithm, can further comprise IFI27 such that the expression levels of RUNX1, ITGAM, PSTPIP2, LY6E, IRF9 and IFI27, or RUNX1, ITGAM, PSTPIP2, LY6E, IRF9, IFI27 and ABL1 are used for input into an algorithm or in the derivation of an algorithm.

Non-claimed methods can include treating a subject suspected of having a bacterial infection, a viral infection, or a non-infectious cause of fever. The methods of treating a subject can include a) identifying the subject as having a bacterial infection, a viral infection, or a non-infectious cause of fever, based on the expression levels of biomarkers RUNX1, ITGAM, PSTPIP2, LY6E and IRF9 in a biological sample from the subject; b) administering to the subject: i) at least one antiviral therapy when the subject is identified to have a viral infection, ii) at least one antibiotic therapy when the subject is identified to have a bacterial infection, or iii) at least one antipyretic therapy when the subject is identified to have a non-infectious cause of fever. As described herein, the expression level of each biomarker can be measured by performing microarray analysis, polymerase chain reaction (PCR), reverse transcriptase polymerase chain reaction (RT-PCR), or RNA sequencing analysis. In some examples, the biomarkers are RNA biomarkers quantified by PCR.

In some aspects of the preceding methods disclosed herein, the methods can further include identifying the subject as having a bacterial infection, a viral infection, or a non-infectious cause of fever, based on the expression levels of one or more biomarkers in addition to RUNX1, ITGAM, PSTPIP2, LY6E and IRF9 in a biological sample from the subject.

For example, the viral infection score, the bacterial infection score, and/or the non-infectious cause of fever score can be determined based on the expression levels of RUNX1, ITGAM, PSTPIP2, LY6E, IRF9, and IF127. Specifically, the methods of treating or determining if a subject has a bacterial infection, a viral infection, or a non-infectious cause of fever can comprise determining the expression levels of RUNX1, ITGAM, PSTPIP2, LY6E, IRF9 and IFI27 in a biological sample from the subject and determining a viral infection score and a bacterial infection score based on the expression levels of RUNX1, ITGAM, PSTPIP2, LY6E, IRF9, and IFIF27. The methods of triaging or treating a patient suspected of having a bacterial infection, a viral infection, or a non-infectious cause of fever in a subject in need thereof can comprise identifying the subject as having a bacterial infection, a viral infection, or a non-infectious cause of fever, based on the expression levels of biomarkers RUNX1, ITGAM, PSTPIP2, LY6E, IRF9 and IF127 in a biological sample from the subject. The methods for identifying a specific pathogenic viral infection or specific pathogenic bacterial infection can comprise determining a specific pathogenic viral infection score or a specific pathogenic bacterial infection score based on the expression levels of at least two biomarkers selected from RUNX1, ITGAM, PSTPIP2, LY6E, IRF9 and IF127.

Any biomarker measured as part of the methods of the present disclosure (e.g. RUNX1, ITGAM, PSTPIP2, LY6E, IRF9 and ABL1) can be mRNA. Thus, in a non-limiting example, determining the expression level of RUNX1 (or any other biomarker recited herein) in a biological sample from the subject can comprise determining the amount of RUNX1 mRNA in the biological sample from the subject.

In some aspects of the methods of the present disclosure, wherein the biomarker is mRNA, the RNA can be reverse transcribed to produce cDNA, and the produced cDNA expression level can be detected. RNA may be reverse transcribed using MML V reverse transcriptase or modified variants of MMLV that have been mutated to be to increase thermostability, decrease terminal deoxynucleotidyl transferase activity, and/or increase fidelity. In some aspects, the expression level of a biomarker can be detected by forming a complex between the biomarker and a labeled probe or primer. In some aspects of the present disclosure, wherein a biomarker is RNA and/or cDNA, the RNA and/or cDNA can be detected by forming a complex between the RNA and/or cDNA and a labeled nucleic acid probe or primer. A complex between the RNA or cDNA and the labeled nucleic acid probe or primer can be a hybridization complex. In some aspects, a label can be a fluorescent label.

Non-limiting examples of fluorescent labels include ALEXA FLUOR™ 350, ALEXA FLUOR™ 405, ALEXA FLUOR™ 430, ALEXA FLUOR™ 532, ALEXA FLUOR™ 546, ALEXA FLUOR™ 568, ALEXA FLUOR™ 594, ALEXA FLUOR™ 647, BODIPY 493/503, BODIPY FL, BODIPY R6G, BODIPY 530/550, BODIPY TMR, BODIPY 558/568, BODIPY 558/568, BODIPY 564/570, BODIPY 576/589, BODIPY 581/591, BODIPY TR, BODIPY 630/650, BODIPY 650/665, Cascade Blue, Cascade Yellow, Dansyl, lissamine rhodamine B, Marina Blue, Oregon Green 488, Oregon Green 514, Pacific Blue, Pacific Orange, rhodamine 6G, rhodamine green, rhodamine red, tetramethyl rhodamine, Texas Red, Cy2, Cy3, Cy3.5, Cy5, Cy5.5, Cy7 and the like.

In some aspects of the methods of the present disclosure, determining the expression level of a biomarker (e.g. RUNX1, ITGAM, PSTPEP2, LY6E, IRF9 and ABL1) can comprise performing quantitative PCR on nucleic acids extracted from a biological sample from a subject. As would be appreciated by the skilled artisan, in aspects wherein quantitative PCR is used to quantify the expression level of a biomarker, the expression level of a biomarker can be expressed as a cycle threshold (Ct) value.

As would be appreciated by the skilled artisan, a non-limiting example of a quantitative PCR method includes reverse transcriptase quantitative PCR.

As would be appreciated by the skilled artisan, the methods described herein can be used in combination with the GeneXpert® (Cepheid). The GeneXpert® system can be used to determine the expression levels of the biomarkers recited herein. As would be appreciated by the skilled artisan, the GeneXpert® system utilizes a self-contained, single use cartridge. Sample extraction, amplification, and detection may all carried out within this self-contained "laboratory in a cartridge." (See e.g., US Patents 5,958,349, 6,403,037, 6,440,725, 6,783,736, 6,818,185, 9,873,909, 10,562,030).

Components of the cartridge include, but are not limited to, processing chambers containing reagents, filters, and capture technologies useful to extract, purify, and amplify target nucleic acids. A valve enables fluid transfer from chamber to chamber and contain nucleic acids lysis and filtration components. An optical window enables real-time optical detection. A reaction tube enables very rapid thermal cycling.

In some embodiments, the GeneXpert system includes a plurality of modules for scalability. Each module includes a plurality of cartridges, along with sample handling and analysis components.

In some aspects, after a sample is added to the cartridge, the sample can be optionally contacted with lysis buffer and released nucleic acid, which may be RNA, DNA and/or cDNA can be bound to a nucleic acid-binding substrate such as a silica or glass substrate. The sample supernatant is then removed and the nucleic acid is eluted in an elution buffer such as a Tris/EDTA buffer. The eluate may then be processed in the cartridge to detect target genes as described herein. In some embodiments, the eluate is used to reconstitute at least some of the PCR reagents, which are present in the cartridge as lyophilized particles.

As would be appreciated by the skilled artisan, a Ct value is the number of cycles in a quantitative PCR experiment that are required for the fluorescent signal associated with the amplification of a specific target nucleic acid to exceed a predetermined threshold value. As would be appreciated by the skilled artisan, this threshold value can be the background fluorescence levels measured in the experiment.

The biological sample comprises blood.

In some aspects, a biological sample in a training set can comprise blood.

Biological samples used in the methods of the present disclosure can be collected from a subject using appropriate methods known in the art, as would be appreciated by the skilled artisan.

In aspects wherein a biological sample comprises blood, the blood can be collected from a subject using any blood collection method known in the art, as would be appreciated by the skilled artisan.

In aspects wherein a biological sample comprises blood, the blood can be collected from a subject using the PAXgene® collection method, as would be appreciated by the skilled artisan.

In aspects wherein a biological sample comprises blood, the blood can be collected from **a** subject using EDTA sample collection tube, as would be appreciated by the skilled artisan.

In some aspects wherein a biological sample comprises blood, the blood can be collected from a subject and subsequently mixed with a stabilization solution. As would be appreciated by the skilled artisan, a non-limiting example of a stabilization solution is RNAlater™.

In some aspects, biomarkers, including DNA and RNA, can be extracted from biological samples using any method known in the art, including, but not limited to, methods described in US Patent No 10,465,182.

In aspects wherein the biomarkers to be measured are RNA transcripts, RNA can be extracted from the biological sample using any suitable RNA extraction method known in the art, as would be appreciated by the skilled artisan.

Kits

Disclosed but not claimed are kits for use in performing any of the methods of the present disclosure.

In some aspects, a kit of the present disclosure can comprise instructions for use. The instructions can be written instructions. A kit of the present disclosure can be used for any method described herein.

In some aspects, a kit of the present disclosure can comprise at least about 1 agent, or at least about 2, or at least about 3, or at least about 4, or at least about 5, or at least about 6, or at least about 7, or at least about 8, or at least about 9, or at least about 10 agents specific to detect the expression of at least about one biomarker, or at least about 2, or at least about 3, or at least about 4, or at least about 5, or at least about 6, or at least about 7, or at least about 8, or at least about 9, or at least about 10 biomarkers, including, but not limited to RUNX1, ITGAM,

PSTPIP2, LY6E, IRF9 and ABL1. In some aspects, an agent specific to detect the expression of at least one biomarker can comprise a primer, a pair of primers, a sense and anti-sense primer pair, a polynucleotide that specifically hybridizes to a biomarker or any combination thereof.

The present disclosure provides kits for determining if a subject has a bacterial infection, a viral infection, or a non-infectious cause of fever in a sample from a human subject. Kits of the present disclosure can comprise: (a) a first primer pair for detecting a RUNX1 gene; (b) a second primer pair for detecting an ITGAM gene; (c) a third primer pair for detecting a PSTPIP2 gene; (d) a fourth primer pair for detecting a LY6E gene,; (e) a fifth primer pair for detecting an IRF9 gene; and (f) a sixth primer pair for detecting an ABL1 gene. In some aspects, at least one primer is detectably labeled.

The kits disclosed herein can further (a) a probe for detecting the RUNX1 gene; (b) a probe for detecting the ITGAM gene; (c) a probe for detecting the PSTPIP2 gene; (d) a probe for detecting the LY6E gene; (e) a probe for detecting the IRF9 gene; and (f) a probe for detecting the ABL1 gene.. In some aspects, at least one probe is detectably labeled.

The kits of the present disclosure can further comprise a primer pair for detecting an exogenous control and/or an endogenous control, wherein the exogenous control is a sample processing control, and wherein the endogenous control is a sample adequacy control. The kits can further comprise an exogenous control probe and/or endogenous control probe.

In some aspects, the kits of the present disclosure can comprise the reagents described above provided in one or more GeneXpert® Sample cartridge(s). These cartridges permit extraction, amplification, and detection to be carried out within this self-contained "laboratory in a cartridge." (See e.g., US Patents 5,958,349, 6,403,037, 6,440,725, 6,783,736, 6,818,185, 9,873,909, 10,562,030).

As described in the methods disclosed herein, diagnostic or treatment recommendation can be provided from a clinical decision support (CDS) system. In some aspects, the clinical decision support comprises a storage system that provides clinicians or patients with knowledge, intelligently filtered or presented at appropriate times, to enhance health and health care, and can be effective to improve patient safety, providing, for instance, alerts for error reduction. The CDS systems can make dynamic predictions, allowing interactions with clinicians and taking into consideration the longitudinal nature of health and disease. More specifically, the CDS system can include (i) temporally ordered steps, each leading to new data, which in turn becomes useful for a new decision, (ii) feedback loops where acquisition of new data improves certainty and generates new questions to examine, (iii) combining different kinds of clinical data for decision making, (iv) reusing the same data in two or more different decisions, and (v) clinical decisions requiring human cognitive skills and knowledge, to process the available information.

The CDS system can include an input for providing a subject's viral infection score and/or bacterial infection score based on the expression levels of biomarkers measured and its functional readout, for example the subject has a viral infection, a bacterial infection, or a non-infectious cause of fever. The CDS system may further include an external data module. The external data module may include data gathered from historical data, or data provided by external organizations such as hospitals, universities, public health organizations, or research organizations relating to patient health, disease conditions and states, clinical laboratory data for decision support, and the like Together, data from the external data module and CDS system may provide even richer information and intelligence to the data collected. For example, data from external module may contain patient information such as treatment plans, prescription drugs, implanted medical devices, invasive and non-invasive procedures, diseases and parameters, and the like that are often associated with certain test results obtained from instruments.

In one specific embodiment, the CDS system can further include a decision-making workstation. The decision-making workstation can be used for deciding on the initiation continuation of a drug therapy for a subject based on the infection status of the subject. In some instances, the decision-making workstation can be used for diagnostic test ordering useful for a new decision. For example, if a subject is diagnosed with a bacterial infection, a confirmatory diagnostic test for the specific bacterial infection can be ordered. Similarly, if a subject is diagnosed with a viral infection or non-infectious of cause of fever using the methods described herein, a confirmatory diagnostic test for the specific viral infection or non-infectious disease can be ordered, respectively. Patient history, symptoms, and physical examination contribute to decisions for diagnostic test ordering. Test results form the basis for patient diagnosis and/or recommendation. The diagnosis and/or recommendation, in turn, is decisive for the choice of an optimal treatment. In some instances, the result of a diagnostic test may direct physicians to order additional tests, as a requirement for successful differential diagnosis. In addition, a diagnostic test may be repeated, during a periodic assessment, to confirm or alter the therapeutic scheme in response to the updated diagnostic test results. These feedback loops and repeated measurements are often mandated as requirements of hospital clinical pathways and protocols of care, and can be included in the CDS system.

In some aspects, the clinical decision support system can combine data for decision making. Examples include combining diagnostic test results with patient history, physical examination and symptoms, to form the diagnosis. Or the CDS system may be used for deciding on the probability and likelihood of responsiveness to a therapy. For example, the combination of lab test results, the diagnosis, patient history, physical examination and treatment can predict the patient prognosis.

The CDS systems can also be useful for therapeutic selection, determining response to treatment, adjustment, and dosing of treatment, monitoring ongoing therapeutic efficiency, and indication for change in therapeutic regimen. As described herein, the CDS system can integrate patient history data such as clinical, epigenomic, and genomic data to optimize clinical decision support, for example to select the drug(s) that have the highest probability of a positive therapeutic outcome for a particular patient.

The CDS system can be used to predict patient decompensation, to identify patients at risk of readmission, or for remote patient management in post-acute care.

As used herein, the terms "expression level" and "amount" are used interchangeably to refer to the amount of a specific molecule (*e.g.* a specific RNA transcript) present in a given biological sample or from biological material extracted form a biological sample.

The terms "diagnosis" and "diagnostics" also encompass the terms "prognosis" and "prognostics", respectively, as well as the applications of such procedures over two or more time points to monitor the diagnosis and/or prognosis over time, and statistical modeling based thereupon. Furthermore the term diagnosis includes: a. prediction (determining if a patient will likely develop aggressive disease (hyperproliferative/invasive)), b. prognosis (predicting whether a patient will likely have a better or worse outcome at a pre-selected time in the future), c. therapy selection, d. therapeutic drug monitoring, and e. relapse monitoring.

The term "subject" as used herein refers to a mammal, preferably a human. In some aspects, a subject can have a fever. In some aspects, a subject can be a subject who has previously undergone a surgery, herein referred to as a "postoperative subject".

The term "antiviral therapy" as used herein refers to any therapy known in the art administered to a subject to treat a viral infection. Non-limiting examples of antiviral therapies include, but are not limited to, rapivab, relenza, Tamiflu, xofluza, remdesivir or any combination thereof.

The term "antibiotic therapy" as used herein refers to any therapy known in the art administered to a subject to treat a bacterial infection. Non-limiting examples of antibiotic therapies include, but are not limited to, amoxicillin, doxycycline, cephalexin, ciprofloxacin, clindamycin, metronidazole, azithromycin, sulfamethoxazole, trimethroprim, clavulanate, levofloxacin or any combination thereof.

The term "antipyretic therapy" as used herein refers to any therapy known in the art administered to a subject to treat, prevent and/or reduce a fever. Non-limiting examples of antipyretic therapies include, but are not limited to, acetaminophen, a nonsteroidal antiinflammatory drug (NSAID) or any combination thereof. Non-limiting examples of NSAIDs include, but are not limited to, ibuprofen, aspirin, or naproxen.

The term "oligonucleotide" is used to refer to a nucleic acid that is relatively short, generally shorter than 200 nucleotides, more particularly, shorter than 100 nucleotides, most particularly, shorter than 50 nucleotides. Typically, oligonucleotides are single-stranded DNA molecules.

The term "primer" refers to an oligonucleotide that is capable of hybridizing (also termed "annealing") with a nucleic acid and serving as an initiation site for nucleotide (RNA or DNA) polymerization under appropriate conditions (i.e., in the presence of four different nucleoside triphosphates and an agent for polymerization, such as DNA or RNA polymerase or reverse transcriptase) in an appropriate buffer and at a suitable temperature. The appropriate length of a primer depends on the intended use of the primer, but primers are typically at least 7 nucleotides long and, in some aspects, range from 10 to 30 nucleotides, or, in some aspects, from 10 to 60 nucleotides, in length. In some aspects, primers can be, e.g., 15 to 50 nucleotides long. A primer need not reflect the exact sequence of the template but must be sufficiently complementary to hybridize with a template.

The term "primer pair" refers to a set of primers including a 5' "upstream primer" or "forward primer" that hybridizes with the complement of the 5' end of the DNA sequence to be amplified and a 3' "downstream primer" or "reverse primer" that hybridizes with the 3' end of the sequence to be amplified. As will be recognized by those of skill in the art, the terms "upstream" and "downstream" or "forward" and "reverse" are not intended to be limiting, but rather provide illustrative orientations in some aspects.

A "probe" is a nucleic acid capable of binding to a target nucleic acid of complementary sequence through one or more types of chemical bonds, generally through complementary base pairing, usually through hydrogen bond formation, thus forming a duplex structure. The probe can be labeled with a detectable label to permit facile detection of the probe, particularly once the probe has hybridized to its complementary target. Alternatively, however, the probe may be unlabeled, but may be detectable by specific binding with a ligand that is labeled, either directly or indirectly. Probes can vary significantly in size. Generally, probes are at least 7 to 15 nucleotides in length. Other probes are at least 20, 30, or 40 nucleotides long. Still other probes are somewhat longer, being at least 50, 60, 70, 80, or 90 nucleotides long. Yet other probes are longer still, and are at least 100, 150, 200 or more nucleotides long. Probes can also be of any length that is within any range bounded by any of the above values (e.g., 15-20 nucleotides in length).

The primer or probe can be perfectly complementary to the target nucleotide sequence or can be less than perfectly complementary. In some aspects, the primer has at least 65% identity to the complement of the target nucleotide sequence over a sequence of at least 7 nucleotides, more typically over a sequence in the range of 10-30 nucleotides, and, in some aspects, over a sequence of at least 14-25 nucleotides, and, in some aspects, has at least 75% identity, at least 85% identity, at least 90% identity, or at least 95%, 96%, 97%, 98%, or 99% identity. It will be understood that certain bases (e.g., the 3' base of a primer) are generally desirably perfectly complementary to corresponding bases of the target nucleotide sequence. Primer and probes typically anneal to the target sequence under stringent hybridization conditions.

As used herein with reference to a portion of a primer or a nucleotide sequence within the primer, the term "specific for" a nucleic acid, refers to a primer or nucleotide sequence that can specifically anneal to the target nucleic acid under suitable annealing conditions.

"Treating" or "treatment" as used herein with regard to a condition may refer to preventing the condition, slowing the onset or rate of development of the condition, reducing the risk of developing the condition, preventing or delaying the development of symptoms associated with the condition, reducing or ending symptoms associated with the condition, generating a complete or partial regression of the condition, or any combination thereof.

The terms "effective amount" and "therapeutically effective amount" of an agent or compound are used in the broadest sense to refer to a nontoxic but sufficient amount of an active agent or compound to provide the desired effect or benefit.

The term "benefit" is used in the broadest sense and refers to any desirable effect and specifically includes clinical benefit as defined herein. Clinical benefit can be measured by assessing various endpoints, e.g., inhibition, to some extent, of disease progression, including slowing down and complete arrest; reduction in the number of disease episodes and/or symptoms; reduction in lesion size; inhibition (i.e., reduction, slowing down or complete stopping) of disease cell infiltration into adjacent peripheral organs and/or tissues; inhibition (i.e. reduction, slowing down or complete stopping) of disease spread; inhibition (i.e. reduction, slowing down or complete stopping) of the spread of an infection in a subject; decrease of autoimmune response, which may, but does not have to, result in the regression or ablation of the disease lesion; relief, to some extent, of one or more symptoms associated with the disorder; increase in the length of disease-free presentation following treatment, e.g., progression-free survival; increased overall survival; higher response rate; and/or decreased mortality at a given point of time following treatment.

A "biomarker" in the context of the present disclosure refers to a biological compound, such as a polynucleotide or polypeptide which is differentially expressed in a sample taken from patients having a bacterial infection, a viral infection, or a non-infectious cause of fever as compared to a comparable sample taken from control subjects (e.g., a person with a negative diagnosis, normal or healthy subject, or non-infected subject) or differentially expressed in a sample from a patient having a bacterial or viral infection as compared to a sample from a patient having for example, a viral or bacterial infection, respectively. The biomarker can be a nucleic acid, a fragment of a nucleic acid, a polynucleotide, or an oligonucleotide that can be detected and/or quantified. Bacterial infection, viral infection, and non-infectious causes of fever biomarkers include polynucleotides comprising nucleotide sequences from genes or RNA transcripts of genes, including but not limited to, RUNX1, ITGAM, PSTPIP2, LY6E, IRF9, and ABL1, and their expression products. T

As would be appreciated by the skilled artisan, the term "RUNX1" refers to any isoform of runt-related transcription factor 1 (RUNX1). An example of the nucleotide sequence of RUNX1 is published in the NCBI database under the accession number KF305770.1 (SEQ ID NO: 1).

As would be appreciated by the skilled artisan, the term "ITGAM" refers to any isoform of integrin subunit alpha M (ITGAM). An example of the nucleotide sequence of ITGAM is published in the NCBI database under the accession number JA738583.1 (SEQ ID NO: 2).

As would be appreciated by the skilled artisan, the term "PSTPIP2" refers to any isoform of proline-serine-threonine phosphatase interacting protein 2 (PSTPIP2). An example of the nucleotide sequence of PSTPIP2 is published in the NCBI database under the accession number AK023100.1 (SEQ ID NO: 3).

As would be appreciated by the skilled artisan, the term "LY6E" refers to any isoform of lymphocyte antigen 6E (LY6E). An example of the nucleotide sequence of LY6E is published in the NCBI database under the accession number U66711.1 (SEQ ID NO: 4).

As would be appreciated by the skilled artisan, the term "IRF9" refers to any isoform of interferon regulatory factor 9 (IRF9). An example of the nucleotide sequence of IRF9 is published in the NCBI database under the accession number BC035716.2 (SEQ ID NO: 5).

As would be appreciated by the skilled artisan, the term "ABL1" refers to any isoform of tyrosine-protein kinase ABL1 (ABL1). An example of the nucleotide sequence of ABL1 is published in the NCBI database under the accession number AH005332 (SEQ ID NO: 6).

## **Examples**

### Example 1

The following is a non-limiting example describing the development of the methods described herein. In brief, 21 mRNA targets were identified and evaluated for their ability to distinguish subjects with bacterial infections, viral infections, and subjects with non-infectious causes of fever. The 21 mRNA targets included human host response target genes rather than bacterial or viral genes.

### *Training Cohort*

The mRNA targets were measured by RT-PCR in a training cohort of 176 samples collected from subjects with fevers, or otherwise suspected of having infections, and were from diverse sites and settings. A broad range of infectious and non-infectious causes of fever were represented. The training set also included 54 samples from healthy control subjects for a total of 230 samples (176+54) in the training set. The infection statuses of the subjects were determined according to the standard of care (SOC) testing methods used at the sites where the subjects were located. One sample was included per subject with the exceptions of 17 of the healthy subjects where both a pre-operative and post-operative sample was included, and for 19 of the subjects that developed post-operative bacterial sepsis, where a sample collected pre-operative and a sample collected on the first or second day of symptom onset was included. Except for the healthy control samples, all samples were collected from subjects with fever or otherwise suspected of having infections, and of these, only samples from patients with confirmed infections or confirmed non-infectious causes of fever were included in the cohort. Table 1 summarizes the training samples by infection status.

**Table 1.**

| Infection Type: | Bacterial | Coinfection | Healthy | Malarial | Symptomatic non-infectious (SNI) | Viral |
|---|---|---|---|---|---|---|
| # of Samples in Training Set: | 79 | 6 | 54 | 10 | 29 | 52 |

The 79 subjects classified as having bacterial infections had positive gram stains, culture or molecular tests performed, on blood or other specimen types, that indicated bacterial infection. Table 2 summarizes the organisms identified (generally by genus name) for the samples from patients with bacterial infections.

**Table 2.**

| Organism Identified | Number of samples |
|---|---|
| >1 organism | 13 |
| *Acinetobacter* | 1 |
| *Aerococcus* | 1 |
| *Bacillus* | 1 |
| *Bacteriodes* | 1 |
| *Borrelia* | 2 |
| *Clostridium* | 3 |
| *Enterobacter* | 1 |
| *Enterococcus* | 1 |
| *Escherichia* | 15 |
| *Klebsiella* | 5 |
| *Neisseria* | 1 |
| *Pseudomonas* | 1 |
| *Serratia* | 1 |
| *Staphylococcus* | 14 |
| *Streptococcus* | 6 |
| Unspecified bacteria | 11 |
| *Ehrlichia* | 1 |

The six subjects classified as having a coinfection had both bacterial and viral infections identified are summarized in Table 3.

**Table 3.**

| Subject # | Bacterial and Viral Infections Identified |
|---|---|
| 1 | Flu A, Adenovirus, H. influenza |
| 2 | Strep A and Flu A |
| 3 | Strep A and Flu A |
| 4 | Bacterial Multiple; Flu B |
| 5 | Rhinovirus/Enterovirus, MSSA |
| 6 | E. coli & Rhinovirus/Enterovirus |

Of the 54 healthy samples, 17 were pre-operative samples from subjects that did not develop sepsis, 20 were pre-operative samples from subjects that later developed sepsis, and 17 were post-operative samples from subjects that did not develop sepsis.

10 subjects were classified as having malarial infections.

29 subjects were classified as symptomatic non-infected (SNI) and were determined to have non-infectious causes of fever as summarized in Table 4.

**Table 4.**

| Non-infectious cause of fever | Number of samples |
|---|---|
| Hypersensitivity Pneuomonitis | 1 |
| immune response to IgG treatment | 1 |

(continued)

| Non-infectious cause of fever | Number of samples |
|---|---|
| lupus | 1 |
| Metastatic Pancreatic Cancer | 1 |
| Motor Vehicle Collision | 1 |
| NSTEMI (Non-ST-elevation myocardial infarction) | 1 |
| polytrauma | 1 |
| poor GI motility | 1 |
| Post-op fever | 2 |
| Pulmonary Embolism | 1 |
| rheumatic fever | 1 |
| Sarcoidosis | 1 |
| Sickle Cell Pain Crisis | 1 |
| SIRS | 13 |
| Transaminitis | 1 |
| ureterovesical junction stone | 1 |

52 subjects were classified as having viral infections that were confirmed by molecular or serological tests. Table 5 summarizes the organisms identified with viral infections.

**Table 5.**

| Organism Identified | Number of samples |
|---|---|
| >1 organism | 4 |
| Astrovirus | 1 |
| coronavirus | 2 |
| Dengue | 14 |
| Flu | 2 |
| Flu A | 13 |
| Flu B | 11 |
| Metapneumovirus | 1 |
| Rhinovirus | 1 |
| RSV | 2 |
| Zika | 1 |

*Sample collection and mRNA quantification*

All samples were venous blood collected into PAXgene® Blood RNA tubes, or into EDTA tubes. The EDTA blood was either combined with the preservative RNAlater™ or aliquoted and stored frozen to preserve the RNA. The RNA from the blood samples was purified using commercially available RNA extraction kits. The purified RNA samples were stored at - 80°C prior to testing.

The levels of the 21 mRNA targets were measured in the RNA purified from 5 µL of blood using 4-plex RT-PCR assays on the QuantStudio 5 instrument. ABL1 mRNA was measured in each of the 4-plex assays as a reference.

*Statistical Analysis*

Initial multivariate exploratory analyses were conducted amongst the 22 mRNA target data. Principal component analysis was conducted to understand the dimensionality of the data. It was observed that five to six dimensions explained most of the variance in the mRNA target variables. The cluster analysis showed that there were highly correlated groupings of different mRNA targets.

Further in the exploratory data analysis stage, univariate analysis including box plots and correlation analyses were performed with the 22 mRNA target (including ABL1) measurements and clinical variables to identify relationships amongst the mRNA targets, and between mRNA targets and demographic/clinical variables for all 230 samples. Without wishing to be bound by theory, the correlation analyses work as a further refinement of the cluster analysis to identifying the strength of relationships between variable pairs including between mRNA targets. The correlation analysis aided in the selection of the subset of mRNAs for the final model because the correlation analysis helps to avoid any multicollinearity caused by the inclusion of two or more highly correlated covariates in the final model. As would be appreciated by the skilled artisan, multicollinearity makes models inefficient because two or more of the covariates are explaining the same portion of the variance and increase error.

The analyses revealed that the mRNA targets TSPO, CTSB, ITGAM, PECR, S100A12, and HK3 are highly correlated, with correlations greater than 0.7.

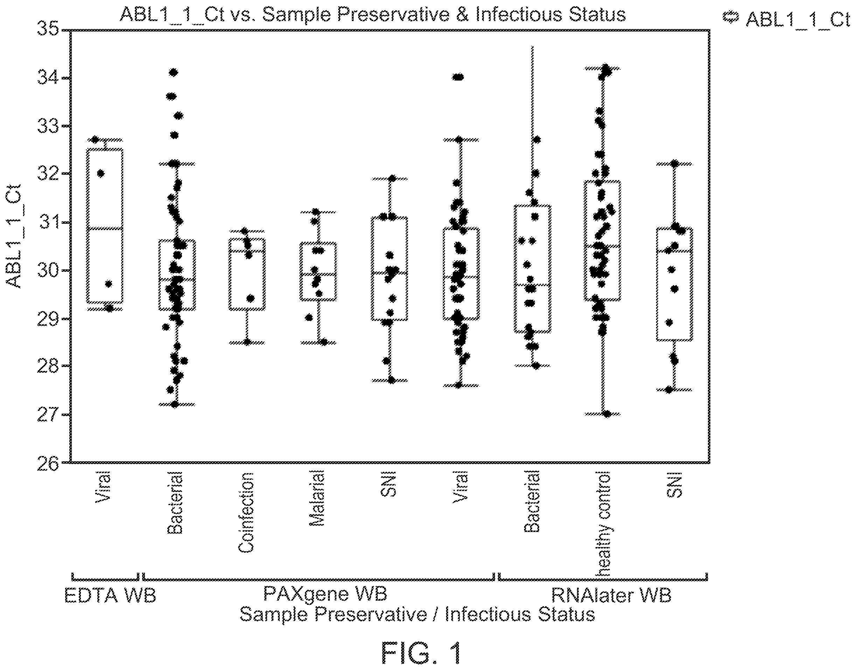

FIG. 1 shows a boxplot of the measured expression levels, expressed as a cycle threshold (Ct) value, of ABL1 (a reference gene) vs. sample preservative type (EDTA, PAXgene and RNAlater) and infection status.

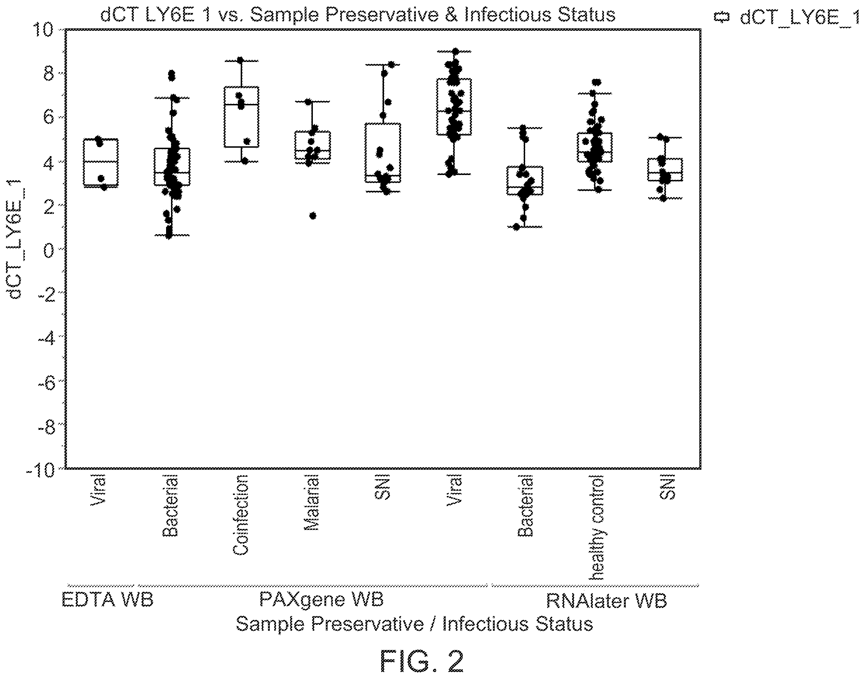

FIG. 2 shows a boxplot of the measured expression levels, expressed as a delta cycle threshold (dCt) value, of LY6E vs. sample preservative type (EDTA, PAXgene and RNAlater) and infection status. The dCt value was calculated by subtracting the expression Ct value of LY6E from the Ct value of ABL1 in each sample.

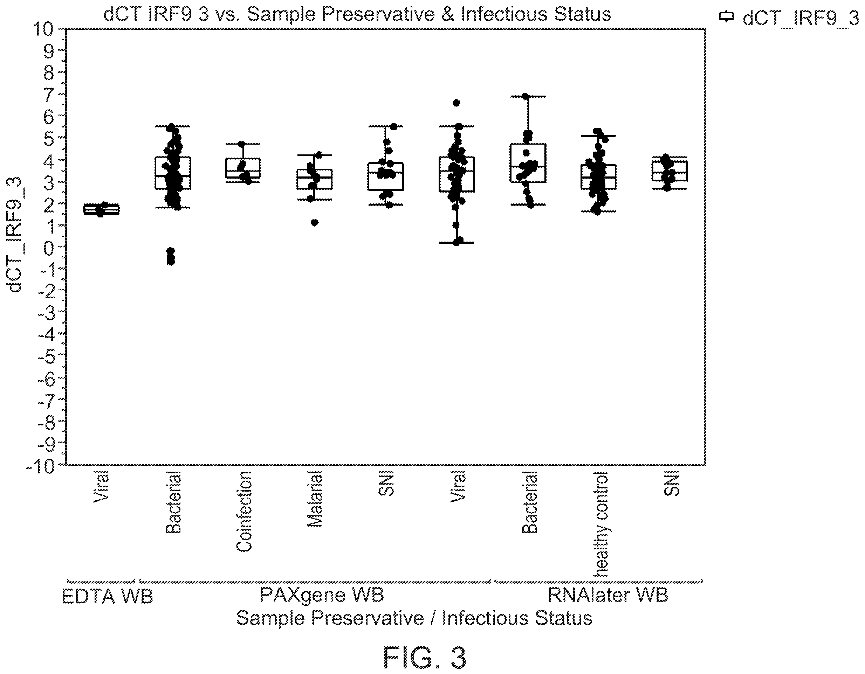

FIG. 3 shows a boxplot of the measured expression levels, expressed as a dCt value, of IRF9 vs. sample preservative type (EDTA, PAXgene and RNAlater) and infection status. The dCt value was calculated by subtracting the expression Ct value of IRF9 from the Ct value of ABL1 in each sample.

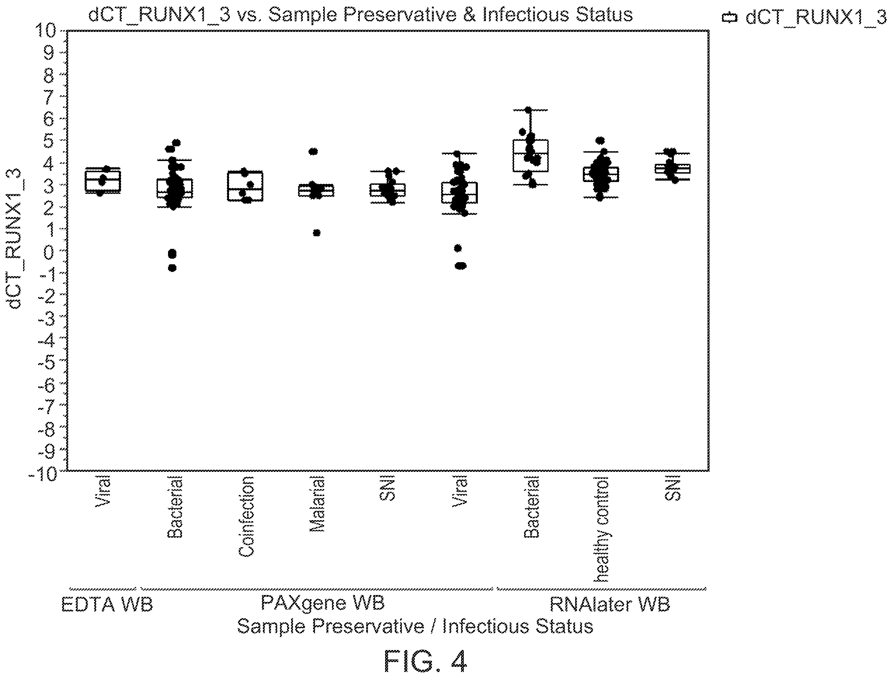

FIG. 4 shows a boxplot of the measured expression levels, expressed as a dCt value, of RUNX1 vs. sample preservative type (EDTA, PAXgene and RNAlater) and infection status. The dCt value was calculated by subtracting the expression Ct value of RUNX1 from the Ct value of ABL1 in each sample.

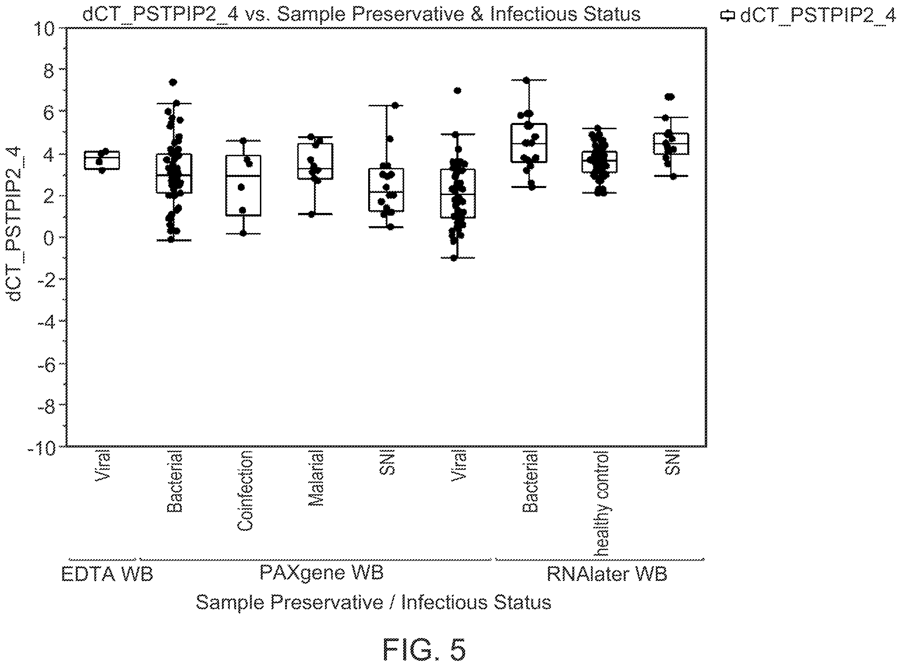

FIG. 5 shows a boxplot of the measured expression levels expressed as a dCt value, of PSTPIP2 vs. sample preservative type (EDTA, PAXgene and RNAlater) and infection status. The dCt value was calculated by subtracting the expression Ct value of PSTPIP2 from the Ct value of ABL1 in each sample.

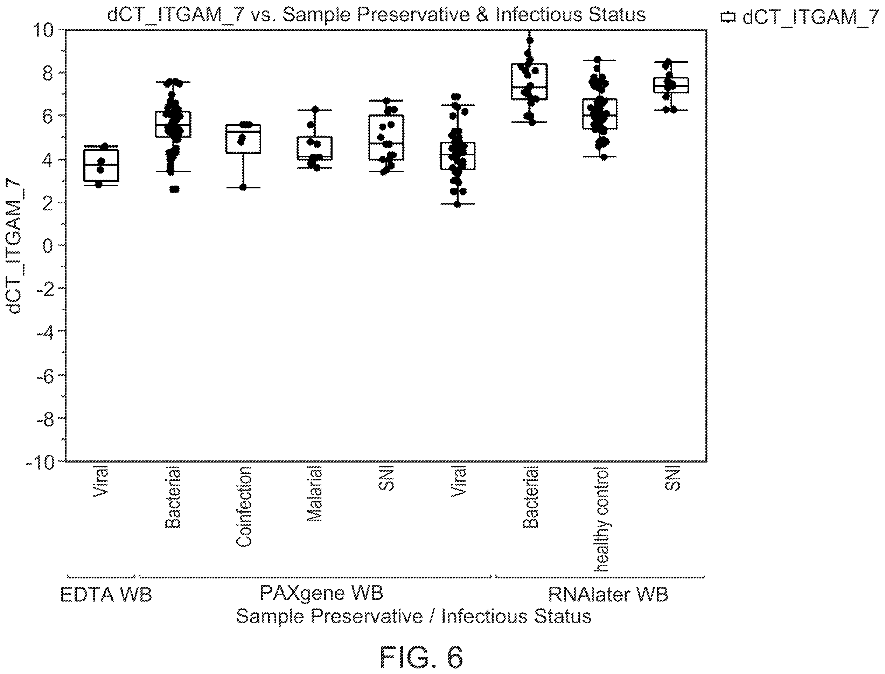

FIG. 6 shows a boxplot of the measured expression levels, expressed as a dCt value, of ITGAM vs. sample preservative type (EDTA, PAXgene and RNAlater) and infection status. The dCt value was calculated by subtracting the expression Ct value of ITGAM from the Ct value of ABL1 in each sample.

A set of univariate analyses was then conducted for each mRNA target to evaluate any relationship between the target and infection status. Without wishing to be bound by theory, the univariate analysis represented a preliminary step prior to performing multivariate analysis, as the univariate analyses identified mRNA targets that are statistically significant in classifying between infection statuses. Accordingly, an mRNA target that is not significant at all in the univariate analysis is unlikely to be useful in any final model. As would be appreciated by the skilled artisan, the performance characteristics in the univariate analyses can also be used to rank the mRNA targets in the order of their classification performance which aids in the selection of a smaller subset of the 21 mRNA targets and other clinical variables to include in the final models for best subset selection.

Three different pairwise comparisons in infection status were made in the univariate analysis using all 230 samples; bacterial versus non-infected (includes healthy SNI, and malaria), bacterial versus viral, and viral versus non-infectious illness. The objective of this analysis was to identify targets that were significantly different between the paired infection statuses. These three comparative infection statuses in the univariate analysis were selected in order to identify whether the significant mRNA targets or other significant clinical variables were indeed markers of a bacterial host response or a viral host response or both host responses. Without wishing to be bound by theory, this identification is important for best subset selection in the final models as it helps ensure that the potential subset pools will have at least one bacterial host response marker, at least one viral host response marker, and at least one marker for both responses.

Results of the univariate analysis conducted using logistic regression are shown in Table 6. The intercept implies intercept (or shift adjustment) of the univariate model, Beta is the slope, while the Log Likelihood (expressed as a negative Chi-square value) is a measure the ability of the target to classify between the infection statuses. The Log Likelihood is interpreted based on the true value but not the absolute values.

**Table 6.**

| mRNA | Bacterial vs. non-infectious illness (BvH) | | | Bacterial vs. Viral (BvV) | | | Viral vs. non-infectious illness (BvH) | | |
|---|---|---|---|---|---|---|---|---|---|
| | BvH Intercept | BrH Beta | BvH Log likelihood | BvV Intercept | BvV Beta | BvH Log likelihood | VvH Intercept | VvH Beta | VvH Log likelihood |
| IFI27 | 0.14 | -0.24 | -111.6 | 1.70 | -0.47 | -69.44 | -0.04 | -0.28 | -137.64 |
| LAX1 | 0.40 | -0.73 | -112.03 | 0.97 | -0.65 | -86.59 | -0.01 | -0.70 | -143.36 |
| LY6E | 1.59 | -0.44 | -111.53 | 4.48 | -0.81 | -64.82 | 1.64 | -0.50 | -136.37 |
| LCN2 | -0.10 | -0.03 | -118.59 | 0.09 | 0.28 | -84.27 | -0.70 | 0.08 | -150.60 |
| PECR | -0.13 | -0.03 | -118.61 | 0.15 | 0.92 | -74.49 | -0.71 | 0.19 | -149.57 |
| TSPO | 0.34 | -0.14 | -117.69 | -2.27 | 1.23 | -59.68 | -1.03 | 0.16 | -149.50 |
| CETP | -0.17 | -0.01 | -118.64 | 1.47 | 0.48 | -75.19 | -0.37 | 0.13 | -148.67 |
| IRF9 | -0.54 | 0.11 | -118.36 | 0.13 | 0.11 | -90.65 | -0.96 | 0.13 | -151.00 |
| RUNX1 | 0.42 | -0.18 | -118.08 | -0.96 | 0.51 | -86.22 | -0.89 | 0.11 | -151.17 |
| CAMK1 | 1.03 | -1.08 | -96.46 | 0.44 | 0.10 | -90.79 | -0.08 | -0.52 | -142.70 |
| PSTPIP2 | 0.06 | -0.06 | -118.49 | -0.78 | 0.46 | -82.93 | -0.95 | 0.13 | -150.47 |
| S100A12 | -0.65 | 0.08 | -118.02 | -2.12 | 0.55 | -68.00 | -1.75 | 0.21 | -144.52 |
| CTSB | 3.14 | -0.60 | -108.23 | -1.83 | 1.24 | -69.60 | -0.28 | -0.05 | -151.38 |
| GPAA1 | 0.41 | -0.42 | -115.21 | 0.11 | 0.52 | -85.42 | -0.50 | -0.03 | -151.48 |
| TNIP1 | 1.82 | -0.37 | -116.20 | -3.94 | 0.94 | -78.78 | -0.85 | 0.06 | -151.39 |
| HK3 | -0.64 | 0.21 | -116.62 | -0.93 | 0.91 | -71.29 | -1.30 | 0.38 | -143.21 |
| JUP | -0.38 | -0.22 | -113.97 | 0.35 | -0.50 | -74.89 | -0.76 | -0.32 | -140.12 |
| RTCB | 0.86 | -0.69 | -113.47 | 0.95 | -0.32 | -89.86 | 0.21 | -0.50 | -147.70 |
| ITGAM | -0.57 | 0.07 | -118.48 | -5.32 | 1.15 | -65.16 | -2.28 | 0.31 | -146.22 |
| MX1 | -0.20 | -0.04 | -118.55 | 0.57 | -0.61 | -69.49 | -0.65 | -0.19 | -146.98 |
| STAT1 | 0.59 | -0.20 | -117.37 | 1.31 | -0.21 | -89.69 | 0.24 | -0.20 | -149.87 |

Next, multivariate analysis was conducted using logistic regression with a backward selection approach to identify combination of mRNAs that could achieve the desired infection status classifications. To this end, two separate types of model classification were used: bacterial infection versus rest, (where rest included viral infections, healthy controls coinfections, SNI, and malaria) and viral infection versus rest (where rest included bacterial infections, healthy controls, coinfections, SNI, and malaria). The model selection had the benchmark of the prediction performance in cross-validation. Both of the models used the full dataset (N=230) and sample preservative was not entered as a variable. Eight mRNA targets (CAMK1, IRF9, LY6E, RUNX1, JUP, HK3, LAX1, RTCB, were selected in the bacterial model; the AUC of it was 0.8592. Six mRNA targets (CETP, LY6E, PSTPIP2, GPAA1, CTSB, STAT1) were selected in the viral model; the AUC of it was 0.9712. A total of 13 mRNA targets were used in the two models and LY6E was the common marker shared by both models. Some of these eight targets are markers of bacterial infection, others are markers of viral infection, and a few others are differentially expressed in both types of infections. Summaries of the backward selection (elimination) steps for the two models are shown in Tables 7 and 8.

**Table 7.** Backward selection Bacterial vs. Rest Modeling elimination

| Summary of Backward Elimination | | | | |
|---|---|---|---|---|
| Step | Effect Removed | Number In | Wald Chi-Square | Pr > ChiSq |
| 1 | dCf_CTSB_5 | 20 | 0.02 | 0.8921 |
| 2 | dCT_ITGAM_7 | 19 | 0.02 | 0.8958 |
| 3 | dCT_GPAA1_5 | 18 | 0.04 | 0.8389 |

(continued)

| Step | Effect Removed | Number In | Wald Chi-Square | Pr > ChiSq |
|------|----------------|-----------|-----------------|------------|
| | **Summary of Backward Elimination** | | | |
| 4 | dCT_LCN2_2 | 17 | 0.12 | 0.7264 |
| 5 | dCT_STAT1_7 | 16 | 0.10 | 0.7573 |
| 6 | dCT_S100A12_4 | 15 | 0.08 | 0.7721 |
| 7 | dCT_TSPO_2 | 14 | 0.22 | 0.641 |
| 8 | dCT_PECR_2 | 13 | 0.23 | 0.6286 |
| 9 | dCT_PSTPIP2_4 | 12 | 1.03 | 0.3096 |
| 10 | dCT_IFI27_1 | 11 | 1.04 | 0.3086 |
| 11 | dCT_CETP_3 | 10 | 1.22 | 0.2685 |
| 12 | dCT_MX1_7 | 9 | 2.20 | 0.138 |
| 13 | dCT_TNIP1_5 | 8 | 2.15 | 0.1428 |

**Table 8. Backward selection Viral vs. Rest Modeling elimination**

| Step | Effect Removed | Number In | Wald Chi-Square | Pr > ChiSq |
|------|----------------|-----------|-----------------|------------|
| | **Summary of Backward Elimination** | | | |
| 1 | dCT_LCN2_2 | 20 | 0.00 | 0.9912 |
| 2 | dCT_HK3_6 | 19 | 0.00 | 0.9483 |
| 3 | dCT_TSPO_2 | 18 | 0.01 | 0.9083 |
| 4 | dCf_JUP_6 | 17 | 0.02 | 0.8911 |
| 5 | dCT_MX1_7 | 16 | 0.11 | 0.7425 |
| 6 | dCT_LAX1_1 | 15 | 0.08 | 0.7761 |
| 7 | dCT_TNIP1_5 | 14 | 0.39 | 0.5307 |
| 8 | dCT_S100A12_4 | 13 | 0.37 | 0.5436 |
| 9 | DCT-PECR 2 | 12 | 0.40 | 0.5248 |
| 10 | dCT_RUNX1_3 | 11 | 1.48 | 0.223 |
| 11 | dCT_CAMK1_4 | 10 | 2.46 | 0.1171 |
| 12 | dCf_IRF9_3 | 9 | 3.05 | 0.0807 |
| 13 | dCT_RTCB_6 | 8 | 2.65 | 0.1035 |
| 14 | dCT_ITGAM_7 | 7 | 2.44 | 0.1179 |
| 15 | dCT_IFI27_1 | 6 | 3.71 | 0.0541 |

The backward selection approach identified a set of eight significant mRNA targets from the initial 21 targets that could provide the maximum classification between bacterial and rest. Similarly, it identified a set of 6 markers that could provide the maximum classification between viral and rest Further analysis with these multivariate models using a full model versus reduced model approach showed that after inclusion of the first five covariates in either model, inclusion of additional covariates only added trivial classification ability to the model. Accordingly, the final models did not include more than five covariates. This is consistent with the results of the principal component analysis that demonstrated earlier on that there were five-six dimensions to the data.

An additional round of analyses were performed with the aim of determining the best subset of five mRNA targets that could provide the best possible classification in both bacterial versus rest and viral versus rest models. The reason for considering five covariates is explained above. Multiple combination sets of five targets each were created based on the different findings from the univariate analysis, correlation analysis, and multivariate analysis. Based on target identification observed from the univariate analysis, logical rules were applied such as any combination of five targets should have a mix of bacterial infection markers, viral infection markers, and targets that are differentially expressed in both infections. Also based on findings in the correlation analysis, a combination of five targets should not have two or more highly correlated targets. If one target had to be chosen from two highly correlated targets, then the choice would consider their relative ranks in the univariate analysis plus their relative ranking in the multivariate analysis backward elimination. The LY6E target which was common in both multivariate models was included in most combinations of five targets.

Using each combination of five targets as independent variables, two logistic regression model, bacterial versus rest and viral versus rest were fit. These models were fit in a sub-dataset (N = 195) in which symptomatic-non-infected (SNI) and coinfected samples were eliminated to reduce the total noise of the model. As would be appreciated by the skilled artisan, when fitting classification models for comparison, well-defined and mutually exclusive categories are used. The SNI were excluded because they are not well-defined and the coinfections were excluded because of ambiguity in infection status. Sample preservative type was included in all models to adjust for its significant impact on the targets. For each fitted model, the AUC score was used as the fit criteria to compare fitted models.

A total of 25 models were tested, and the range of AUC scores was from 0.73 to 0.95 for the bacterial models and from 0.84 to 0.97 for the viral models. The five targets. RUNX1, ITGAM, PSTPIP2, LY6E, and IRF9, were identified as the most robust combination for both bacterial and viral detection models and are referred to as the V1 targets. As summarized in Table 9, the bacterial with sample preservative model and V1 targets had an AUC of 0.92 (%95 CI: 0.88-0.96) and the log likelihood was -68.89; The viral with sample preservative model and V1 targets had an AUC of 0.97 (%95 CI: 0.94- 0.99) and the log likelihood was -39.96. The bacterial model performed better than the benchmark (benchmark targets and sample with sample preservative) model (AUC 0.90) and the viral model performed equally as the benchmark model (AUC 0.97).

Taken together, the results described in this example demonstrate that the specific combination of the biomarkers RUNX1, ITGAM, PSTPIP2, LY6E and IRF9 can be used in the methods described herein to distinguish between viral infections, bacterial infections, and non-infectious causes of fevers in subjects.

**Table 9.**

| Model | Bacterial | | | Viral | | |
|---|---|---|---|---|---|---|
| | Targets | Training Cohort 2 (N-230) | Excluding SNI & Coinfection (N = 195) | Targets | Training Cohort 2 (N=230) | Excluding SNI & Coinfection (N = 195) |
| | | AUC (95%CI) | AUC (95% CI) | | AUC (95% CI) | AUC (95% CI) |
| V1 | RUNX1 ITGAM PSTPIP2 LY6E IRF9 | 0.77 (0.71-0.84) | 0.86 (0.81-0.92) | RUNX1 ITGAM PSTPIP2 LY6E IRF9 | 0.93 (0.89-0.97) | 0.94 (0.90-0.98) |
| V1 with Sample Preservative | Sample_Preservative RUNX1 ITGAM PSTPIP2 LY6E IRF9 | 0.85 (0.80-0.90) | 0.92 (0.88-0.96) | Sample_Preservative RUNX1 ITGAM PSTPIP2 LY6E IRF9 | 0.96 (0.93-0.98) | 0.97 (0.94-0.99) |
| Benchmark | CTSB GPAA1 HK3 IFI27 JUP LAX1 TNIP1 | 0.82 (0.76-0.88) | 0.87 (0.82-0.92) | CTSB GPAA1 HK3 IF127 JUP LAX1 TNIP1 | 0.94 (0.91-0.98) | 0.95 (0.92-0.99) |
| Benchmark with Sample Preservative | Sample_Preservative CTSB GPAA1 HK3 IFI27 JUP LAX1 TNIP1 | 0.86 (0.81-0.91) | 0.90 (0.86-0.95) | Sample_Preservative CTSB GPAA1 HK3 1FI27 JUP LAX 1 TNIP1 | 0.96 (0.94-0.98) | 0.97 (0.94-0.99) |

Example 2

**Prototype GeneXpert Cartridge Analysis**

A prototype cartridge was developed to demonstrate proof-of-principle for translation of mRNA signatures into a Cepheid GeneXpert compatible assay. The Cepheid prototype Xpert Bacterial vs Viral assay is a multiplex RT-PCR assay that measures 5 mRNA targets (RUNX1, LY6E, IRF9, ITGAM, PSTPIP2) and one sample adequacy control (SAC), ABL1. In this study, whole blood specimens from patients with known infections were tested on the assay to evaluate the targets and refine prediction models.

Testing was performed with residual de-identified specimens that were collected as part of standard of care (SOC). Inclusion criteria included a new onset of fever (>37.5°C) or otherwise suspected of having an infection and a confirmed positive test result for infection. Whole blood in EDTA was drawn within 72 hours of enrollment and stored at 4°C to minimize RNA degradation. Assay testing followed the package insert: 200 µl whole blood was added to a lysis buffer, mixed, and 1mL was added to the cepheid cartridge. Test run time was 35 minutes. The specimens were classified as either viral or bacterial based on the culture or molecular diagnostic SOC result. The Ct parameters for the six targets were combined in a logistic regression model for predicting bacterial infection and an ROC curve was created to estimate clinical performance.

208 patient samples were enrolled and tested. 111 were from patients with a bacterial infection, 95 with viral infections (including 65 positive for SARS-CoV-2), 1 Candida infection, and 1 mixed viral and bacterial infection. Initial testing found a non-determinate rate of the assay to be 5.9%, 2 were cartridge errors and 12 failed SAC. The bacterial prediction model had an AUCROC of 0.91 using re-substitution. The results are shown in Table 10.

A SARS-CoV-2 yes/no model was also determined using nominal logistic fit. The SARS-CoV-2 prediction model had an AUC of 0.897. The results are shown in Table 11.

**Table 10. Bacterial yes/no model made with Ct values for all 6 targets and using nominal logistic fit**

| Parameter Estimates | | | | |
|---|---|---|---|---|
| **Term** | **Estimate** | **Std Error** | **Chi-Square** | **Pr > ChiSq** |
| **Intercept** | -10.493194 | 5.256359 | 3.99 | 0.0459 |
| **RUNX1 Ct** | 0.63977159 | 0.3933218 | 2.65 | 0.1038 |
| **LY6E Ct** | -0.9488302 | 0.2056103 | 21.30 | <.0001 |
| **IRF9 Ct** | 0.19331711 | 0.4198852 | 0.21 | 0.6452 |
| **ITGAM Ct** | -0.7658186 | 0.3707695 | 4.27 | 0.0389 |
| **ABL1 Ct** | -0.2978481 | 0.1825711 | 2.66 | 0.1028 |
| **PSTPIP2 Ct** | 1.52680986 | 0.4391648 | 12.09 | 0.0005 |

**Table 11. SARS-CoV-2 yes/no model**

| Effect Likelihood Ratio Tests | | | | |
|---|---|---|---|---|
| **Source** | **Nparm** | **DF** | **L-R Chi-Square** | **Pr > ChiSq** |
| **RUNX1 Ct** | 1 | 1 | 3.9528366 | 0.0468 |
| **LY6E Ct** | 1 | 1 | 22.786822 | <0.0001 |
| **IRF9 Ct** | 1 | 1 | 0.7636536 | 0.3822 |
| **ITGAM Ct** | 1 | 1 | 2.9431935 | 0.0862 |
| **ABL1 Ct** | 1 | 1 | 5.9422481 | 0.0148 |
| **PSTPIP2 Ct** | 1 | 1 | 8.6711243 | 0.0032 |

The results demonstrated that the prototype Xpert Bacterial vs Viral assay can be used to differentiate bacterial vs viral infections from patient samples.

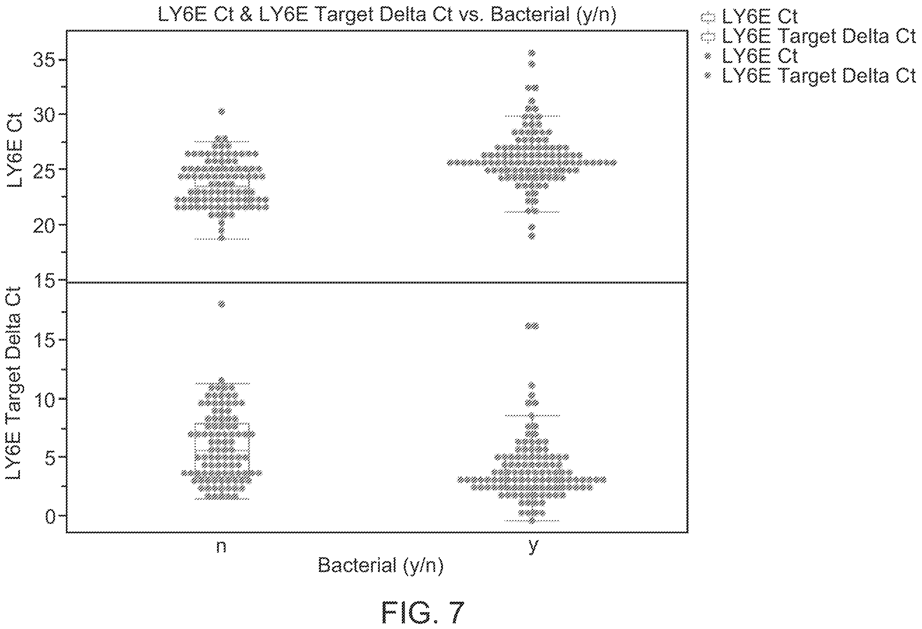

FIG. 7 shows a boxplot of the measured expression levels, expressed as a cycle threshold (Ct) value, and change in cycle threshold (Delta Ct) value, of LY6E vs. bacterial infection status. The Delta Ct value was calculated by subtracting the expression Ct value of LY6E from the Ct value of ABL1 in each sample.

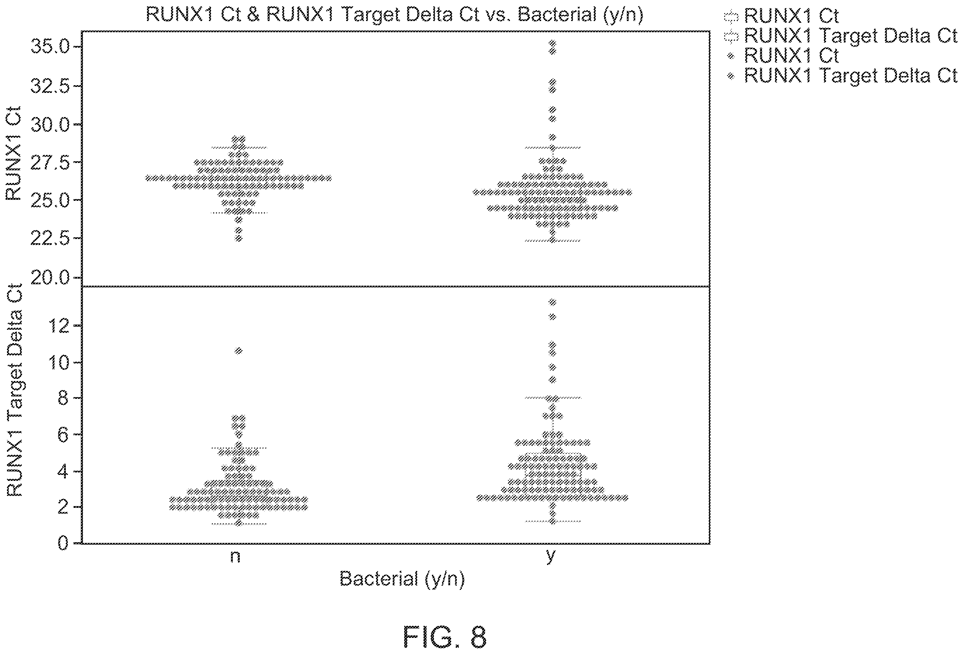

FIG. 8 shows a boxplot of the measured expression levels, expressed as a cycle threshold (Ct) value, and change in cycle threshold (Delta Ct) value, of RUNX1 vs. bacterial infection status. The Delta Ct value was calculated by subtracting the expression Ct value of RUNX1 from the Ct value of ABL1 in each sample.

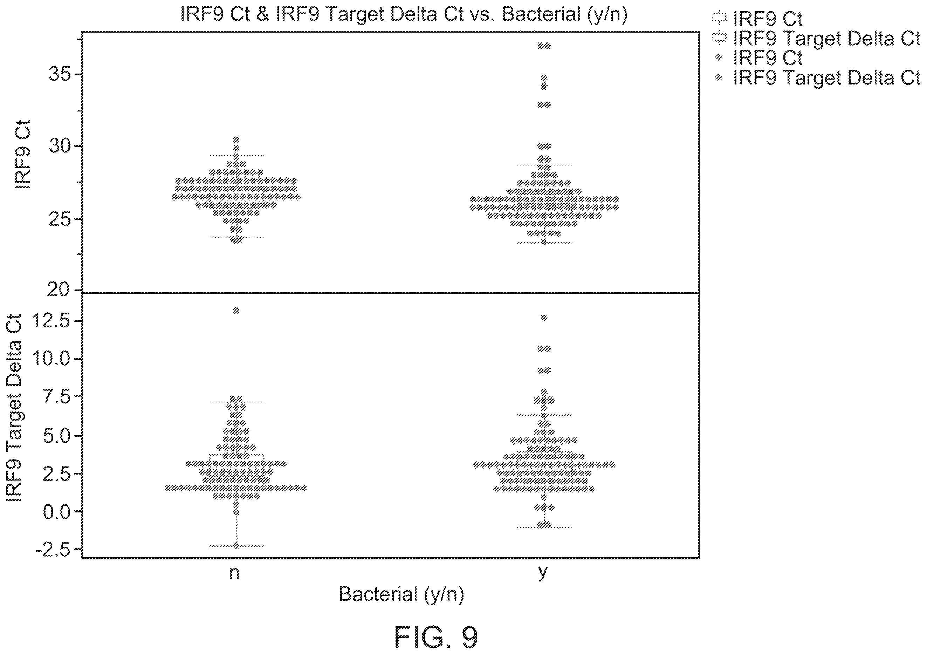

FIG. 9 shows a boxplot of the measured expression levels, expressed as a cycle threshold (Ct) value, and change in cycle threshold (Delta Ct) value, of IRF9 vs. bacterial infection status. The Delta Ct value was calculated by subtracting the expression Ct value of IRF9 from the Ct value of ABL 1 in each sample.

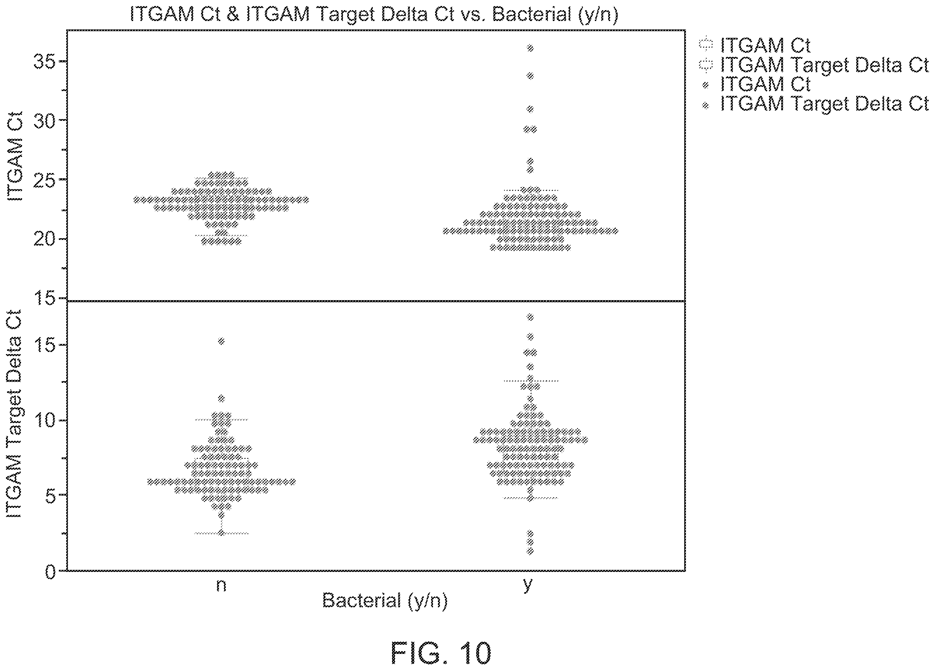

FIG. 10 shows a boxplot of the measured expression levels, expressed as a cycle threshold (Ct) value, and change in cycle threshold (Delta Ct) value, of ITGAM vs. bacterial infection status. The Delta Ct value was calculated by subtracting the expression Ct value of ITGAM from the Ct value of ABL1 in each sample.

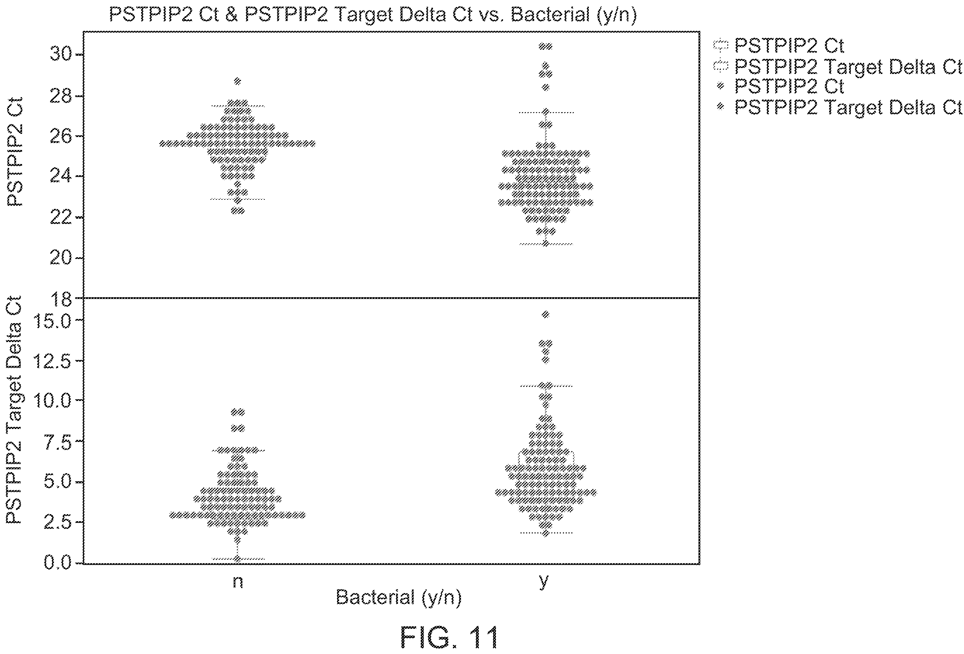

FIG. 11 shows a boxplot of the measured expression levels, expressed as a cycle threshold (Ct) value, and change in cycle threshold (Delta Ct) value, of PSTPIP2 vs. bacterial infection status. The Delta Ct value was calculated by subtracting the expression Ct value of PSTPIP2 from the Ct value of ABL1 in each sample.

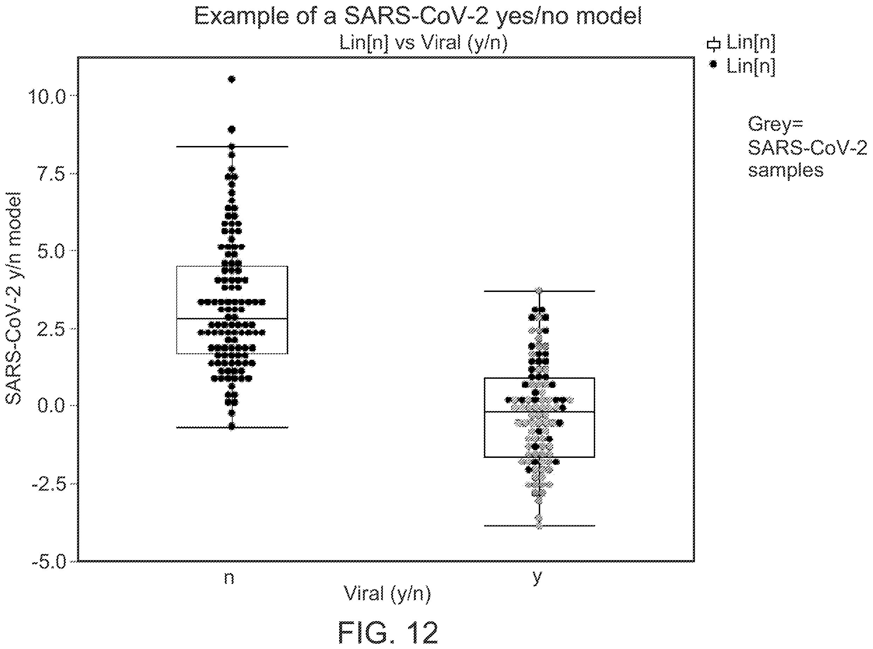

FIG. 12 shows a boxplot of the SARS-CoV-2 yes/no model vs. viral infection status.

<u>Example 3</u>

**Prototype GeneXpert Cartridge Analysis**

In this study, whole blood specimens from patients with new onset of fever were tested on the Cepheid prototype Xpert Bacterial vs Viral assay to evaluate the targets and refine prediction models. The assay is a multiplex RT-PCR assay that measures 5 mRNA targets (RUNX1, LY6E, IRF9, ITGAM, PSTPIP2) and one sample adequacy control (SAC), ABL1.

Testing was performed with residual de-identified specimens that were collected as part of standard of care (SOC). Inclusion criteria included a new onset of fever (>37.5°C) within the past 3 days or otherwise suspected of having an infection. Whole blood in EDTA was drawn within 72 hours of enrollment and stored at 4°C to minimize RNA degradation. Assay testing followed the package insert: 200 µl whole blood was added to a lysis buffer, mixed, and 1mL was added to the cepheid cartridge. Test run time was 35 minutes. The specimens were classified as either viral or bacterial based on the culture or molecular diagnostic SOC result. The Ct parameters for the six targets were combined in a logistic regression model for predicting bacterial infection and an ROC curve was created to estimate clinical performance.

54 patient samples were enrolled and tested. 35 were from patients with a "clear" diagnosis and 19 were excluded pending further questions. 12 external control samples were tested. 70 tests were conducted. Initial testing found a non-determinate rate of the assay to be 8.6% (6/70), 1 was cartridge error and 5 invalid (ABL1 Ct >35).

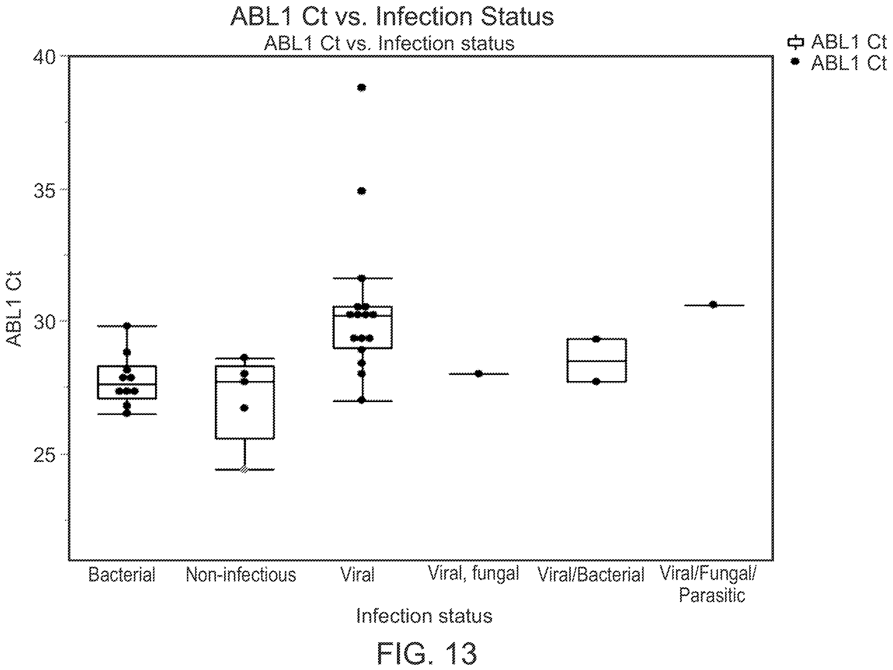

FIG. 13 shows a boxplot of the measured expression levels, expressed as a cycle threshold (Ct) value, of ABL1 vs. infection status. Table 12 shows the count of infection status based on Figure 13.

**Table 12. Count of Infection Status**

| Infection | Count of Infection Status |
|---|---|
| Bacterial | 10 |
| Non-infectious | 5 |
| Viral | 16 |
| Viral, fungal | 1 |
| Viral, bacterial | 2 |
| Viral, fungal, parasitic | 1 |
| Total | 35 |

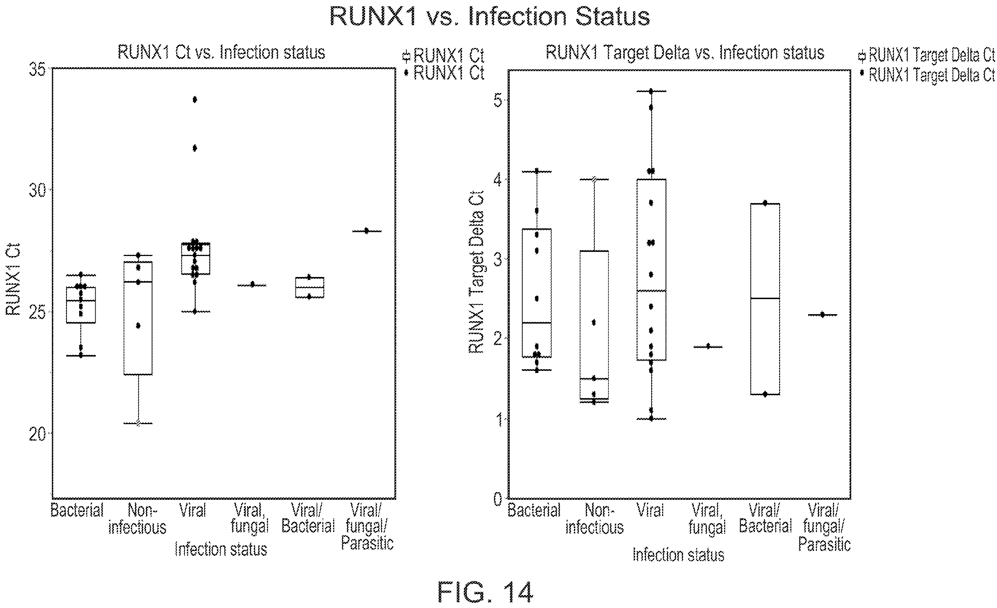

FIG. 14 shows a boxplot of the measured expression levels, expressed as a cycle threshold (Ct) value (left), and change in cycle threshold (Delta Ct) value (right), of RUNX1 vs. infection status. The Delta Ct value was calculated by subtracting the expression Ct value of RUNX1 from the Ct value of ABL1 in each sample.

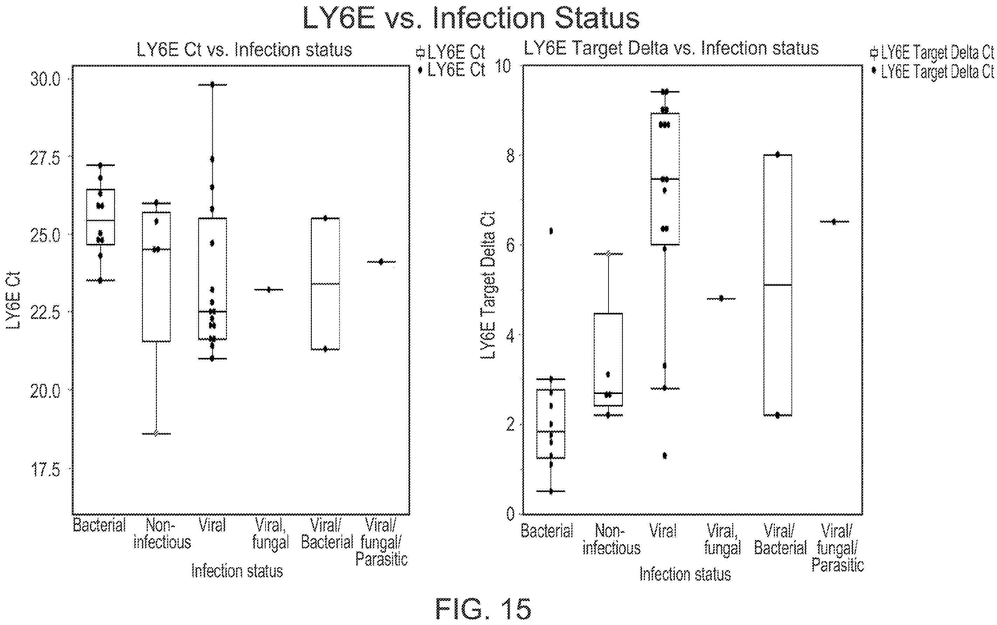

FIG. 15 shows a boxplot of the measured expression levels, expressed as a cycle threshold (Ct) value (left), and change in cycle threshold (Delta Ct) value (right), of LY6E vs. infection status. The Delta Ct value was calculated by subtracting the expression Ct value of LY6E from the Ct value of ABL1 in each sample.

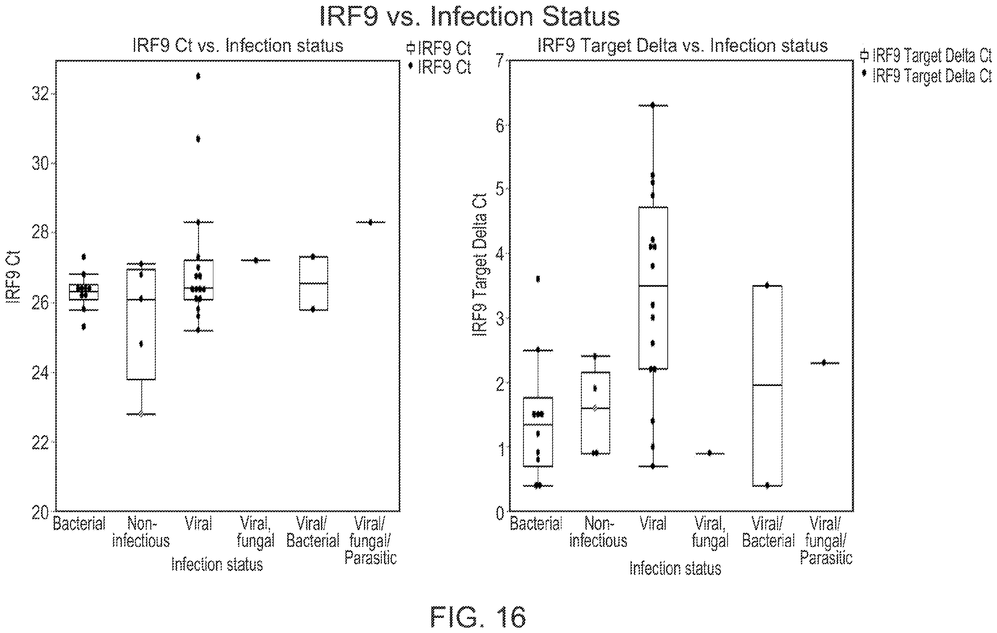

FIG. 16 shows a boxplot of the measured expression levels, expressed as a cycle threshold (Ct) value (left), and change in cycle threshold (Delta Ct) value (right), of IRF9 vs. infection status. The Delta Ct value was calculated by subtracting the expression Ct value of IRF9 from the Ct value of ABL1 in each sample.

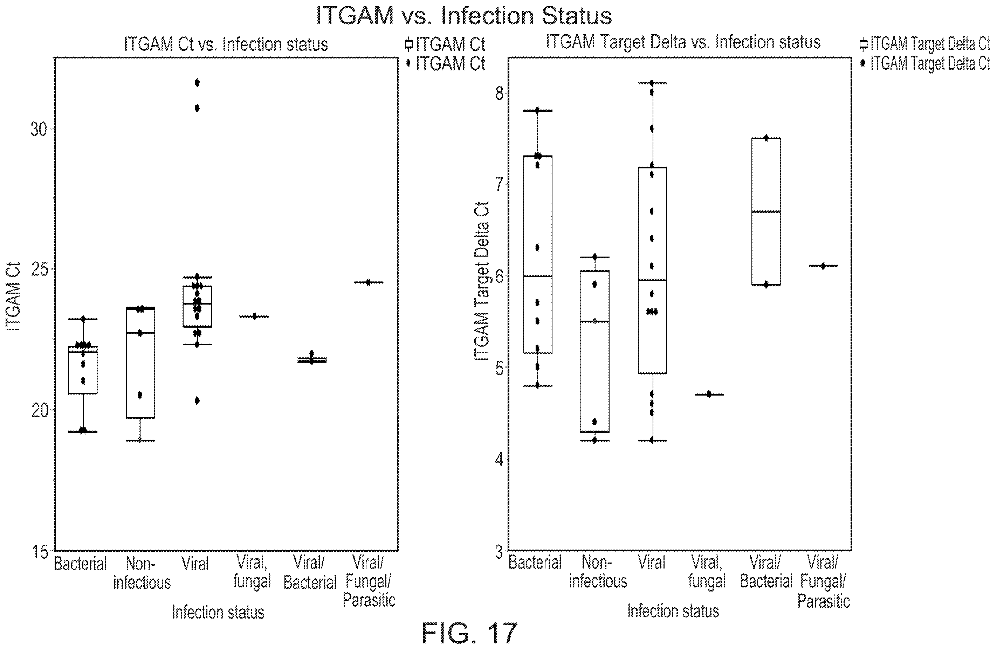

FIG. 17 shows a boxplot of the measured expression levels, expressed as a cycle threshold (Ct) value (left), and change in cycle threshold (Delta Ct) value (right), of ITGAM vs. infection status. The Delta Ct value was calculated by subtracting the expression Ct value of ITGAM from the Ct value of ABL1 in each sample.

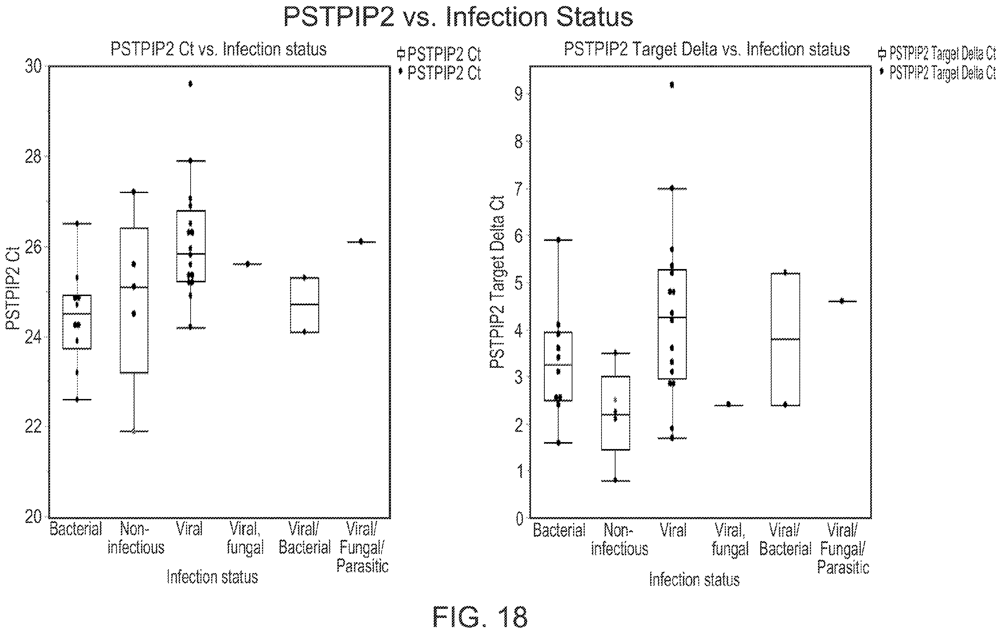

FIG. 18 shows a boxplot of the measured expression levels, expressed as a cycle threshold (Ct) value (left), and change in cycle threshold (Delta Ct) value (right), of PSTPIP2 vs. infection status. The Delta Ct value was calculated by subtracting the expression Ct value of PSTPIP2 from the Ct value of ABL1 in each sample.

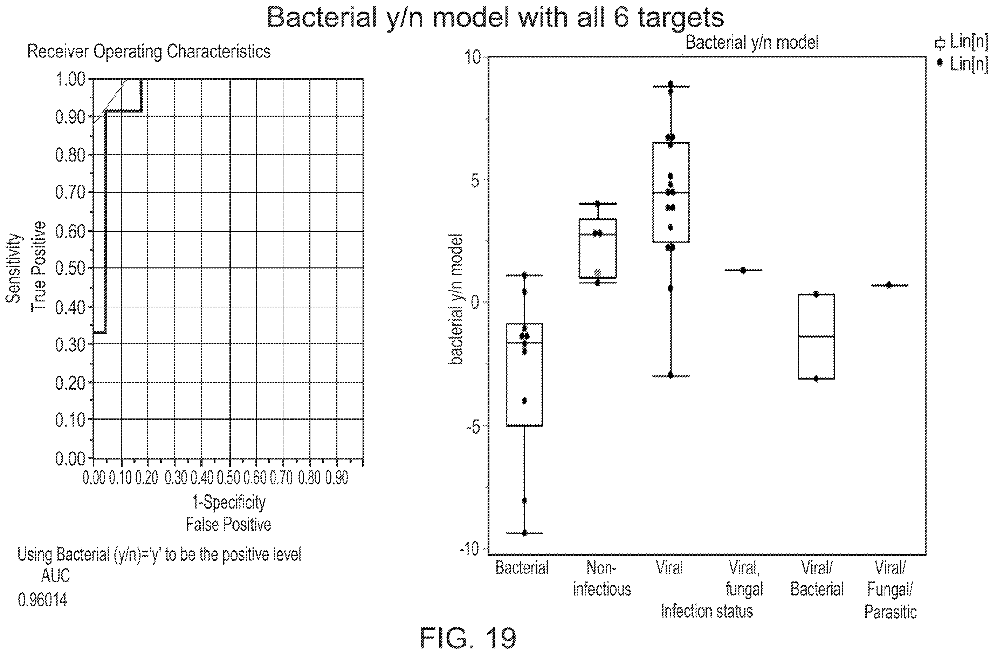

FIG. 19 shows a boxplot of the bacterial y/n model vs. infection status. The bacterial prediction model had an AUCROC of 0.96 using re-substitution.

Example 4

**Nasopharyngeal eNAT sample testing using Xpert BvV assay**

In this study, two nasopharyngeal samples from patients with known infections were tested on the assay to evaluate if mRNA targets can be detected in respiratory samples. The Cepheid prototype Xpert Bacterial vs Viral assay was used that measures 5 mRNA targets (RUNX1, LY6E, IRF9, ITGAM, PSTPIP2) and one sample adequacy control (SAC), ABL1. Respiratory samples could be a more convenient specimen type to collect for patients with respiratory symptoms compared to blood. Testing was performed using the cepheid cartridge.

The Ct parameters for the six targets are shown in Table 14.

**Table 14. Results with two eNAT NP samples.**

| Analyte | Sample 1 | | | Sample 2 | | |
|---|---|---|---|---|---|---|
| | Ct | Endpoint Fluorescence | Analyte Result | Ct | Analyte Result | Analyte Result |
| RUNX1 Ct | 30.2 | 92 | POS | 26.9 | 171 | POS |
| LY6E Ct | 28.1 | 258 | POS | 25.0 | 433 | POS |
| IRF9 Ct | 30.0 | 274 | POS | 26.6 | 695 | POS |
| ITGAM Ct | 0.0 | 11 | NEG | 0.0 | 20 | NEG |
| ABL1 Ct | 30.2 | 135 | PASS | 27.0 | 359 | PASS |
| PSTPIP2 Ct | 38.1 | 22 | POS | 31.6 | 45 | POS |

The results demonstrated detection of 4 of the Bacterial vs Viral mRNA targets in eNAT NP samples.

Example 5

**MIS-C sample testing using Xpert BvV assay**

In this study, specimens from patients were tested on the Cepheid prototype Xpert Bacterial vs Viral assay to evaluate the targets and refine prediction models. The assay is a multiplex RT-PCR assay that measures 5 mRNA targets (RUNX1, LY6E, IRF9, ITGAM, PSTPIP2) and one sample adequacy control (SAC), ABL1.

103 unique samples from 91 unique patients were tested. 16 samples were from patients with a bacterial diagnosis, 28 samples were from 26 unique patients with a healthy diagnosis, 17 samples were from 10 unique patients with a MISC-C diagnosis, 11 samples were from patients with a non-infectious diagnosis, 30 samples were from patients with a viral diagnosis, and 1 sample was from a patient with other diagnosis. 136 tests were conducted. Initial testing found a non-determinate rate of the assay to be 4.4% (6/136) due to cartridge error and invalid (ABL 1 Ct >35).

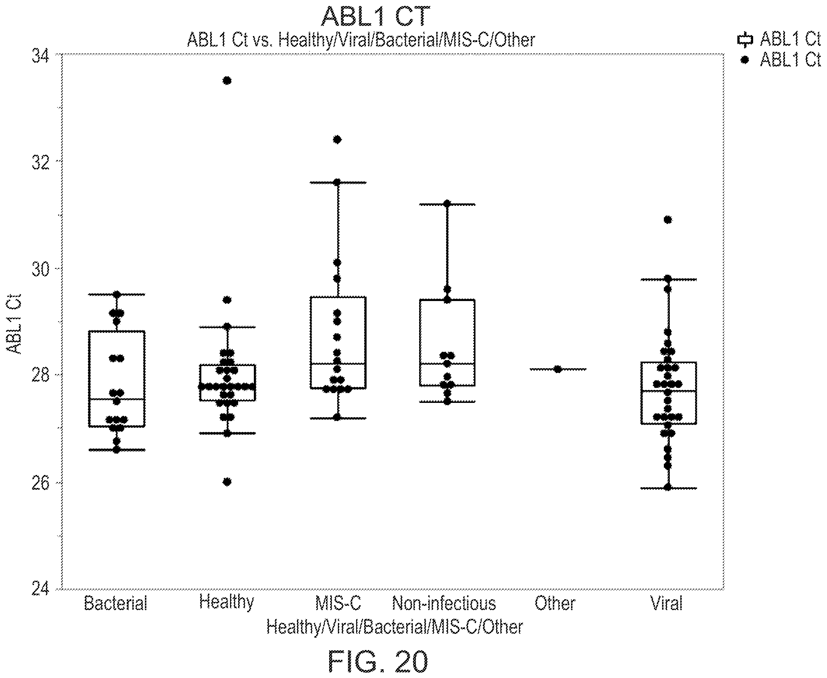

FIG. 20 shows a boxplot of the measured expression levels, expressed as a cycle threshold (Ct) value, of ABL1 vs. healthy/viral/bacterial/MIS-C/other infection status.

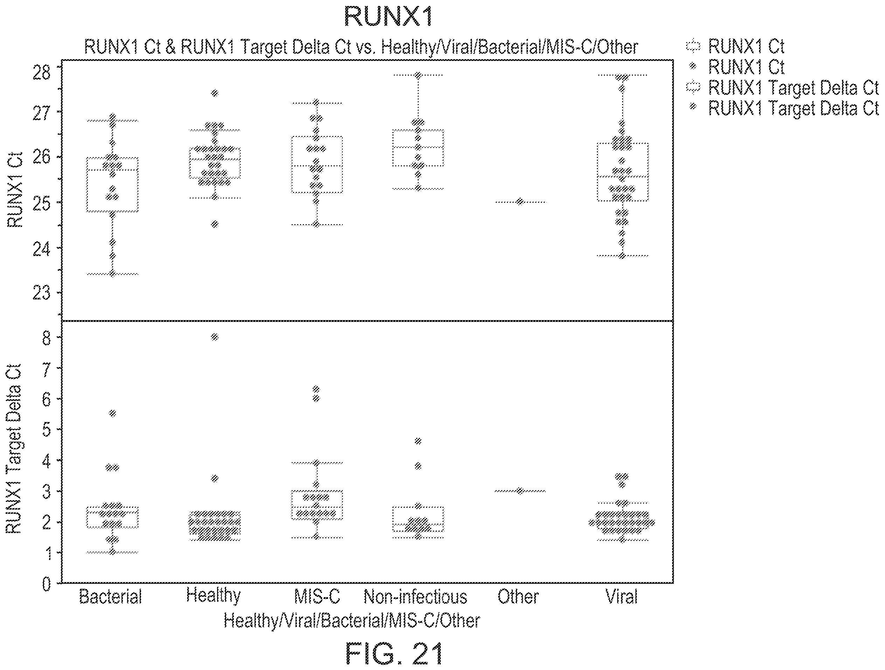

FIG. 21 shows a boxplot of the measured expression levels, expressed as a cycle threshold (Ct) value (top), and change in cycle threshold (Delta Ct) value (bottom), of RUNX1 vs. healthy/viral/bacterial/MIS-C/other infection status. The Delta Ct value was calculated by subtracting the expression Ct value of RUNX1 from the Ct value of ABL1 in each sample.

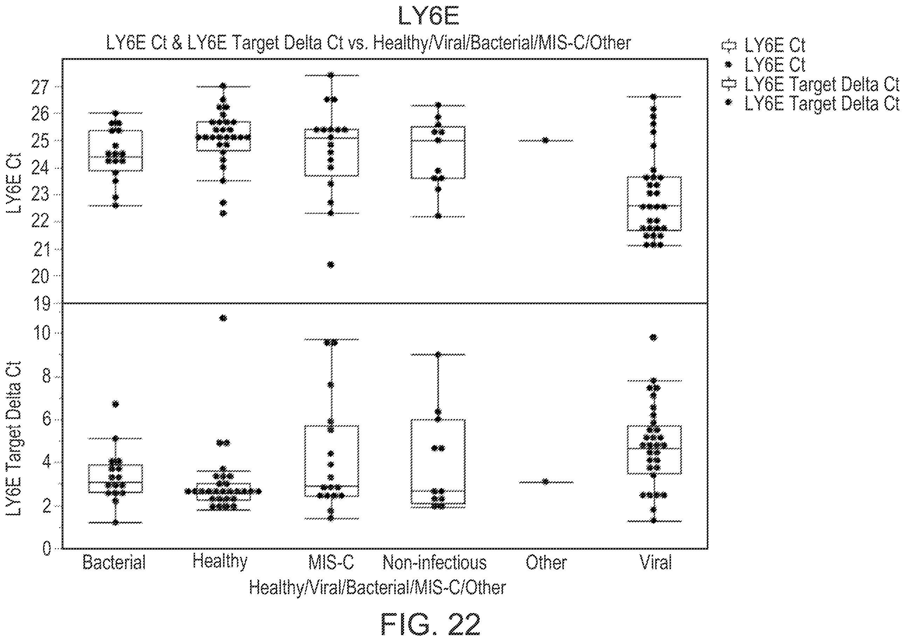

FIG. 22 shows a boxplot of the measured expression levels, expressed as a cycle threshold (Ct) value (top), and change in cycle threshold (Delta Ct) value (bottom), of LY6E vs. healthy/viral/bacterial/MIS-C/other infection status. The Delta Ct value was calculated by subtracting the expression Ct value of LY6E from the Ct value of ABL1 in each sample.

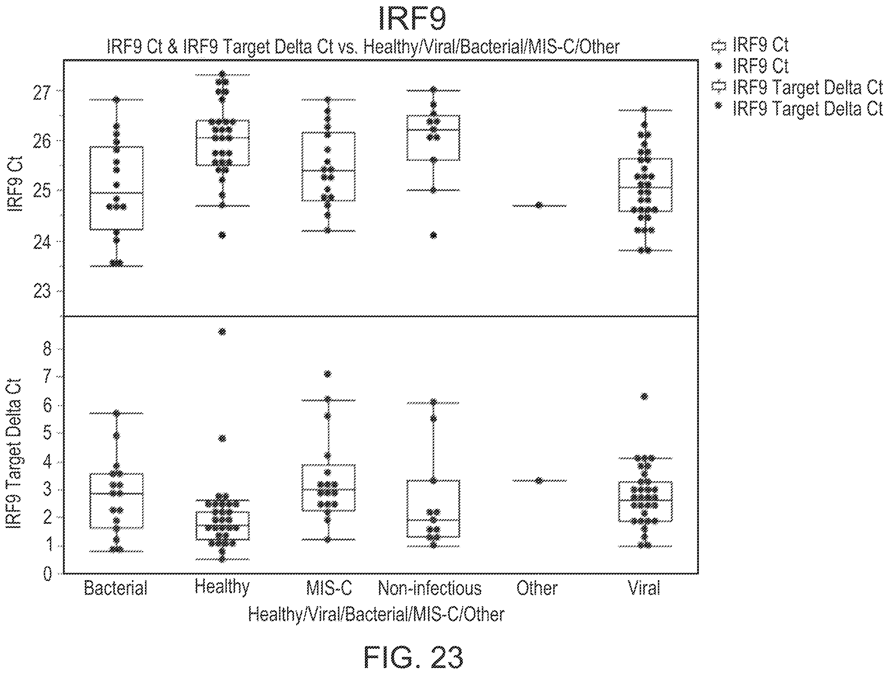

FIG. 23 shows a boxplot of the measured expression levels, expressed as a cycle threshold (Ct) value (top), and change in cycle threshold (Delta Ct) value (bottom), of IRF9 vs. healthytviral/bacterial/MIS-C/other infection status. The Delta Ct value was calculated by subtracting the expression Ct value of IRF9 from the Ct value of ABL1 in each sample.

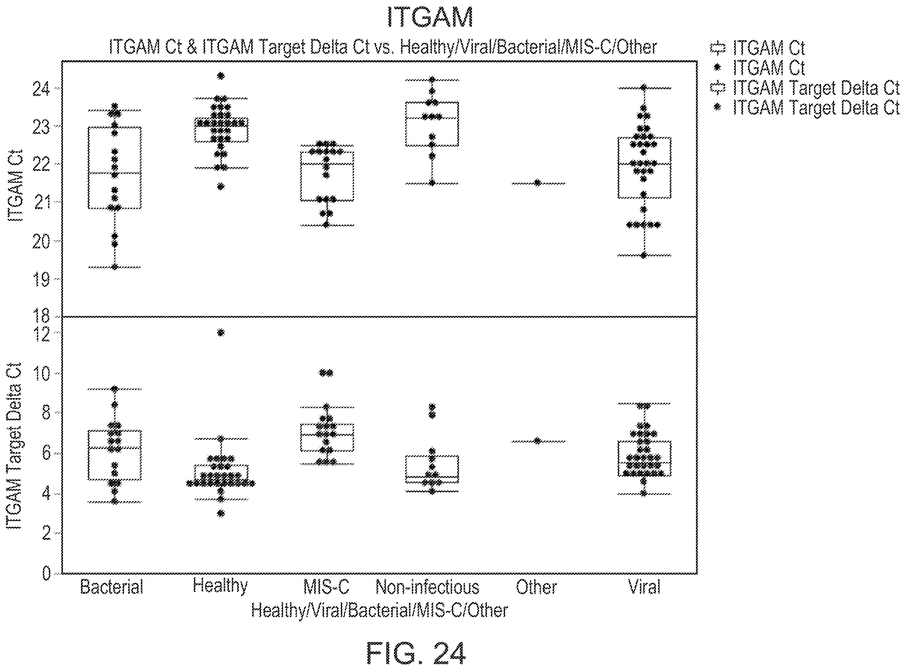

FIG. 24 shows a boxplot of the measured expression levels, expressed as a cycle threshold (Ct) value (top), and change in cycle threshold (Delta Ct) value (bottom), of ITGAM vs. healthy/viral/bacterial/MIS-C/other infection status. The Delta Ct value was calculated by subtracting the expression Ct value of ITGAM from the Ct value of ABL1 in each sample.

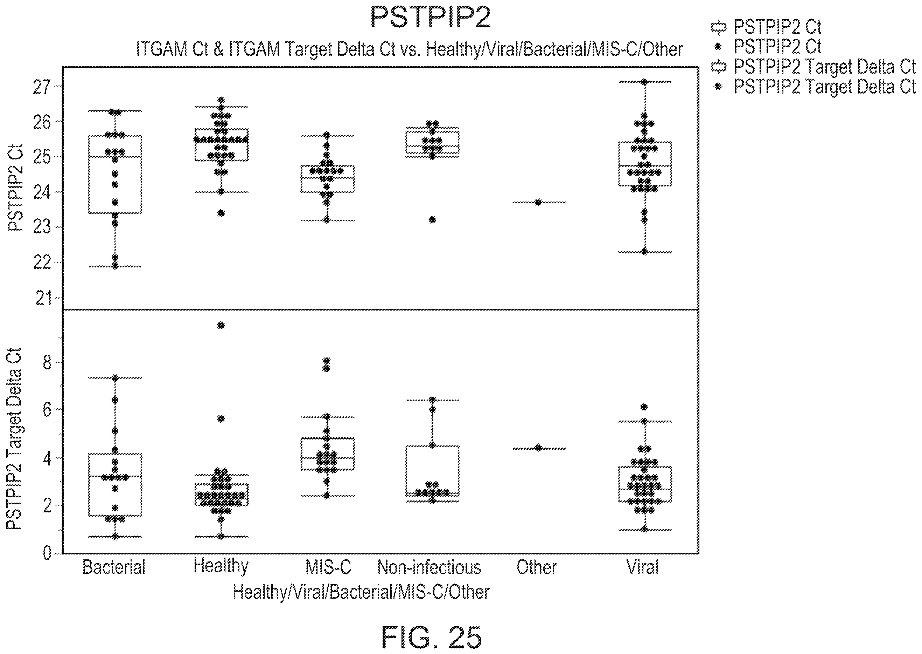

FIG. 25 shows a boxplot of the measured expression levels, expressed as a cycle threshold (Ct) value (top), and change in cycle threshold (Delta Ct) value (bottom), of PSTPIP2 vs. healthy/viral/bacterial/MIS-C/other infection status. The Delta Ct value was calculated by subtracting the expression Ct value of PSTPIP2 from the Ct value of ABL1 in each sample.

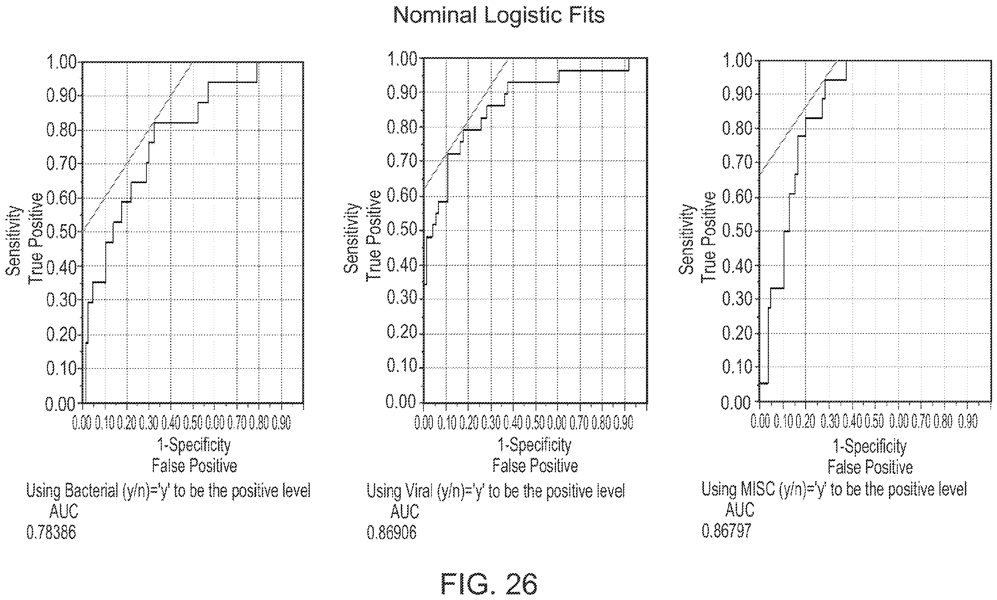

FIG. 26 shows nominal logistic fits for a bacterial y/n model, viral y/n model, and MIS-C y/n model. The bacterial prediction model had an AUCROC of 0.784 using re-substitution. The viral prediction model had an AUCROC of 0.869 using re-substitution. The MIS-C prediction model had an AUCROC of 0.868 using re-substitution.

Example 6

**Prototype GeneXpert Cartridge Analysis in Patients with Lyme Disease**

3 samples from patients with Lyme disease in TC2 (training cohort 2) had different expression profiles for some targets including ABL1 and IRF9. Additional samples from patients with Lyme disease were ordered and tested to determine if the same expression patterns would be observed and if a Lyme disease-specific model could be built to diagnose Lyme disease.
In this example, 23 banked blood samples in PAXgene from patients with Borrelia and other tick-born infections were obtained. 3 were excluded from analysis due to unclear or negative clinical test results. RNA was purified and tested in 96-well plate assays to measure 38 mRNA targets. The data was analyzed as part of a larger cohort of samples called TC4 (training cohort 4) as outlined in Table 15.

**Table 15.**

| Infection Status | Total Number of samples | Number of samples with Borrelia |
|---|---|---|
| Bacterial | 102 | 13 |
| Bacterial, Parasitic | 10 | 4 (coinfection with Bebesia) |
| Bacterial, Viral | 6 | |
| healthy control | 40 | |
| Parasitic | 21 | |
| Symptomatic not infected | 29 | |
| Viral | 92 | |
| **Grand Total** | **300** | |

The results demonstrated that the prototype Xpert Bacterial vs Viral assay can be used to provide a Lyme disease-specific model driven by downregulation of IRF9 to predict Lyme disease. Since both IRF9 and ABL1 detection are included in the Xpert BvV assay, this assay could be used with a Lyme disease specific algorithm to diagnose Lyme disease.

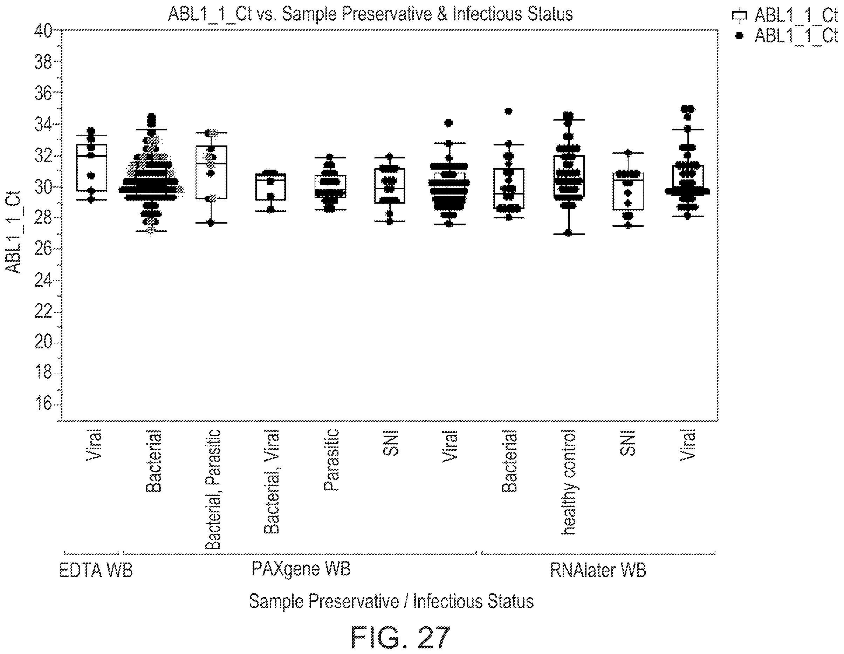

FIG. 27 shows a boxplot of the measured expression levels, expressed as a cycle threshold (Ct) value, of ABL1 vs. sample preservative and infection status.

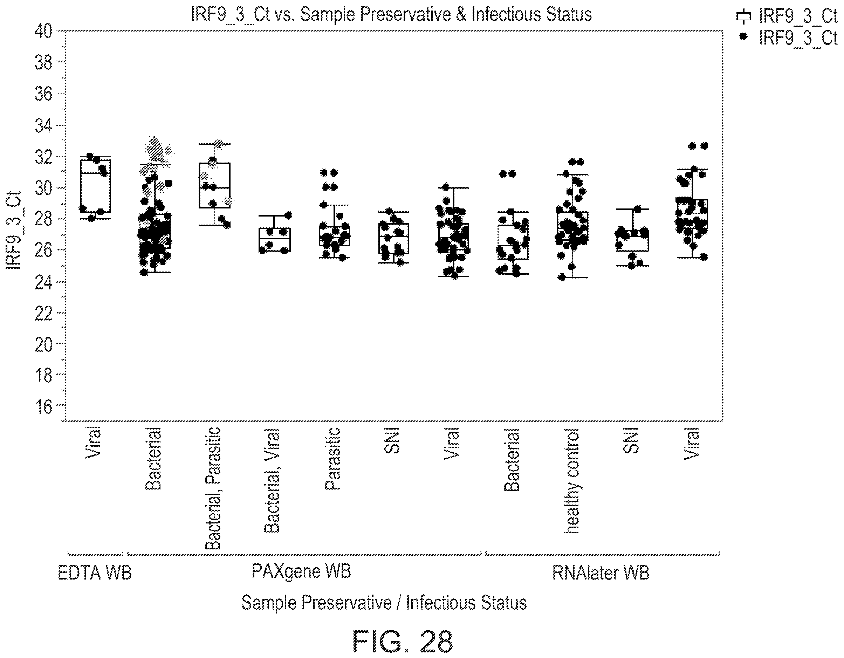

FIG. 28 shows a boxplot of the measured expression levels, expressed as a cycle threshold (Ct) value, of IRF9 vs. sample preservative and infection status.

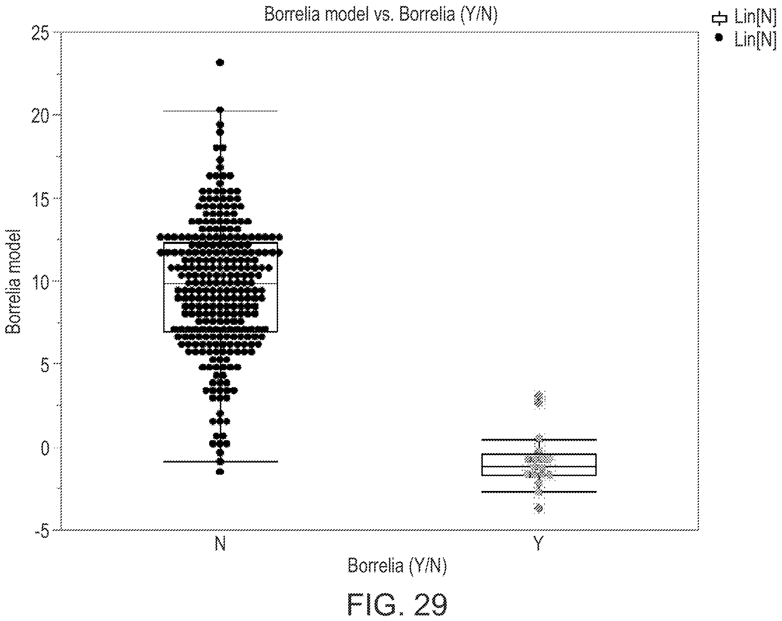

FIG. 29 shows a boxplot of the Borrelia yes/no model determined using nominal logistic fit. The Borrelia prediction model had an AUC of 0.99127.
While various specific embodiments have been illustrated and described, it will be appreciated that changes can be made without departing from the scope of the invention(s).

**Claims**

1.  A method of determining if a subject has a bacterial infection, a viral infection, or a non-infectious cause of favor, the method comprising:

    a) determining the mRNA expression levels of RUNX1, ITGAM, PSTPIP2, LY6E, IRF9 and ABL1 in a blood sample from the subject, wherein the mRNA expression of ABL1 is used as a positive control biomarker that is indicative of the quality of the blood sample;
    b) determining a viral infection score and a bacterial infection score based on the mRNA expression levels of RUNX1, ITGAM, PSTPIP2, LY6E and IRF9;
    c) comparing the viral infection score to a first predetermined cutoff value and the bacterial infection score to a second predetermined cutoff value and comparing the mRNA expression level of ABL1 to a third predetermined cutoff value; and

d) determining:

that the subject has a viral infection when the viral infection score is greater than the first predetermined cutoff value and the mRNA expression level of ABL1 is greater than the third predetermined cutoff value; that the subject does not have viral infection when the viral infection score is less than the first predetermined cutoff value and the mRNA expression level of ABL1 is greater than the third predetermined cutoff value; that the subject has a bacterial infection when the bacterial infection score is greater than the second predetermined cutoff value and the mRNA expression level of ABL1 is greater than the third predetermined cutoff value that the subject does not have a bacterial infection when the bacterial infection score is less than or equal to the second predetermined cutoff value and the mRNA expression level of ABL 1 is greater than the third predetermined cutoff value; and

that the subject has a non-infectious cause of fever in the subject when the viral infection score is less than the first predetermined cutoff value, the bacterial infection score is less than the second predetermined cutoff value and the mRNA expression level of ABL1 is greater than the third predetermined cutoff value.

2. The method of claim 1, wherein the subject is identified as having both a bacterial infection and a viral infection when the viral infection score is greater than the first predetermined cutoff value, the bacterial infection score is greater than the second predetermined cutoff value and the mRNA expression level of ABL1 is greater than the third predetermined cutoff value.

3. The method of any one of the preceding claims, wherein determining a viral infection score based on the mRNA expression levels of RUNX1, ITGAM, PSTPIP2, LY6E and IRF9 comprises:

(a) inputting the mRNA expression levels of RUNX1, ITGAM, PSTPIP2, LY6E, IRF9 and optionally ABL1, into an algorithm that determines the viral infection score; or
(b) inputting the normalized mRNA expression levels of RUNX1, ITGAM, PSTPIP2, LY6E and IRF9 into an algorithm that determines the viral infection score, preferably wherein the normalized mRNA expression levels of RUNX1, ITGAM, PSTPIP2, LY6E and IRF9 are obtained by normalizing the mRNA expression levels of RUNX1, ITGAM, PSTPIP2, LY6E and IRF9 determined in step (a) to the mRNA expression level of ABL1 determined in step (a).

4. The method of any one of the preceding claims, wherein determining a bacterial infection score based on the mRNA expression levels of RUNX1, ITGAM, PSTPIP2, LY6E and IRF9 comprises:

(a) inputting the mRNA expression levels of RUNX1, ITGAM, PSTPIP2, LY6E, IRF9 and optionally ABL1, into an algorithm that determines the bacterial infection score; or
(b) inputting the normalized mRNA expression levels of RUNX1, ITGAM, PSTPIP2, LY6E and IRF9 into an algorithm that determines the bacterial infection score, preferably wherein the normalized mRNA expression levels of RUNX1, ITGAM, PSTPIP2, LY6E and IRF9 are obtained by normalizing the mRNA expression levels of RUNX1, ITGAM, PSTPIP2, LY6E and IRF9 determined in step (a) to the mRNA expression level of ABL1 determined in step (a).

5. The method of claim 3 or claim 4, wherein the algorithm is a product of a logistic regression model generated using the mRNA expression levels of RUNX1, ITGAM, PSTPIP2, LY6E, IRF9 and optionally ABL1, measured in a training set of blood samples, wherein the training set of blood samples comprises:

i) a plurality of blood samples isolated from subjects identified as having a bacterial infection;
ii) a plurality of blood samples isolated from subjects identified as having a viral infection;
iii) a plurality of blood samples isolated from subjects identified has having both a bacterial infection and a viral infection;
iv) a plurality of blood samples isolated from subjects identified as having a non-infectious cause of fever; and
v) a plurality of blood samples isolated from healthy subjects not having a bacterial infection, viral infection or non-infectious cause of fever.
preferably wherein the mRNA expression levels of RUNX1, ITGAM, PSTPIP2, LY6E and IRF9 measured in the training set of blood samples are normalized mRNA expression levels of RUNX1, ITGAM, PSTPIP2, LY6E and IRF9 obtained by normalizing the mRNA expression levels of RUNX1, ITGAM, PSTPIP2, LY6E and IRF9 measured in the training set of blood samples to the mRNA expression level of ABL1 measured in the training set of blood samples,

preferably wherein the logistic regression model is a product of univariate analysis and/or multivariate analysis of the mRNA expression levels of RUNX1, ITGAM, PSTPIP2, LY6E, IRF9 and optionally, ABL1, in the training set of blood samples.

6. The method of any one of claims 3-5, wherein the algorithm that determines the viral infection score is:

$$p = \frac{1}{1+e^{-(b+ m_1*RUNX1+ m_2*ITGAM + m_3*PSTPIP2+ m_4*LY6E+ m_5*IRF9)}} \text{ or }$$

$$p = \frac{1}{1+e^{-(b+ m_1*RUNX1+ m_2*ITGAM + m_3*PSTPIP2+ m_4*LY6E+ m_5*IRF9+ m_6*ABL1)}};$$

wherein *RUNX1* is the mRNA expression level or the normalized mRNA expression level of RUNX1 in the blood sample from the subject,

*ITGAM* is the mRNA expression level or the normalized mRNA expression level of ITGAM in the blood sample from the subject,

*PSTPIP2* is the mRNA expression level or the normalized mRNA expression level of PSTPIP2 in the blood sample from the subject,

*LY6E* is the mRNA expression level or the normalized mRNA expression level of LY6E in the blood sample from the subject,

*IRF9* is the mRNA expression level or the normalized mRNA expression level of IRF9 in the blood sample from the subject,

*ABL1* is the mRNA expression level of ABL1 in the blood sample from the subject, and

*b* is an intercept,

$m_1$ is a scaling factor for the mRNA expression level or the normalized mRNA expression level of RUNX,

$m_2$ is a scaling factor for the mRNA expression level or the normalized mRNA expression level of ITGAM,

$m_3$ is a scaling factor for the mRNA expression level or the normalized mRNA expression level of PSTPIP2,

$m_4$ is a scaling factor for the mRNA expression level or the normalized mRNA expression level of LY6E,

$m_5$ is a scaling factor for the mRNA expression level or the normalized mRNA expression level of IRF9,

$m_6$ is a scaling factor for the mRNA expression level of ABL1,

preferably wherein the normalized mRNA expression levels of RUNX1, ITGAM, PSTPIP2, LY6E and IRF9 are obtained by normalizing the mRNA expression levels of RUNX1, ITGAM, PSTPIP2, LY6E and IRF9 determined in step (a) to the mRNA expression level of ABL1 determined in step (a),

preferably wherein $b$, $m_1$, $m_2$, $m_3$, $m_4$, $m_5$ and/or $m_6$ are derived from the logistic regression model used to produce the algorithm.

7. The method of any one of claims 3-6, wherein the algorithm that determines the bacterial infection score is:

$$p = \frac{1}{1+e^{-(b+ m_1*RUNX1\ m_2*ITGAM + m_3*PSTPIP2+ m_4*LY6E+ m_5*IRF9)}}$$

or

$$p = \frac{1}{1+e^{-(b+ m_1*RUNX1+ m_2*ITGAM + m_3*PSTPIP2+ m_4*LY6E+ m_5*IRF9+ m_6*ABL1)}};$$

wherein *RUNX1* is the mRNA expression level or the normalized mRNA expression level of RUNX1 in the blood sample from the subject,

*ITGAM* is the mRNA expression level or the normalized mRNA expression level of ITGAM in the blood sample from the subject,

*PSTPIP2* is the mRNA expression level or the normalized mRNA expression level of PSTPIP2 in the blood sample from the subject,

*LY6E* is the mRNA expression level or the normalized mRNA expression level of LY6E in the blood sample from the subject,

*IRF9* is the mRNA expression level or the normalized mRNA expression level of IRF9 in the blood sample from the subject,

*ABL1* is the mRNA expression level of ABL1 in the blood sample from the subject, and

*b* is an intercept,

$m_1$ is a scaling factor for the mRNA expression level or the normalized mRNA expression level of RUNX,

$m_2$ is a scaling factor for the mRNA expression level or the normalized mRNA expression level of ITGAM,

$m_3$ is a scaling factor for the mRNA expression level or the normalized mRNA expression level of PSTPIP2,

$m_4$ is a scaling factor for the mRNA expression level or the normalized mRNA expression level of LY6E,

$m_5$ is a scaling factor for the mRNA expression level or the normalized mRNA expression level of IRF9,

$m_6$ is a scaling factor for the mRNA expression level of ABL1,

preferably wherein the normalized mRNA expression levels of RUNX1, ITGAM, PSTPIP2, LY6E and IRF9 are obtained by normalizing the mRNA expression levels of RUNX1, ITGAM, PSTPIP2, LY6E and IRF9 determined in step (a) to the mRNA expression level of ABL1 determined in step (a),

preferably wherein $b$, $m_1$, $m_2$, $m_3$, $m_4$, $m_5$ and/or $m_6$ are derived from the logistic regression model used to produce the algorithm.

8. The method of any one of claims 5-7, wherein the training set comprises at least 200 unique blood samples.

9. The method of any one of the preceding claims, wherein

(a) a bacterial infection is **characterized by** infection with *Acinetobacter, Aerococcus, Bacillus, Bacteriodes, Borrella, Clostridium, Enterobacter, Enterococcus, Escherichia, Klebstella, Neisseria, Pseudomonas, Serratia, Staphylococcus, Streptococcus* or any combination thereof;

(b) a non-infectious cause of fever is Hypersensitivity Pneuomonitis, immune response to IgG treatment, lupus, Metastatic Pancreatic Cancer, traumatic injury, NSTEMI (Non-ST-elevation myocardial infarction), polytrauma, poor GI motility, post-operative favor, pulmonary embolism, rheumatic favor, sarcoidosis, Sickle Cell Pain Crisis, Systemic inflammatory response syndrome (SIRS), Transaminitis, ureterovesical junction stone or any combination thereof, and/or

(c) a viral infection is **characterized by** infection with astrovirus, coronavirus, multisystem inflammatory syndrome in children (MIS-C), dengue, influenza, influenza A, influenza B, metapneumovirus, rhinovirus, RSV, Zika or any combination thereof.

10. The method of any one of the preceding claims, further comprising identifying a specific pathogenic viral infection, a specific pathogenic bacterial infection, or a specific non-infectious cause of fever in the subject,

wherein the specific pathogenic viral infection is SARS-CoV-2, the specific pathogenic bacterial infection is *Barrella,* and the specific non-infectious cause of fever in the subject is multisystem inflammatory syndrome in children (MIS-C);

preferably wherein identifying the specific pathogenic viral infection or specific pathogenic bacterial infection in the subject comprises:

i) comparing the viral infection score and bacterial infection score to a library of predetermined cutoff values **characterized by** specific pathogenic infections; and

ii) determining:

the specific pathogenic viral infection when the viral infection score is greater than or equal to the predetermined cutoff value **characterized by** the specific pathogenic infection, or

the specific pathogenic bacterial infection when the bacterial infection score is greater than or equal to the predetermined cutoff value **characterized by** the specific pathogenic infection.

11. The method of any one of claims 1-9, wherein identifying the specific pathogenic viral infection or specific pathogenic bacterial infection in the subject comprises assaying for a specific pathogenic viral infection signature or a specific pathogenic bacterial infection signature,

wherein the specific pathogenic viral infection is SARS-CoV-2, the specific pathogenic bacterial infection is *Barrelia,* and the specific non-infectious cause of fever in the subject is multisystem inflammatory syndrome in children (MIS-C);

preferably wherein assaying for the specific pathogenic viral infection signature or the specific pathogenic bacterial infection signature comprises:

i) determining a specific pathogenic viral infection score or a specific pathogenic bacterial infection score based on the mRNA expression levels of at least two biomarkers selected from RUNX1, ITGAM, PSTPIP2, LY6E, IRF9, and ABL1;

ii) comparing the specific pathogenic viral infection score and the specific pathogenic bacterial infection score to a library of predetermined cutoff values **characterized by** specific pathogenic infections; and

iii) determining

the specific pathogenic viral infection when the specific pathogenic viral infection score is greater than or equal to the predetermined cutoff value **characterized by** the specific pathogenic infection, or

the specific pathogenic bacterial infection when the specific pathogenic bacterial infection score is greater than or equal to the predetermined cutoff value **characterized by** the specific pathogenic infection,

preferably wherein determining the specific pathogenic viral infection score and the specific pathogenic bacterial infection score comprise inputting the mRNA expression levels of RUNX1, ITGAM, PSTPIP2, LY6E, IRF9 and optionally ABL1, into an algorithm that determines the specific pathogenic viral infection score or the specific pathogenic bacterial infection score,

preferably wherein the algorithm is a product of a logistic regression model generated using the mRNA expression levels of RUNX1, ITGAM, PSTPIP2, LY6E, IRF9 and optionally ABL1, measured in a training set of blood samples, wherein the training set of blood samples comprises:

i) a plurality of blood samples isolated from subjects identified as having a specific pathogenic bacterial infection selected from *Borrelia;*

ii) a plurality of blood samples isolated from subjects identified as having a specific pathogenic viral infection selected from MIS-C; or

iii) a plurality of blood samples isolated from subjects identified has having both a specific pathogenic bacterial infection and a specific pathogenic viral infection.

12. The method of any one of the preceding claims, further comprising determining the mRNA expression level of IFI27 in the blood sample from the subject, and determining the viral infection score and the bacterial infection score based on the mRNA expression levels of RUNX1, ITGAM, PSTPIP2, LY6E, IRF9, IFI27 and optionally ABL1.

13. The method of any one of the preceding claims, wherein:

i) the first and second predetermined cutoffs are generated using area under the receiver operating characteristics curve analysis; and/or

ii) the mRNA expression levels are measured using quantitative PCR.

14. The method of any one of the preceding claims, wherein the subject

(a) has a fever; and/or
(b) is a postoperative subject.

15. A method of triaging a patient suspected of having a bacterial infection, a viral infection, or a non-infectious cause of fever, the method comprising:

a) determining if the patient has a bacterial infection, a viral infection, or a non-infectious cause of fever according to any one of the previous claims, and

b) providing a diagnostic or treatment recommendation based on the determined infection,

preferably wherein the diagnostic or treatment recommendation:

(a) is from a clinical decision support system;

(b) is selected based on the infection status of the patient, the patient clinical history, the patient demography, algorithms, nature and severity of the infection, or a combination thereof;

(c) is selected from one or more of administration of an antibiotics, administration of an anti-viral medication, lab test, a procedure, an exam, prescription, referral, scheduling of an appointment, discharge, lifestyle suggestions, health monitoring, invasive monitoring, sedation, mechanical ventilation, intensive care admission, surgical intervention, drug of last resort, hospital admittance, or a combination thereof;

(d) comprises recommending the administration of an antiviral to an individual diagnosed with a viral infection; and/or

(e) comprises recommending the administration of an antibiotic to an individual diagnosed with a bacterial infection.

**Patentansprüche**

1.  Verfahren zur Bestimmung, ob eine Person eine bakterielle Infektion, eine virale Infektion oder eine nicht-infektiöse Ursache für Fieber hat, wobei das Verfahren Folgendes umfasst:

    a) Bestimmen der mRNA-Expressionsniveaus von RUNX1, ITGAM, PSTPIP2, LY6E, IRF9 und ABL1 in einer Blutprobe von dem Subjekt, wobei die mRNA-Expression von ABL1 als positiver Kontroll-Biomarker verwendet wird, der auf die Qualität der Blutprobe hinweist;
    b) Bestimmen eines viralen Infektionswertes und eines bakteriellen Infektionswertes auf der Grundlage der mRNA-Expressionsniveaus von RUNX1, ITGAM, PSTPIP2, LY6E und IRF9;
    c) Vergleichen des viralen Infektionswertes mit einem ersten vorbestimmten Grenzwert und des bakteriellen Infektionswertes mit einem zweiten vorbestimmten Grenzwert und Vergleichen des mRNA-Expressionsniveaus von ABL1 mit einem dritten vorbestimmten Grenzwert; und
    d) Bestimmen:

        dass das Subjekt eine Virusinfektion hat, wenn der virale Infektionswert größer ist als der erste vorbestimmte Grenzwert und das mRNA-Expressionsniveau von ABL1 größer ist als der dritte vorbestimmte Grenzwert;
        dass das Subjekt keine Virusinfektion hat, wenn der virale Infektionswert kleiner als der erste vorbestimmte Grenzwert ist und das mRNA-Expressionsniveau von ABL1 größer als der dritte vorbestimmte Grenzwert Ist;
        dass das Subjekt eine bakterielle Infektion hat, wenn der bakterielle Infektionswert größer ist als der zweite vorbestimmte Grenzwert und das mRNA-Expressionsniveau von ABL1 größer ist als der dritte vorbestimmte Grenzwert, dass das Subjekt keine bakterielle Infektion hat, wenn der bakterielle Infektionswert kleiner oder gleich dem zweiten vorbestimmten Grenzwert ist und das mRNA-Expressionsniveau von ABL1 größer als der dritte vorbestimmte Grenzwert Ist;
        und
        dass das Subjekt eine nicht-infektiöse Ursache für Fieber In dem Subjekt hat, wenn der virale Infektionswert kleiner als der erste vorbestimmte Grenzwert Ist, der bakterielle Infektionswert kleiner als der zweite vorbestimmte Grenzwert Ist und das mRNA-Expressionsniveau von ABL1 größer als der dritte vorbestimmte Grenzwert ist.

2.  Verfahren nach Anspruch 1, wobei das Subjekt als sowohl eine bakterielle Infektion als auch eine virale Infektion aufweisend identifiziert wird, wenn der virale Infektionswert größer als der erste vorbestimmte Grenzwert Ist, der bakterielle Infektionswert größer als der zweite vorbestimmte Grenzwert Ist und das mRNA-Expressionsniveau von ABL1 größer als der dritte vorbestimmte Grenzwert ist.

3.  Verfahren nach einem der vorstehenden Ansprüche, wobei das Bestimmen eines viralen Infektionswertes auf der Grundlage der mRNA-Expressionsniveaus von RUNX1, ITGAM, PSTPIP2, LY6E und IRF9 Folgendes umfasst:

    (a) Eingabe der mRNA-Expressionsniveaus von RUNX1, ITGAM, PSTPIP2, LY6E, IRF9 und optional ABL1 In einen Algorithmus, der den viralen Infektionswert bestimmt; oder
    (b) Eingabe der normalisierten mRNA-Expressionsniveaus von RUNX1, ITGAM, PSTPIP2, LY6E und IRF9 In einen Algorithmus, der den viralen Infektionswert bestimmt, vorzugsweise wobei die normalisierten mRNA-Expressionsniveaus von RUNX1, ITGAM, PSTPIP2, LY6E und IRF9 durch Normalisieren der In Schritt (a) bestimmten mRNA-Expressionsniveaus von RUNX1, ITGAM, PSTPIP2, LY6E und IRF9 auf das in Schritt (a) bestimmte mRNA-Expressionsniveau von ABL1 erhalten werden.

4.  Verfahren nach einem der vorstehenden Ansprüche, wobei das Bestimmen eines bakteriellen Infektionswertes auf der Grundlage der mRNA-Expressionsniveaus von RUNX1, ITGAM, PSTPIP2, LY6E und IRF9 Folgendes umfasst:

    (a) Eingabe der mRNA-Expressionsniveaus von RUNX1, ITGAM, PSTPIP2, LY6E, IRF9 und optional ABL1 in einen Algorithmus, der den bakteriellen Infektionswert bestimmt; oder
    (b) Eingabe der normalisierten mRNA-Expressionsniveaus von RUNX1, ITGAM, PSTPIP2, LY6E und IRF9 In einen Algorithmus, der den bakteriellen Infektionswert bestimmt, vorzugsweise wobei die normalisierten mRNA-Expressionsniveaus von RUNX1, ITGAM, PSTPIP2, LY6E und IRF9 durch Normalisieren der in Schritt (a) bestimmten mRNA-Expressionsniveaus von RUNX1, ITGAM, PSTPIP2, LY6E und IRF9 auf das in Schritt (a) bestimmte mRNA-Expressionsniveau von ABL1 erhalten werden.

5. Verfahren nach Anspruch 3 oder Anspruch 4, wobei der Algorithmus ein Produkt eines logistischen Regressionsmodells ist, das unter Verwendung der mRNA-Expressionsniveaus von RUNX1, ITGAM, PSTPIP2, LY6E, IRF9 und optional ABL1, gemessen in einem Trainingssatz von Blutproben, erzeugt wird, wobei der Trainingssatz von Blutproben Folgendes umfasst:

> i) eine Vielzahl von Blutproben, die von Subjekten isoliert wurden, bei denen eine bakterielle Infektion festgestellt wurde;
> ii) eine Vielzahl von Blutproben, die von Subjekten isoliert wurden, bei denen eine virale Infektion festgestellt wurde;
> iii) eine Vielzahl von Blutproben, die von Subjekten isoliert wurden, bei denen sowohl eine bakterielle als auch eine virale Infektion festgestellt wurde;
> iv) eine Vielzahl von Blutproben, die von Subjekten isoliert wurden, bei denen eine nicht-infektiöse Ursache für Fieber festgestellt wurde; und
> v) eine Vielzahl von Blutproben, die von gesunden Subjekten isoliert wurden, die keine bakterielle Infektion, virale Infektion oder nicht-infektiöse Ursache für Fieber haben.
> vorzugsweise wobei die in dem Trainingssatz von Blutproben gemessenen mRNA-Expressionsniveaus von RUNX1, ITGAM, PSTPIP2, LY6E und IRF9 normalisierte mRNA-Expressionsniveaus von RUNX1, ITGAM, PSTPIP2, LY6E und IRF9 sind, die durch Normalisierung der mRNA-Expressionsniveaus von RUNX1, ITGAM, PSTPIP2, LY6E und IRF9, gemessen in dem Trainingssatz von Blutproben, auf das mRNA-Expressionsniveau von ABL1, gemessen in dem Trainingssatz von Blutproben, erhalten werden,
> vorzugsweise wobei das logistische Regressionsmodell ein Produkt der univariaten Analyse und/oder der multivariaten Analyse der mRNA-Expressionsniveaus von RUNX1, ITGAM, PSTPIP2, LY6E, IRF9 und optional ABL1 in dem Trainingssatz von Blutproben ist.

6. Verfahren nach einem der Ansprüche 3-5, wobei der Algorithmus, der den viralen Infektionswert bestimmt, folgender Ist:

$$p= \frac{1}{1+e^{-(b+m_1*RUNX1+m_2*ITGAM+m_3*PSTPIP2+m_4*LY6E+m_5*IRF9)}} \text{ oder}$$

$$p= \frac{1}{1+e^{-(b+m_1*RUNX1+m_2*ITGAM+m_3*PSTPIP2+m_4*LY6E+m_5*IRF9+m_6*ABL1)}};$$

wobei *RUNX1* das mRNA-Expressionsniveau oder das normalisierte mRNA-Expressionsniveau von RUNX1 in der Blutprobe von dem Subjekt ist,
*ITGAM* ist das mRNA-Expressionsniveau oder das normalisierte mRNA-Expressionsniveau von ITGAM in der Blutprobe von dem Subjekt,
*PSTPIP2* ist das mRNA-Expressionsniveau oder das normalisierte mRNA-Expressionsniveau von PSTPIP2 in der Blutprobe von dem Subjekt,
*LY6E* ist das mRNA-Expressionsniveau oder das normalisierte mRNA-Expressionsniveau von LY6E in der Blutprobe von dem Subjekt,
*IRF9* ist das mRNA-Expressionsniveau oder das normalisierte mRNA-Expressionsniveau von IRF9 in der Blutprobe von dem Subjekt,
*ABL1* ist das mRNA-Expressionsniveau von ABL1 in der Blutprobe von dem Subjekt,
und
*b ist* ein Schnittpunkt,
$m_1$ ist ein Skalierungsfaktor für das mRNA-Expressionsniveau oder das normalisierte mRNA-Expressionsniveau von RUNX,
$m_2$ ist ein Skalierungsfaktor für das mRNA-Expressionsniveau oder das normalisierte mRNA-Expressionsniveau von ITGAM,
$m_3$ ist ein Skalierungsfaktor für das mRNA-Expressionsniveau oder das normalisierte mRNA-Expressionsniveau

von PSTPIP2,

$m_4$ ist ein Skalierungsfaktor für das mRNA-Expressionsniveau oder das normalisierte mRNA-Expressionsniveau von LY6E,

$m_5$ ist ein Skalierungsfaktor für das mRNA-Expressionsniveau oder das normalisierte mRNA-Expressionsniveau von IRF9,

$m_6$ ist ein Skalierungsfaktor für das mRNA-Expressionsniveau von ABL1,

vorzugsweise wobei die normalisierten mRNA-Expressionsniveaus von RUNX1, ITGAM, PSTPIP2, LY6E und IRF9 durch Normalisieren der in Schritt (a) bestimmten mRNA-Expressionsniveaus von RUNX1, ITGAM, PSTPIP2, LY6E und IRF9 auf das in Schritt (a) bestimmte mRNA-Expressionsniveau von ABL1 erhalten werden, vorzugsweise wobei $b$, $m_1$, $m_2$, $m_3$, $m_4$, $m_5$ und/oder $m_6$ aus dem logistischen Regressionsmodell abgeleitet werden, das zur Erstellung des Algorithmus verwendet wird.

7. Verfahren nach einem der Ansprüche 3-6, wobei der Algorithmus, der den bakteriellen Infektionswert bestimmt, folgender ist:

$$p = \frac{1}{1+e^{-(b+m_1*RUNX1\ m_2*ITGAM+m_3*PSTPIP2+m_4*LY6E+m_5*IRF9)}} \text{ oder}$$

$$p = \frac{1}{1+e^{-(b+m_1*RUNX1+m_2*ITGAM+m_3*PSTPIP2+m_4*LY6E+m_5*IRF9+m_6*ABL1)}};$$

wobei *RUNX1* das mRNA-Expressionsniveau oder das normalisierte mRNA-Expressionsniveau von RUNX1 in der Blutprobe von dem Subjekt ist,

*ITGAM* ist das mRNA-Expressionsniveau oder das normalisierte mRNA-Expressionsniveau von ITGAM in der Blutprobe von dem Subjekt,

*PSTPIP2* ist das mRNA-Expressionsniveau oder das normalisierte mRNA-Expressionsniveau von PSTPIP2 in der Blutprobe von dem Subjekt,

*LY6E* ist das mRNA-Expressionsniveau oder das normalisierte mRNA-Expressionsniveau von LY6E in der Blutprobe von dem Subjekt,

*IRF9* ist das mRNA-Expressionsniveau oder das normalisierte mRNA-Expressionsniveau von IRF9 in der Blutprobe von dem Subjekt,

*ABL1* ist das mRNA-Expressionsniveau von ABL1 in der Blutprobe von dem Subjekt, und

$b$ ist ein Schnittpunkt,

$m_1$ ist ein Skalierungsfaktor für das mRNA-Expressionsniveau oder das normalisierte mRNA-Expressionsniveau von RUNX,

$m_2$ ist ein Skalierungsfaktor für das mRNA-Expressionsniveau oder das normalisierte mRNA-Expressionsniveau von ITGAM,

$m_3$ ist ein Skalierungsfaktor für das mRNA-Expressionsniveau oder das normalisierte mRNA-Expressionsniveau von PSTPIP2,

$m_4$ ist ein Skalierungsfaktor für das mRNA-Expressionsniveau oder das normalisierte mRNA-Expressionsniveau von LY6E,

$m_5$ ist ein Skalierungsfaktor für das mRNA-Expressionsniveau oder das normalisierte mRNA-Expressionsniveau von IRF9,

$m_6$ ist ein Skalierungsfaktor für das mRNA-Expressionsniveau von ABL1,

vorzugsweise wobei die normalisierten mRNA-Expressionsniveaus von RUNX1, ITGAM, PSTPIP2, LY6E und IRF9 durch Normalisieren der in Schritt (a) bestimmten mRNA-Expressionsniveaus von RUNX1, ITGAM, PSTPIP2, LY6E und IRF9 auf das in Schritt (a) bestimmte mRNA-Expressionsniveau von ABL1 erhalten werden,

vorzugsweise wobei $b$, $m_1$, $m_2$, $m_3$, $m_4$, $m_5$ und/oder $m_6$ aus dem logistischen Regressionsmodell abgeleitet werden, das zur Erstellung des Algorithmus verwendet wird.

8. Verfahren nach einem der Ansprüche 5-7, wobei der Trainingssatz mindestens 200 einzigartige Blutproben umfasst.

9. Verfahren nach einem der vorstehenden Ansprüche, wobei

(a) eine bakterielle Infektion durch eine Infektion mit *Acinetobacter,* Aerococcus, *Bacillus, Bacteriodes, Borrelia, Clostridium, Enterobacter, Enterococcus, Escherichia, Klebsiella, Neisseria, Pseudomonas, Serratia, Staphylococcus, Streptococcus* oder einer beliebigen Kombination davon gekennzeichnet ist;

(b) eine nicht-infektiöse Ursache für Fieber ist Hypersensitivitäts-Pneumomonitis, eine Immunreaktion auf IgG-Behandlung, Lupus, metastasierter Bauchspeicheldrüsenkrebs, eine traumatische Verletzung, NSTEMI (Non-STelevation myocardial infarction), Polytrauma,

schlechte Magen-Darm-Motilität, postoperatives Fieber, Lungenembolie, rheumatisches Fieber, Sarkoidose, Sichelzellenschmerzkrise, systemisches Entzündungssyndrom (SIRS), Transaminitis, Steine an der ureterovesikalen Verbindung oder eine beliebige Kombination davon; und/oder

(c) eine Virusinfektion ist **gekennzeichnet durch** eine Infektion mit Astrovirus, Coronavirus, Multisystem-Inflammationssyndrom bei Kindern (MIS-C), Dengue, Influenza, Influenza A, Influenza B, Metapneumovirus, Rhinovirus, RSV, Zika oder eine beliebige Kombination davon.

10. Verfahren nach einem der vorstehenden Ansprüche, weiter umfassend das Identifizieren einer spezifischen pathogenen viralen Infektion, einer spezifischen pathogenen bakteriellen Infektion oder einer spezifischen nicht-infektiösen Ursache für Fieber in dem Subjekt,

wobei die spezifische pathogene virale Infektion SARS-CoV-2 Ist, die spezifische pathogene bakterielle Infektion Borrelia ist und die spezifische nicht-infektiöse Ursache des Fiebers in dem Subjekt das Multisystem-Entzündungssyndrom bei Kindern (MIS-C) Ist;

vorzugsweise wobei das Identifizieren der spezifischen pathogenen viralen Infektion oder der spezifischen pathogenen bakteriellen Infektion in dem Subjekt Folgendes umfasst:

i) Vergleichen des viralen Infektionswertes und des bakteriellen Infektionswertes mit einer Bibliothek vorbestimmter Grenzwerte, die durch spezifische pathogene Infektionen gekennzeichnet sind; und

ii) Bestimmen:

der spezifischen pathogenen viralen Infektion, wenn der virale Infektionswert größer oder gleich dem vorbestimmten Grenzwert ist, der durch die spezifische pathogene Infektion gekennzeichnet ist, oder der spezifischen pathogenen bakteriellen Infektion, wenn der bakterielle Infektionswert größer oder gleich dem vorbestimmten Grenzwert ist, der durch die spezifische pathogene Infektion gekennzeichnet ist.

11. Verfahren nach einem der Ansprüche 1-9, wobei das Identifizieren der spezifischen pathogenen viralen Infektion oder der spezifischen pathogenen bakteriellen Infektion in dem Subjekt das Testen auf eine spezifische pathogene virale Infektionssignatur oder eine spezifische pathogene bakterielle Infektionssignatur umfasst,

wobei die spezifische pathogene virale Infektion SARS-CoV-2 Ist, die spezifische pathogene bakterielle Infektion Borrelia ist und die spezifische nicht-infektiöse Ursache des Fiebers in dem Subjekt das Multisystem-Entzündungssyndrom bei Kindern (MIS-C) Ist;

vorzugsweise wobei das Testen auf die spezifische pathogene virale Infektionssignatur oder die spezifische pathogene bakterielle Infektionssignatur Folgendes umfasst:

i) Bestimmen eines spezifischen pathogenen viralen Infektionswerts oder eines spezifischen pathogenen bakteriellen Infektionswerts auf der Grundlage der mRNA-Expressionsniveaus von mindestens zwei Biomarkern, ausgewählt aus RUNX1, ITGAM, PSTPIP2, LY6E, IRF9 und ABL1;

ii) Vergleichen des spezifischen pathogenen viralen Infektionswertes und des spezifischen pathogenen bakteriellen Infektionswertes mit einer Bibliothek vorbestimmter Grenzwerte, die durch spezifische pathogene Infektionen gekennzeichnet sind; und

iii) Bestimmen

der spezifischen pathogenen viralen Infektion, wenn der spezifische pathogene virale Infektionswert größer oder gleich dem vorbestimmten Grenzwert ist, der durch die spezifische pathogene Infektion gekennzeichnet ist, oder der spezifischen pathogenen bakteriellen Infektion, wenn der spezifische pathogene bakterielle Infektionswert größer oder gleich dem vorbestimmten Grenzwert ist, der durch die spezifische pathogene Infektion gekennzeichnet ist, vorzugsweise wobei das Bestimmen des spezifischen pathogenen viralen Infektionswerts und des spezifischen pathogenen bakteriellen Infektionswerts das Eingeben der mRNA-Expressionsniveaus von RUNX1, ITGAM, PSTPIP2, LY6E, IRF9 und optional ABL1 in einen Algorithmus umfasst, der den spezifischen pathogenen viralen Infektionswert oder den spezifischen pathogenen bakteriellen Infektionswert bestimmt,

vorzugsweise wobei der Algorithmus ein Produkt eines logistischen Regressionsmodells ist, das unter Verwendung der mRNA-Expressionsniveaus von RUNX1, ITGAM, PSTPIP2, LY6E, IRF9 und optional ABL1, gemessen in einem Trainingssatz von Blutproben, erzeugt wird, wobei der Trainingssatz von Blutproben Folgendes umfasst:

i) eine Vielzahl von Blutproben, die von Subjekten isoliert wurden, bei denen eine spezifische pathogene bakterielle Infektion, ausgewählt aus *Borrelia,* festgestellt wurde;
ii) eine Vielzahl von Blutproben, die von Subjekten isoliert wurden, bei denen eine spezifische pathogene virale Infektion, ausgewählt aus MIS-C, festgestellt wurde; oder
iii) eine Vielzahl von Blutproben, die von Subjekten isoliert wurden, bei denen sowohl eine spezifische pathogene bakterielle Infektion als auch eine spezifische pathogene virale Infektion festgestellt wurde.

12. Verfahren nach einem der vorstehenden Ansprüche, weiter umfassend das Bestimmen des mRNA-Expressionsniveaus von IFI27 in der Blutprobe von dem Subjekt und das Bestimmen des viralen Infektionswerts und des bakteriellen Infektionswerts auf der Grundlage der mRNA-Expressionsniveaus von RUNX1, ITGAM, PSTPIP2, LY6E, IRF9, IFI27 und optional ABL1.

13. Verfahren nach einem der vorstehenden Ansprüche, wobei:

i) die ersten und zweiten vorbestimmten Grenzwerte unter Verwendung der Analyse der Fläche unter der Receiver Operating Characteristic-Kurve erzeugt werden;
und/oder
ii) die mRNA-Expressionsniveaus mittels quantitativer PCR gemessen werden.

14. Verfahren nach einem der vorstehenden Ansprüche, wobei das Subjekt:

(a) Fieber hat; und/oder
(b) ein postoperatives Subjekt ist.

15. Verfahren zur Einstufung eines Patienten, bei dem der Verdacht auf eine bakterielle Infektion, eine virale Infektion oder eine nicht-infektiöse Ursache für Fieber besteht, wobei das Verfahren Folgendes umfasst:

(a) Bestimmen, ob der Patient eine bakterielle Infektion, eine virale Infektion oder eine nicht-infektiöse Ursache für Fieber nach einem der vorherigen Ansprüche hat, und
(b) Bereitstellen einer Diagnose- oder Behandlungsempfehlung auf der Grundlage der festgestellten Infektion,

vorzugsweise wobei die Diagnose- oder Behandlungsempfehlung:

(a) aus einem Unterstützungssystem für klinische Entscheidungen stammt;
(b) auf der Grundlage des Infektionsstatus des Patienten, der klinischen Vorgeschichte des Patienten, der Demografie des Patienten, der Algorithmen, der Art und des Schweregrads der Infektion oder einer Kombination davon ausgewählt wird;
(c) ausgewählt wird aus einem oder mehreren von: Verabreichung eines Antibiotikums, Verabreichung eines antiviralen Medikaments, Labortest, einem Verfahren, einer Untersuchung, Verschreibung, Überweisung, Terminvereinbarung, Entlassung, Vorschlägen zur Lebensführung, Gesundheitsüberwachung, invasiver Überwachung, Sedierung, mechanischer Beatmung, Einweisung in die Intensivstation, chirurgischem Eingriff, Medikament als letzter Ausweg, Krankenhauseinweisung oder einer Kombination davon;
(d) ein Empfehlen der Verabreichung eines antiviralen Mittels an ein Individuum, bei dem eine virale Infektion diagnostiziert wurde, umfasst; und/oder
(e) ein Empfehlen der Verabreichung eines Antibiotikums an ein Individuum, bei dem eine bakterielle Infektion diagnostiziert wurde, umfasst.

**Revendications**

1. Procédé de détermination du fait qu'un sujet présente ou non une Infection bactérienne, une infection virale, ou une cause non infectieuse de fièvre, le procédé comprenant :

   a) la détermination des niveaux d'expression d'ARNm de RUNX1, ITGAM, PSTPIP2, LY6E, IRF9 et ABL1 dans un échantillon de sang provenant du sujet, dans lequel l'expression d'ARNm d'ABL1 est utilisée comme un biomarqueur de contrôle positif qui est indicatif de la qualité de l'échantillon de sang ;
   b) la détermination d'un score d'infection virale et d'un score d'infection bactérienne sur la base des niveaux d'expression d'ARNm de RUNX1, ITGAM, PSTPIP2, LY6E et IRF9 ;
   c) la comparaison du score d'infection virale à une première valeur de coupure prédéterminée et du score d'infection bactérienne à une deuxième valeur de coupure prédéterminée et la comparaison du niveau d'expression d'ARNm d'ABL1 à une troisième valeur de coupure prédéterminée ; et
   d) la détermination :

   du fait que le sujet présente une Infection virale lorsque le score d'infection virale est supérieur à la première valeur de coupure prédéterminée et le niveau d'expression d'ARNm d'ABL1 est supérieur à la troisième valeur de coupure prédéterminée ;
   du fait que le sujet ne présente pas d'infection virale lorsque le score d'infection virale est inférieur à la première valeur de coupure prédéterminée et le niveau d'expression d'ARNm d'ABL1 est supérieur à la troisième valeur de coupure prédéterminée ;
   du fait que le sujet présente une infection bactérienne lorsque le score d'infection bactérienne est supérieur à la deuxième valeur de coupure prédéterminée et le niveau d'expression d'ARNm d'ABL1 est à
   la troisième valeur de coupure prédéterminée ; du fait que le sujet ne présente pas d'infection bactérienne lorsque le score d'infection bactérienne est inférieur ou égal à la deuxième valeur de coupure prédéterminée et le niveau d'expression d'ARNm d'ABL1 est supérieur à la troisième valeur de coupure prédéterminée ; et
   du fait que le sujet présente une cause non infectieuse de fièvre chez le sujet lorsque le score d'infection virale est inférieur à la première valeur de coupure prédéterminée, le score d'infection bactérienne est inférieur à la deuxième valeur de coupure prédéterminée et le niveau d'expression d'ARNm d'ABL1 est supérieur à la troisième valeur de coupure prédéterminée.

2. Procédé selon la revendication 1, dans lequel le sujet est identifié comme présentant à la fois une infection bactérienne et une Infection virale lorsque le score d'infection virale est supérieur à la première valeur de coupure prédéterminée, le score d'infection bactérienne est supérieur à la deuxième valeur de coupure prédéterminée et le niveau d'expression d'ARNm d'ABL1 est supérieur à la troisième valeur de coupure prédéterminée.

3. Procédé selon l'une quelconque des revendications précédentes, dans lequel la détermination d'un score d'Infection virale sur la base des niveaux d'expression d'ARNm de RUNX1, ITGAM, PSTPIP2, LY6E et IRF9 comprend :

   (a) l'entrée des niveaux d'expression d'ARNm de RUNX1, ITGAM, PSTPIP2, LY6E, IRF9 et facultativement ABL1, dans un algorithme qui détermine le score d'infection virale ; ou
   (b) l'entrée des niveaux d'expression d'ARNm normalisés de RUNX1, ITGAM, PSTPIP2, LY6E et IRF9 dans un algorithme qui détermine le score d'infection virale, de préférence dans lequel les niveaux d'expression d'ARNm normalisés de RUNX1, ITGAM, PSTPIP2, LY6E et IRF9 sont obtenus en normalisant les niveaux d'expression d'ARNm de RUNX1, ITGAM, PSTPIP2, LY6E et IRF9 déterminés dans l'étape (a) au niveau d'expression d'ARNm d'ABL1 déterminé dans l'étape (a).

4. Procédé selon l'une quelconque des revendications précédentes, dans lequel la détermination d'un score d'infection bactérienne sur la base des niveaux d'expression d'ARNm de RUNX1, ITGAM, PSTPIP2, LY6E et IRF9 comprend :

   (a) l'entrée des niveaux d'expression d'ARNm de RUNX1, ITGAM, PSTPIP2, LY6E, IRF9 et facultativement ABL1, dans un algorithme qui détermine le score d'infection bactérienne ; ou
   (b) l'entrée des niveaux d'expression d'ARNm normalisés de RUNX1, ITGAM, PSTPIP2, LY6E et IRF9 dans un algorithme qui détermine le score d'infection bactérienne, de préférence dans lequel les niveaux d'expression d'ARNm normalisés de RUNX1, ITGAM, PSTPIP2, LY6E et IRF9 sont obtenus en normalisant les niveaux d'expression d'ARNm de RUNX1, ITGAM, PSTPIP2, LY6E et IRF9 déterminés dans l'étape (a) au niveau d'expression d'ARNm d'ABL1 déterminé dans l'étape (a).

**5.** Procédé selon la revendication 3 ou la revendication 4, dans lequel l'algorithme est un produit d'un modèle de régression logistique généré en utilisant les niveaux d'expression d'ARNm de RUNX1, ITGAM, PSTPIP2, LY6E, IRF9 et facultativement ABL1, mesurés dans un ensemble d'entraînement d'échantillons de sang, dans lequel l'ensemble d'entraînement d'échantillons de sang comprend :

    i) une pluralité d'échantillons de sang isolés de sujets identifiés comme présentant une Infection bactérienne ;
    ii) une pluralité d'échantillons de sang isolés de sujets identifiés comme présentant une Infection virale ;
    iii) une pluralité d'échantillons de sang isolés de sujets identifiés comme présentant à la fois une infection bactérienne et une Infection virale ;
    iv) une pluralité d'échantillons de sang isolés de sujets identifiés comme présentant une cause non Infectieuse de fièvre ; et
    v) une pluralité d'échantillons de sang isolés de sujets sains ne présentant pas d'infection bactérienne, d'infection virale ou de cause non infectieuse de fièvre.
    de préférence dans lequel les niveaux d'expression d'ARNm de RUNX1, ITGAM, PSTPIP2, LY6E et IRF9 mesurés dans l'ensemble d'entraînement d'échantillons de sang sont des niveaux d'expression d'ARNm normalisés de RUNX1, ITGAM, PSTPIP2, LY6E et IRF9 obtenus en normalisant les niveaux d'expression d'ARNm de RUNX1, ITGAM, PSTPIP2, LY6E et IRF9 mesurés dans l'ensemble d'entraînement d'échantillons de sang au niveau d'expression d'ARNm d'ABL1 mesuré dans l'ensemble d'entraînement d'échantillons de sang, de préférence dans lequel le modèle de régression logistique est un produit d'une analyse univariée et/ou d'une analyse multivarlée des niveaux d'expression d'ARNm de RUNX1, ITGAM, PSTPIP2, LY6E, IRF9 et facultativement, ABL1, dans l'ensemble d'entraînement d'échantillons de sang.

**6.** Procédé selon l'une quelconque des revendications 3 à 5, dans lequel l'algorithme qui détermine le score d'infection virale est :

$$p == \frac{1}{1 + e^{-(b+m_1*RUNX1+m_2*ITGAM+m_3*PSTPIP2+m_4*LY6E+m_5*IRF9)}} \; ou$$

$$p = \frac{1}{1 + e^{-(b+m_1*RUNX1+m_2*ITGAM+m_3*PSTPIP2+m_4*LY6E+m_5*IRF9+m_6*ABL1)}} \; ;$$

dans lequel *RUNX1* est le niveau d'expression d'ARNm ou le niveau d'expression d'ARNm normalisé de RUNX1 dans l'échantillon de sang provenant du sujet,
*ITGAM* est le niveau d'expression d'ARNm ou le niveau d'expression d'ARNm normalisé d'ITGAM dans l'échantillon de sang provenant du sujet,
*PSTPIP2* est le niveau d'expression d'ARNm ou le niveau d'expression d'ARNm normalisé de PSTPIP2 dans l'échantillon de sang provenant du sujet, *LY6E* est le niveau d'expression d'ARNm ou le niveau d'expression d'ARNm normalisé de LY6E dans l'échantillon de sang provenant du sujet,
*IRF9* est le niveau d'expression d'ARNm ou le niveau d'expression d'ARNm normalisé d'IRF9 dans l'échantillon de sang provenant du sujet,
*ABL1* est le niveau d'expression d'ARNm d'ABL1 dans l'échantillon de sang provenant du sujet,
et
b est une Interception,
$m_1$ est un facteur de mise à l'échelle pour le niveau d'expression d'ARNm ou le niveau d'expression d'ARNm normalisé de RUNX,
$m_2$ est un facteur de mise à l'échelle pour le niveau d'expression d'ARNm ou le niveau d'expression d'ARNm normalisé d'ITGAM,
$m_3$ est un facteur de mise à l'échelle pour le niveau d'expression d'ARNm ou le niveau d'expression d'ARNm normalisé de PSTPIP2,
$m_4$ est un facteur de mise à l'échelle pour le niveau d'expression d'ARNm ou le niveau d'expression d'ARNm normalisé de LY6E,
$m_5$ est un facteur de mise à l'échelle pour le niveau d'expression d'ARNm ou le niveau d'expression d'ARNm normalisé d'IRF9,
$m_6$ est un facteur de mise à l'échelle pour le niveau d'expression d'ARNm d'ABL1,
de préférence dans lequel les niveaux d'expression d'ARNm normalisés de RUNX1, ITGAM, PSTPIP2, LY6E et IRF9 sont obtenus en normalisant les niveaux d'expression d'ARNm de RUNX1, ITGAM, PSTPIP2, LY6E et IRF9 déterminés dans l'étape (a) au niveau d'expression d'ARNm d'ABL1 déterminé dans l'étape (a),

de préférence dans lequel *b, $m_1$, $m_2$, $m_3$, $m_4$, $m_5$* et/ou *$m_6$* sont dérivés du modèle de régression logistique utilisé pour produire l'algorithme.

**7.** Procédé selon l'une quelconque des revendications 3 à 6, dans lequel l'algorithme qui détermine le score d'infection bactérienne est :

$$p == \frac{1}{1 + e^{-(b+m_1*RUNX1\, m_2*ITGAM+m_3*PSTPIP2+m_4*LY6E+m_5*IRF9)}} \; ou$$

$$p = \frac{1}{1 + e^{-(b+m_1*RUNX1+m_2*ITGAM+m_3*PSTPIP2+m_4*LY6E+m_5*IRF9+m_6*ABL1)}} \; ;$$

dans lequel *RUNX1* est le niveau d'expression d'ARNm ou le niveau d'expression d'ARNm normalisé de RUNX1 dans l'échantillon de sang provenant du sujet,

*ITGAM* est le niveau d'expression d'ARNm ou le niveau d'expression d'ARNm normalisé d'ITGAM dans l'échantillon de sang provenant du sujet,

*PSTPIP2* est le niveau d'expression d'ARNm ou le niveau d'expression d'ARNm normalisé de PSTPIP2 dans l'échantillon de sang provenant du sujet, *LY6E* est le niveau d'expression d'ARNm ou le niveau d'expression d'ARNm normalisé de LY6E dans l'échantillon de sang provenant du sujet,

*IRF9* est le niveau d'expression d'ARNm ou le niveau d'expression d'ARNm normalisé d'IRF9 dans l'échantillon de sang provenant du sujet,

*ABL1* est le niveau d'expression d'ARNm d'ABL1 dans l'échantillon de sang provenant du sujet, et

*b* est une Interception,

$m_1$ est un facteur de mise à l'échelle pour le niveau d'expression d'ARNm ou le niveau d'expression d'ARNm normalisé de RUNX,

$m_2$ est un facteur de mise à l'échelle pour le niveau d'expression d'ARNm ou le niveau d'expression d'ARNm normalisé d'ITGAM,

$m_3$ est un facteur de mise à l'échelle pour le niveau d'expression d'ARNm ou le niveau d'expression d'ARNm normalisé de PSTPIP2,

$m_4$ est un facteur de mise à l'échelle pour le niveau d'expression d'ARNm ou le niveau d'expression d'ARNm normalisé de LY6E,

$m_5$ est un facteur de mise à l'échelle pour le niveau d'expression d'ARNm ou le niveau d'expression d'ARNm normalisé d'IRF9,

$m_6$ est un facteur de mise à l'échelle pour le niveau d'expression d'ARNm d'ABL1,

de préférence dans lequel les niveaux d'expression d'ARNm normalisés de RUNX1, ITGAM, PSTPIP2, LY6E et IRF9 sont obtenus en normalisant les niveaux d'expression d'ARNm de RUNX1, ITGAM, PSTPIP2, LY6E et IRF9 déterminés dans l'étape (a) au niveau d'expression d'ARNm d'ABL1 déterminé dans l'étape (a),

de préférence dans lequel *b, $m_1$, $m_2$, $m_3$, $m_4$, $m_5$* et/ou *$m_6$* sont dérivés du modèle de régression logistique utilisé pour produire l'algorithme.

**8.** Procédé selon l'une quelconque des revendications 5 à 7, dans lequel l'ensemble d'entraînement comprend au moins 200 échantillons de sang uniques.

**9.** Procédé selon l'une quelconque des revendications précédentes, dans lequel

(a) une infection bactérienne est **caractérisée par** une infection avec *Acinetobacter, Aerococcus, Bacillus,* Bacteriodes, *Borrelia, Clostridium, Enterobacter, Enterococcus, Escherichia, Klebsiella, Neisseria,* Pseudomonas, *Serratia, Staphylococcus, Streptococcus* ou une combinaison quelconque de celles-ci ;

(b) une cause non infectieuse de fièvre est pneumopathie d'hypersensibilité, réponse Immunitaire à un traitement IgG, lupus, cancer du pancréas métastatique, blessure traumatique, NSTEMI (Infarctus du myocarde sans élévation du segment ST), polytraumatisme, mauvaise motilité gastro-intestinale, fièvre post-opératoire, embolie pulmonaire, fièvre rhumatismale, sarcoTdose, crise de douleur associée à la drépanocytose, syndrome de réponse inflammatoire systémique (SIRS), transaminite, calcul à la jonction urétéro-vésicale ou une combinaison quelconque de ceux-ci ; et/ou

(c) une infection virale est **caractérisée par** une infection avec un astrovirus, un coronavirus, un syndrome Inflammatoire multisystémique chez les enfants (MIS-C), une dengue, une grippe, une grippe A, une grippe B, un métapneumovirus, un rhinovirus, un RSV, le Zika ou une combinaison quelconque de ceux-ci.

10. Procédé selon l'une quelconque des revendications précédentes, comprenant en outre l'identification d'une Infection virale pathogène spécifique, d'une Infection bactérienne pathogène spécifique, ou d'une cause non infectieuse spécifique de fièvre chez le sujet,

dans lequel l'infection virale pathogène spécifique est SARS-CoV-2, l'infection bactérienne pathogène spécifique est *Borrelia,* et la cause non Infectieuse spécifique de fièvre chez le sujet est syndrome Inflammatoire multisystémique chez les enfants (MIS-C) ;
de préférence dans lequel l'identification de l'infection virale pathogène spécifique ou de l'infection bactérienne pathogène spécifique chez le sujet comprend :

i) la comparaison du score d'infection virale et du score d'infection bactérienne à une bibliothèque de valeurs de coupure prédéterminées **caractérisées par** des Infections pathogènes spécifiques ; et
ii) la détermination :

de l'infection virale pathogène spécifique lorsque le score d'Infection virale est supérieur ou égal à la valeur de coupure prédéterminée **caractérisée par** l'infection pathogène spécifique, ou
de l'infection bactérienne pathogène spécifique lorsque le score d'infection bactérienne est supérieur ou égal à la valeur de coupure prédéterminée **caractérisée par** l'infection pathogène spécifique.

11. Procédé selon l'une quelconque des revendications 1 à 9, dans lequel l'identification de l'infection virale pathogène spécifique ou de l'infection bactérienne pathogène spécifique chez le sujet comprend l'analyse d'une signature d'infection virale pathogène spécifique ou d'une signature d'Infection bactérienne pathogène spécifique,

dans lequel l'infection virale pathogène spécifique est SARS-CoV-2, l'infection bactérienne pathogène spécifique est *Borrelia,* et la cause non Infectieuse spécifique de fièvre chez le sujet est syndrome Inflammatoire multisystémique chez les enfants (MIS-C) ;
de préférence dans lequel l'analyse de la signature d'infection virale pathogène spécifique ou de la signature d'infection bactérienne pathogène spécifique comprend :

i) la détermination d'un score d'infection virale pathogène spécifique ou d'un score d'infection bactérienne pathogène spécifique sur la base des niveaux d'expression d'ARNm d'au moins deux biomarqueurs sélectionnés dans RUNX1, ITGAM, PSTPIP2, LY6E, IRF9, et ABL1 ;
ii) la comparaison du score d'infection virale pathogène spécifique et du score d'infection bactérienne pathogène spécifique à une bibliothèque de valeurs de coupure prédéterminées **caractérisées par** des infections pathogènes spécifiques ; et
iii) la détermination

de l'infection virale pathogène spécifique lorsque le score d'infection virale pathogène spécifique est supérieur ou égal à la valeur de coupure prédéterminée **caractérisée par** l'infection pathogène spécifique, ou
de l'infection bactérienne pathogène spécifique lorsque le score d'infection bactérienne pathogène spécifique est supérieur ou égal à la valeur de coupure prédéterminée **caractérisée par** l'infection pathogène spécifique,
de préférence dans lequel la détermination du score d'Infection virale pathogène spécifique et du score d'infection bactérienne pathogène spécifique comprend l'entrée des niveaux d'expression d'ARNm de RUNX1, ITGAM, PSTPIP2, LY6E, IRF9 et facultativement ABL1, dans un algorithme qui détermine le score d'infection virale pathogène spécifique ou le score d'infection bactérienne pathogène spécifique, de préférence dans lequel l'algorithme est un produit d'un modèle de régression logistique généré en utilisant les niveaux d'expression d'ARNm de RUNX1, ITGAM, PSTPIP2, LY6E, IRF9 et facultativement ABL1, mesurés dans un ensemble d'entraînement d'échantillons de sang, dans lequel l'ensemble d'entraînement d'échantillons de sang comprend :

i) une pluralité d'échantillons de sang isolés de sujets identifiés comme présentant une infection bactérienne pathogène spécifique sélectionnée dans Borrelia *;*
ii) une pluralité d'échantillons de sang isolés de sujets identifiés comme présentant une infection virale pathogène spécifique sélectionnée dans MIS-C ; ou

iii) une pluralité d'échantillons de sang isolés de sujets identifiés comme présentant à la fois une infection bactérienne pathogène spécifique et une Infection virale pathogène spécifique.

12. Procédé selon l'une quelconque des revendications précédentes, comprenant en outre la détermination du niveau d'expression d'ARNm d'IFI27 dans l'échantillon de sang provenant du sujet, et la détermination du score d'Infection virale et du score d'infection bactérienne sur la base des niveaux d'expression d'ARNm de RUNX1, ITGAM, PSTPIP2, LY6E, IRF9, IFI27 et facultativement ABL1.

13. Procédé selon l'une quelconque des revendications précédentes, dans lequel :

i) les première et deuxième valeurs de coupure prédéterminées sont générées en utilisant une analyse de l'aire sous la courbe de caractéristiques de fonctionnement du récepteur ;
et/ou
ii) les niveaux d'expression d'ARNm sont mesurés en utilisant une PCR quantitative.

14. Procédé selon l'une quelconque des revendications précédentes, dans lequel le sujet :

(a) présente une fièvre ; et/ou
(b) est un sujet post-opératoire.

15. Procédé de triage d'un patient suspecté de présenter une infection bactérienne, une Infection virale, ou une cause non infectieuse de fièvre, le procédé comprenant :

(a) la détermination du fait que le patient présente ou non une infection bactérienne, une Infection virale, ou une cause non Infectieuse de fièvre selon l'une quelconque des revendications précédentes, et
(b) la fourniture d'une recommandation de diagnostic ou de traitement sur la base de l'Infection déterminée,

de préférence dans lequel la recommandation de diagnostic ou de traitement :

(a) provient d'un système d'aide à la décision clinique ;
(b) est sélectionnée sur la base du statut d'infection du patient, de l'historique clinique du patient, de la démographie du patient, d'algorithmes, de la nature et de la gravité de l'infection, ou d'une combinaison de ceux-ci ;
(c) est sélectionnée dans un ou une ou plusieurs d'administration d'un antibiotique, d'administration d'un médicament antiviral, de test en laboratoire, d'intervention, d'examen, d'ordonnance, de référence, de programmation d'un rendezvous, de sortie, de suggestions de style de vie, de surveillance de santé, de surveillance Invasive, de sédation, de ventilation mécanique, d'admission en soins Intensifs, d'intervention chirurgicale, de médicament de dernier recours, d'admission à l'hôpital, ou d'une combinaison de ceux-ci ;
(d) comprend la recommandation de l'administration d'un antiviral à un individu diagnostiqué avec une infection virale ; et/ou
(e) comprend la recommandation de l'administration d'un antibiotique à un individu diagnostiqué avec une infection bactérienne.

FIG. 1

dCT LY6E 1 vs. Sample Preservative & Infectious Status

FIG. 2

dCT IRF9 3 vs. Sample Preservative & Infectious Status

FIG. 3

FIG. 4

FIG. 5

FIG. 6

FIG. 7

EP 4 308 729 B1

FIG. 8

FIG. 9

EP 4 308 729 B1

ITGAM Ct & ITGAM Target Delta Ct vs. Bacterial (y/n)

FIG. 10

EP 4 308 729 B1

FIG. 11

FIG. 12

FIG. 13

# RUNX1 vs. Infection Status

FIG. 14

# LY6E vs. Infection Status

**LY6E Ct vs. Infection status** ⊡ LY6E Ct / ● LY6E Ct

**LY6E Target Delta vs. Infection status** ⊡ LY6E Target Delta Ct / ● LY6E Target Delta Ct

FIG. 15

FIG. 16

FIG. 17

PSTPIP2 vs. Infection Status

FIG. 18

Bacterial y/n model with all 6 targets

FIG. 19

FIG. 20

FIG. 21

LY6E

LY6E Ct & LY6E Target Delta Ct vs. Healthy/Viral/Bacterial/MIS-C/Other

FIG. 22

FIG. 23

FIG. 24

EP 4 308 729 B1

FIG. 25

## Nominal Logistic Fits

Using Bacterial (y/n)='y' to be the positive level
AUC
0.78386

Using Viral (y/n)='y' to be the positive level
AUC
0.86906

Using MISC (y/n)='y' to be the positive level
AUC
0.86797

## FIG. 26

FIG. 27

EP 4 308 729 B1

FIG. 28

EP 4 308 729 B1

FIG. 29

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2020216904 A
- US 2015376698 A
- WO 2017214061 A
- WO 2011132086 A
- WO 2013117746 A
- US 5958349 A
- US 6403037 B

- US 6440725 B
- US 6783736 B
- US 6818185 B
- US 9873909 B
- US 10562030 B
- US 10465182 B

**Non-patent literature cited in the description**

- **YONKER L et al.** *J Clin Invest*, 2021, vol. 131 (14), e149633